# EUROPEAN PATENT APPLICATION

(11) **EP 4 596 529 A1**
(43) Date of publication of application: **06.08.2025**
(21) Application number: 23914119.5
(22) Date of filing: 19.01.2023
(51) Int. Cl.: C07C 229/16, C07C 227/08, C07C 227/16, C07C 329/06, C07C 333/04, A61K 31/7088, A61K 45/00, A61P 31/00, A61P 35/00, A61P 37/00, A61P 43/00, A61P 3/10, A61P 25/28, A61P 9/00, A61P 13/12, A61P 3/00

(54) **IONIZABLE CATIONIC LIPID COMPOUND AND COMPOSITION FOR DELIVERING NUCLEIC ACID AND USE THEREOF**

(30) Priority: 05.01.2023 CN 202310010951
(71) Applicant: Beijing Youcarekechuang Pharmaceutical Technology Co., Ltd., Beijing 100176 (CN)
(72) Inventor: SONG, Gengshen, Beijing 100176 (CN); CHEN, Xichao, Beijing 100176 (CN); ZHANG, Honglei, Beijing 100176 (CN); WANG, Huanyu, Beijing 100176 (CN); HUANG, Dawei, Beijing 100176 (CN); YU, Xiaowen, Beijing 100176 (CN); JIN, Lijie, Beijing 100176 (CN); MA, Yuqing, Beijing 100176 (CN); AN, Ran, Beijing 100176 (CN); LI, Jing, Beijing 100176 (CN); WANG, Shuai, Beijing 100176 (CN); SUN, Zhenlong, Beijing 100176 (CN); ZHANG, Jinyu, Beijing 100176 (CN)
(74) Representative: Potter Clarkson
(86) International application number: PCT/CN2023/073226
(87) International publication number: WO 2024/145963

(57) **Abstract**

Provided in the present invention is a compound of Formula (I) or an N-oxide, a solvate, a pharmaceutically acceptable salt or a stereoisomer thereof, which compound is an ionizable cationic lipid compound. Further provided are a composition comprising the aforementioned compound and the use thereof in the delivery of a therapeutic agent or a prophylactic agent.

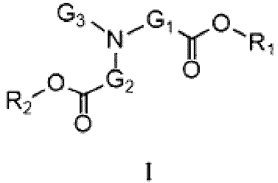

## Description

### CROSS REFERENCE TO RELATED APPLICATIONS

This application claims the priority of Chinese Patent Application No. 202310010951.6, filed with the China National Intellectual Property Administration on January 05, 2023, which is hereby incorporated by reference in its entirety as a part of the present application.

### FIELD

The present disclosure belongs to the field of medicine, and particularly relates to a cationic lipid compound, a composition comprising the same and use thereof.

### BACKGROUND

Effective targeted delivery of bioactive substances such as small molecule drugs, polypeptides, proteins and nucleic acids, especially nucleic acids, is a persistent medical problem. Nucleic acid therapeutics undertake great challenges due to low cell permeability and high sensitivity to degradation of certain nucleic acid molecules (including RNA).

It has been confirmed that compositions comprising cationic lipids, liposomes and lipoplexes are effective as transport vehicles for delivering bioactive substances such as small molecule drugs, peptides, proteins and nucleic acids to cells and/or intracellular compartments. These compositions generally comprise one or more "cationic" and/or amino (ionizable) lipids, including neutral lipids, structured lipids and polymer-conjugated lipids. Cationic and/or ionizable lipids include, for example, amine-containing lipids that can be easily protonated. Although a variety of such lipid-containing nanoparticle compositions have been demonstrated, safety, efficacy and specificity remain to be improved. It is worth noting that the increased complexity of lipid nanoparticles (LNPs) complicates their production and may increase their toxicity, which is a major concern that may limit their clinical application. For example, LNP siRNA particles (such as patisiran) require the use of steroids and antihistamines in advance to eliminate unnecessary immune responses (T. Coelho, D. Adams, A. Silva, et al., Safety and efficacy of RNAi therapy for transthyretin amyloidosis, N Engl J Med, 369 (2013) 819-829.). Therefore, there is a need to develop improved cationic lipid compounds that facilitate the delivery of therapeutic and/or prophylactic agents such as nucleic acids to cells, and a composition comprising the same.

### SUMMARY

In an aspect, the present disclosure provides a novel cationic lipid compound, which is a compound of Formula (I) 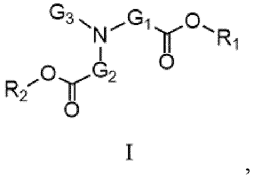 or an N-oxide, solvate, a pharmaceutically acceptable salt or a stereoisomer thereof, wherein: G₁ is C₁₋₆ alkylene; G₂ is C₂₋₈ alkylene; R₁ is C₆₋₂₀ straight chain or branched alkyl; R₂ is C₁₂₋₂₅ branched alkyl; G₃ is HO(CH₂)₂N(CH₃)(CH₂)₂-, HO(CH₂)₂N(CH₂CH₃)(CH₂)₂-, (HO(CH₂)₂)₂N(CH₂)₂-, CH₃O(CH₂)₂N(CH₃)(CH₂)₂-, (CH₃)₂N(CH₂)₃SC(O)O(CH₂)₂-, (CH₃)₂N(CH₂)₃SC(O)-, CH₃NH(CH₂)₂N(CH₃)(CH₂)₂- or CH₃CH₂NH(CH₂)₂-.

For example, the compound of Formula (I) has a structure selected from the group consisting of: 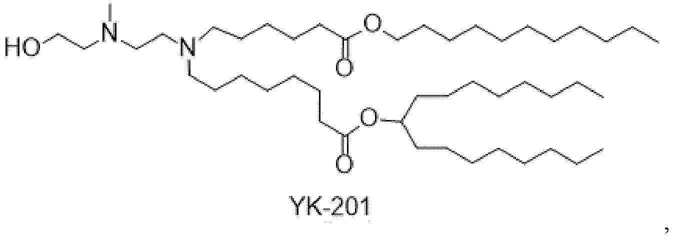 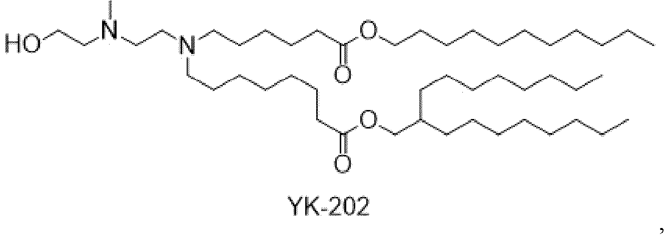 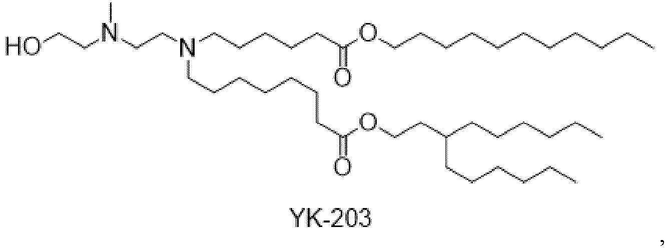 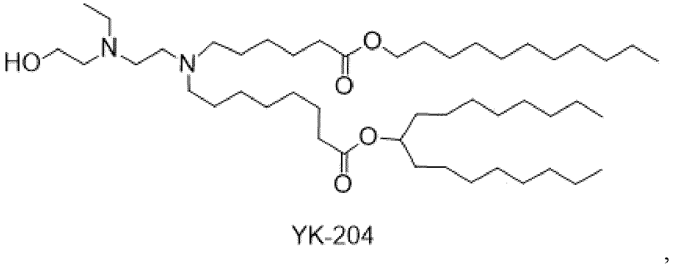 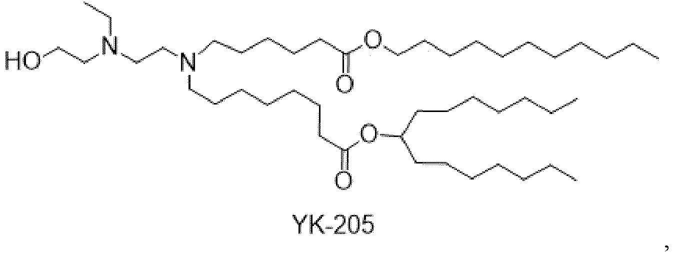 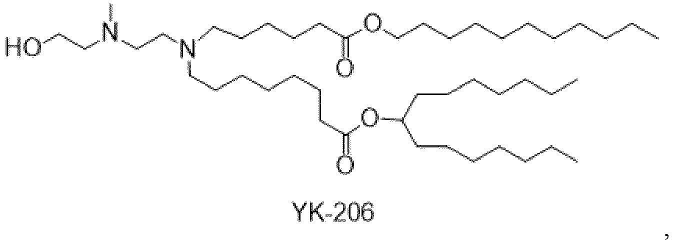 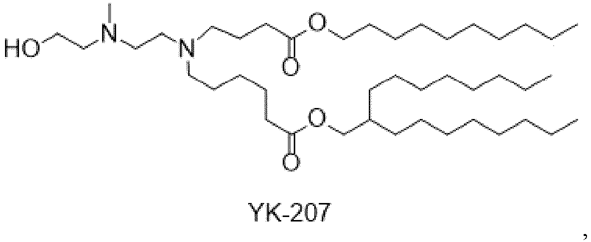 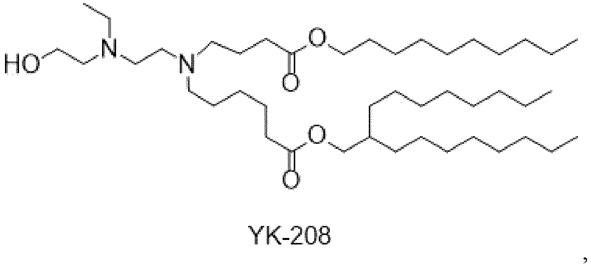 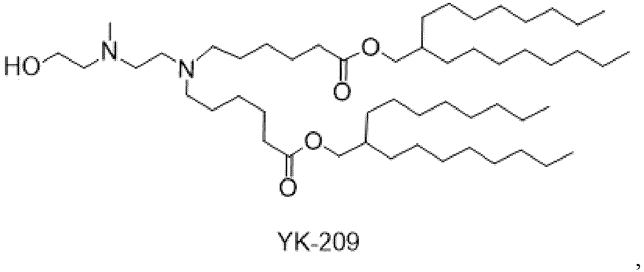 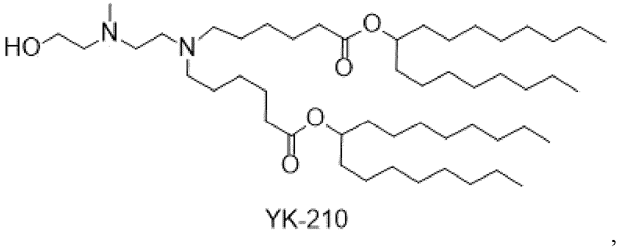 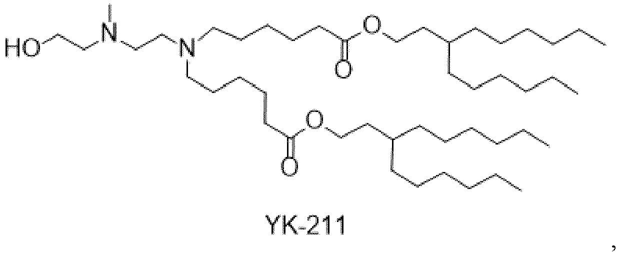 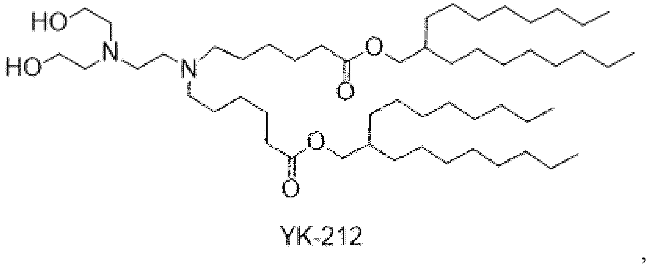 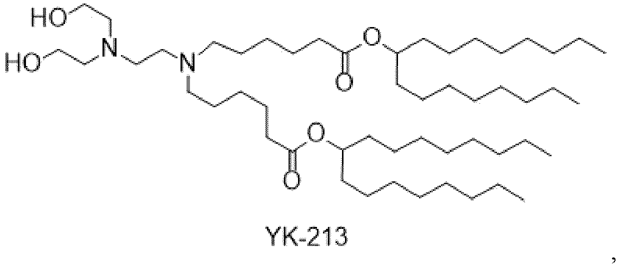 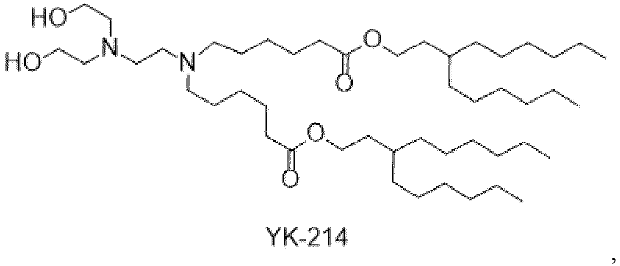 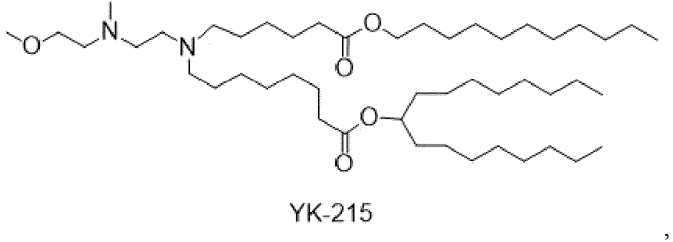 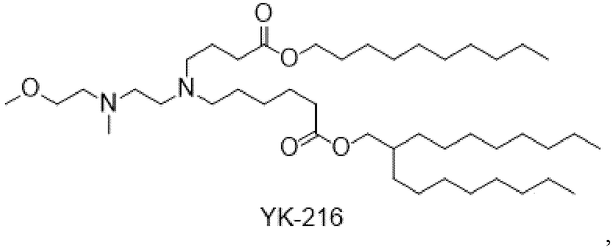 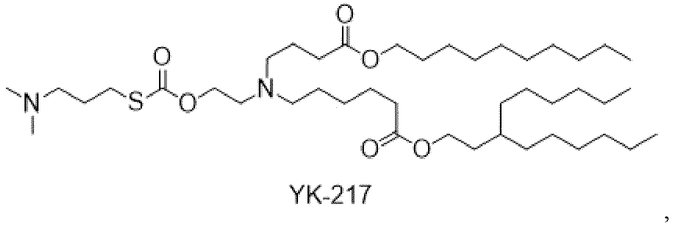 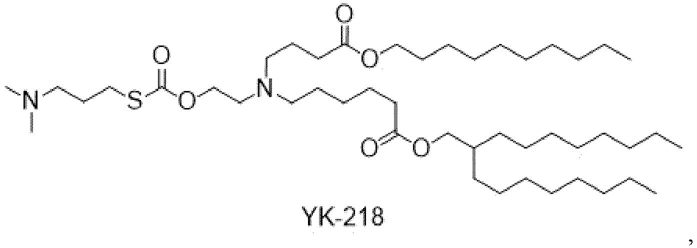 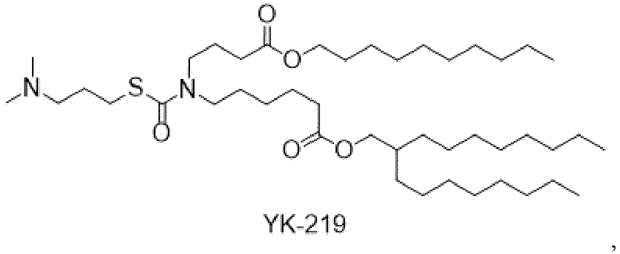 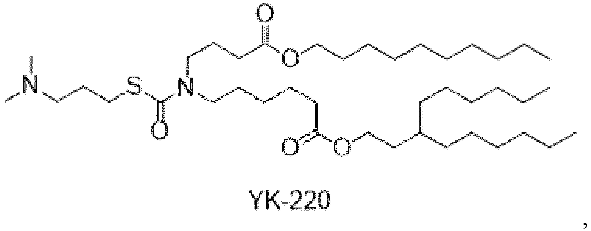 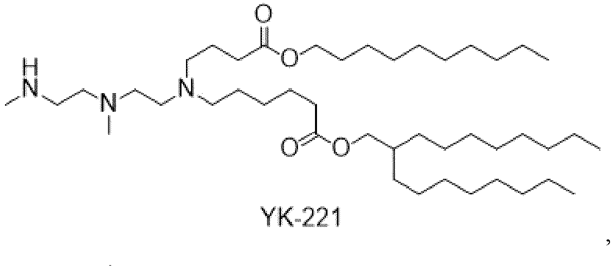 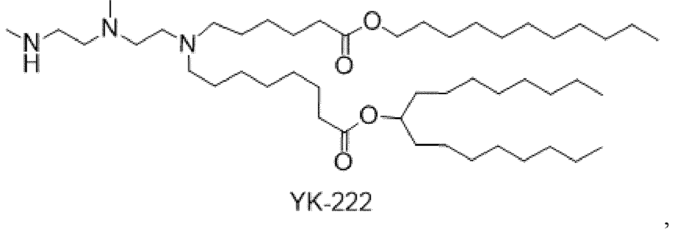 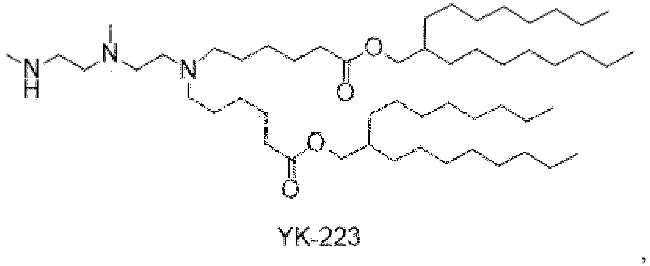 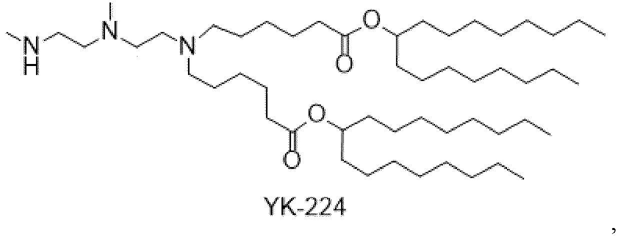 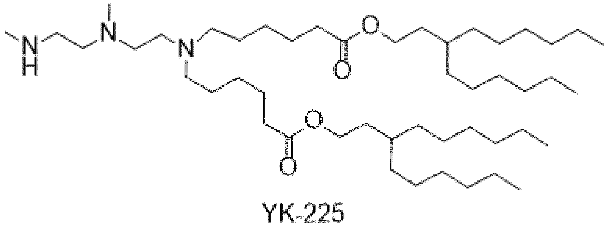 and

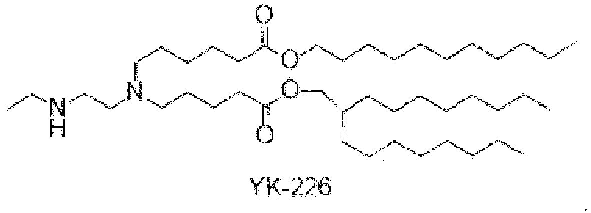

Another aspect of the present disclosure provides a composition comprising a carrier, wherein the carrier comprises a cationic lipid, and the cationic lipid comprises the above-mentioned compound of Formula (I) or an N-oxide, a solvate, a pharmaceutically acceptable salt or a stereoisomer thereof.

Another aspect of the present disclosure provides use of the above-mentioned compound of Formula (I) or the N-oxide, solvate, pharmaceutically acceptable salt or stereoisomer thereof or the above-mentioned composition in the manufacture of a nucleic acid drug, a gene vaccine, a small molecule drug, a polypeptide or a protein drug.

Another aspect of the present disclosure provides use of the above-mentioned compound of Formula (I) or the N-oxide, solvate, pharmaceutically acceptable salt or stereoisomer thereof or the above-mentioned composition in the manufacture of a medicament for treating a disease or condition in a mammal in need thereof.

### BRIEF DESCRIPTION OF DRAWINGS

FIG. 1 shows the results of cell transfection experiments with different weight ratios of carrier to mRNA used in the preparation of a LNP formulation, wherein a is carrier: mRNA = 5:1, b is carrier: mRNA = 15:1, c is carrier: mRNA = 35:1; and d is a blank control.
FIG. 2 shows the results of cell transfection experiments with different molar ratios of cationic lipids to neutral lipid DSPC used in the preparation of a LNP formulation, wherein a is 3.5:1, b is 4.5: 1, c is 4.9: 1, and d is a blank control.
FIG. 3 shows the results of cell transfection experiments with different molar ratios of polymer-conjugated lipids to carriers in the preparation of a LNP formulation, wherein a is 1.5%, b is 10%, and c is a blank control.
FIG. 4 shows the results of cell transfection experiments with different ratios of cationic lipids, neutral lipid DSPC, structured lipid cholesterol and polymer-conjugated lipid DMG-PEG2000 in the carrier when preparing a LNP formulation, wherein a is 49:10:39.5:1.5, b is 45:10:43.5:1.5, c is 35:10:53.5:1.5, and d is a blank control.
FIG. 5 shows the fluorescence absorption intensity of Fluc-mRNA LNP formulation prepared by different cationic lipids (a: YK-201; b: YK-202; c: YK-209; d: SM-102).
FIG. 6 shows the fluorescence absorption intensity of Fluc-mRNA LNP formulation prepared by different cationic lipids (a: YK-204; b: YK-205; c: YK-211; d: Compound 21).
FIG. 7 shows the fluorescence absorption intensity of Fluc-mRNA LNP formulation prepared by different cationic lipids (a: YK-201; b: YK-202; c: YK-221; d: YK-225).
FIG. 8 shows the survival rate of cells after 24 hours of culture with Fluc-mRNA LNP formulations prepared by different cationic lipids (YK-201, YK-202, YK-206, YK-207, YK-209, YK-009, SM-102, ALC-0315, compound 21, compound 23 and HHMA) and Lipofectamine 3000 formulation containing Fluc-mRNA added into the cell culture.
FIG. 9 shows the survival rate of cells after 24 hours of culture with Fluc-mRNA LNP formulations prepared by different cationic lipids (YK-201, YK-202, YK-209, YK-203, YK-204, YK-205, YK-208, YK-210, YK-211, SM-102, ALC-0315, compound 21, compound 23 and HHMA) and Lipofectamine 3000 formulation containing Fluc-mRNA added into the cell culture.
FIG. 10 shows the survival rate of cells after 24 hours of culture with Fluc-mRNA LNP formulations prepared by different cationic lipids (YK-201, YK-202, YK-209, YK-212, YK-213, YK-214, YK-215, YK-216, YK-217, YK-218, YK-219, YK-220, SM-102, ALC-0315, compound 21, compound 23 and HHMA) and Lipofectamine 3000 formulation containing Fluc-mRNA added into the cell culture.
FIG. 11 shows the survival rate of cells after 24 hours of culture with Fluc-mRNA LNP formulations prepared by different cationic lipids (YK-201, YK-202, YK-209, YK-221, YK-222, YK-223, YK-224, YK-225, YK-226, SM-102, ALC-0315, compound 21, compound 23 and HHMA) and Lipofectamine 3000 formulation containing Fluc-mRNA added into the cell culture.
FIG. 12 shows the results of mouse *in vivo* imaging experiments of Fluc-mRNA LNP formulation prepared by different cationic lipids (YK-201, YK-204, YK-206, YK-217, SM-102, ALC-0315, compound 21, compound 23 and HHMA).
FIG. 13 shows the results of mouse *in vivo* imaging experiments of Fluc-mRNA LNP formulation prepared by different cationic lipids (YK-202, YK-207, YK-215, SM-102, ALC-0315, compound 21, compound 23, and HHMA).
FIG. 14 shows the results of mouse *in vivo* imaging experiments of Fluc-mRNA LNP formulation prepared by different cationic lipids (YK-209, YK-223, YK-009, SM-102, ALC-0315, compound 21, compound 23, and HHMA).

### DETAILED DESCRIPTION

In order to make the purpose, technical solution and advantages of the embodiments of the present disclosure clearer, the technical solution of the embodiments of the present disclosure will be clearly and completely described in conjunction with the drawings of the examples of the present disclosure. Obviously, the described examples are a part of the examples of the present disclosure, not all of the examples. Based on the described examples of the present disclosure, all other examples obtained by those of ordinary skill in the art without making inventive effort belong to the scope of protection of the present disclosure.

The present disclosure can be implemented in other specific forms without deviating from the basic attributes of the present disclosure. It should be understood that, under the premise of no conflict, any and all embodiments of the present disclosure can be combined with the technical features in any other embodiment or multiple other embodiments to obtain another embodiment. The present disclosure includes other embodiments obtained by such combination.

All publications and patents mentioned in the present disclosure are hereby incorporated into the present disclosure by reference in their entirety. If the use or terminology used in any publication and patent incorporated by reference conflicts with the use or terminology used in the present disclosure, the use and terminology of the present disclosure shall prevail.

The section titles used herein are only for the purpose of organizing the article and should not be interpreted as limiting the subject matter described.

Unless otherwise specified, all technical terms and scientific terms used herein have the usual meaning in the field to which the subject matter to be protected belongs. If there are multiple definitions for a term, the definition herein shall prevail.

Except in the working examples or otherwise indicated, all numbers stating quantitative properties such as dosage in the specification and claims should be understood to be modified by the term "about" in all cases. It should also be understood that any numerical range listed in the present application is intended to include all subranges within the range and any combination of the various endpoints of the range or subrange.

The words "including", "containing" or "comprising" and the like used in the present disclosure mean that the elements appearing before the word include the elements listed after the word and their equivalents, without excluding unrecorded elements. The terms "containing" or "including (comprising)" used herein can be open, semi-closed and closed. In other words, the term also includes "essentially composed of..." or "consisting of...".

The term "pharmaceutically acceptable" in the present application means that the compound or composition is chemically and/or toxicologically compatible with other ingredients constituting the formulation and/or with mammals for preventing or treating diseases or conditions with it.

The term "subject" or "patient" in the present application includes mammals and non-mammals. Mammals include, but are not limited to, humans, gorillas, apes, monkeys, cows, horses, sheep, pigs, rabbits, dogs, cats, and mice. Non-mammals include, but are not limited to, birds and fish. In some embodiments, a "subject" or "patient" is a mammal, such as a human.

The term "treatment" as used herein refers to administering one or more drug substances to a patient or subject suffering from a disease or having symptoms of the disease to cure, alleviate, mitigate, ameliorate or affect the disease or symptoms of the disease. In the context of the present application, unless otherwise specifically stated, the term "treatment" may also include prevention.

The term "solvate" in the present application refers to a complex formed by combining a compound of Formula (I) or a pharmaceutically acceptable salt thereof with a solvent (e.g., ethanol or water). It should be understood that any solvate of a compound of Formula (I) used in the treatment of a disease or condition, although it may provide different properties (including pharmacokinetic properties), will give a compound of Formula (I) once absorbed into a subject, so that the use of a compound of Formula (I) covers the use of any solvate of the compound of Formula (I).

The term "hydrate" refers to the case where the solvent in the above-mentioned term "solvate" is water.

It should be further understood that the compound of Formula (I) or its pharmaceutically acceptable salt can be isolated in the form of a solvate, and therefore any such solvate is included within the scope of the present disclosure. For example, the compound of Formula (I) or its pharmaceutically acceptable salt can exist in an unsolvated form as well as a solvated form formed with a pharmaceutically acceptable solvent (such as water and ethanol).

The term "pharmaceutically acceptable salt" refers to a relatively non-toxic, inorganic acid or organic acid addition salt of a compound of the present disclosure. For example, see S. M. Berge et al. "Pharmaceutical Salts", J. Pharm. Sci. 1977, 66, 1-19. Among them, inorganic acids include hydrochloric acid, hydrobromic acid, hydroiodic acid, sulfuric acid, phosphoric acid or nitric acid; organic acids include formic acid, acetic acid, acetoacetic acid, pyruvic acid, trifluoroacetic acid, propionic acid, butyric acid, hexanoic acid, heptanoic acid, undecanoic acid, lauric acid, benzoic acid, salicylic acid, 2-(4-hydroxybenzoyl)-benzoic acid, camphoric acid, cinnamic acid, cyclopentanepropionic acid, digluconic acid, 3-hydroxy-2-naphthoic acid, nicotinic acid, pamoic acid, pectinic acid, 3-phenylpropionic acid, picric acid, pivalic acid, 2-hydroxyethanesulfonic acid, itaconic acid, aminosulfonic acid, trifluoromethanesulfonic acid, dodecylsulfuric acid, ethanesulfonic acid, benzenesulfonic acid, p-toluenesulfonic acid, methanesulfonic acid, 2-naphthalenesulfonic acid, naphthalenedisulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptonic acid, glycerophosphoric acid, aspartic acid, sulfosalicylic acid, etc. For example, HCl (or hydrochloric acid), HBr (or hydrobromic acid solution), methanesulfonic acid, sulfuric acid, tartaric acid or fumaric acid can be used to form a pharmaceutically acceptable salt with the compound of Formula (I).

The nitrogen-containing compound of Formula (I) of the present disclosure can be converted into an N-oxide by treating with an oxidizing agent (e.g., m-chloroperbenzoic acid, hydrogen peroxide, ozone). Therefore, under the conditions that the valence state and structure allow, the compounds claimed in the present application include not only the nitrogen-containing compounds of the structural formula, but also the N-oxide derivatives thereof.

Certain compounds of the present disclosure may exist in the form of one or more stereoisomers. Stereoisomers include geometric isomers, diastereomers and enantiomers. Therefore, the compounds claimed in the present disclosure also include racemic mixtures, single stereoisomers and optically active mixtures. It should be understood by those skilled in the art that one stereoisomer may have better efficacy and/or lower side effects than other stereoisomers. Single stereoisomers and optically active mixtures can be obtained by chiral source synthesis, chiral catalysis, chiral resolution and the like. The racemate can be chirally resolved by chromatographic resolution or chemical resolution. For example, chiral acid resolution agents such as chiral tartaric acid and chiral malic acid can be added to the compounds of the present disclosure to form salts, and the physical and chemical properties of the products, such as different solubility, can be used for separation.

The present disclosure also includes all suitable isotopic variants of the compounds of the present disclosure. An isotopic variant is defined as a compound in which at least one atom is replaced by an atom having the same atomic number but an atomic mass different from the atomic mass common or predominant in nature. Examples of isotopes that can be introduced into the compounds of the present disclosure include isotopes of hydrogen, carbon, nitrogen and oxygen, such as ²H (deuterium), ³H (tritium), ¹¹C, ¹³C, ¹⁴C, ¹⁵N, ¹⁷O and ¹⁸O, respectively.

The term "alkyl" in the present disclosure refers to a branched and straight-chain saturated aliphatic monovalent hydrocarbon group having a specified number of carbon atoms. The term "alkylene" in the present disclosure refers to a branched and straight-chain saturated aliphatic divalent hydrocarbon group having a specified number of carbon atoms. Cₙ₋ₘ refers to a group having n to m carbon atoms. For example, C₂₋₅ alkylene includes C₂ alkylene, C₃ alkylene, C₄ alkylene, or C₅ alkylene.

Alkyl (or alkylene) may be unsubstituted, or substituted, wherein at least one hydrogen is replaced by another chemical group.

A "therapeutically effective amount" is an amount of a therapeutic agent that can improve a disease or symptom when administered to a patient. A "prophylactic effective amount" is an amount of a prophylactic agent that can prevent a disease or symptom when administered to a subject. The amount of a therapeutic agent constituting a "therapeutically effective amount" or the amount of a prophylactic agent constituting a "prophylactic effective amount" varies with the therapeutic agent/prophylactic agent, the disease state and its severity, the age, weight, etc. of the patient/subject to be treated/prevented. Those of ordinary skill in the art can routinely determine the therapeutic effective amount and the prophylactic effective amount based on their knowledge and the present disclosure.

In the present application, when the name of a compound is inconsistent with the structural formula, the structural formula shall prevail.

It should be understood that the term "compound of the present disclosure" used herein may include: a compound of Formula (I), a N-oxide, a solvate, a pharmaceutically acceptable salt, a stereoisomer, and a mixture thereof, depending on the context.

The term cationic lipid used herein refers to a lipid that is positively charged at a selected pH value.

Cationic liposomes are easily bound to negatively charged nucleic acids, that is, they interact with negatively charged phosphate groups present in nucleic acids through electrostatic forces to form lipid nanoparticles (LNPs). LNP is one of the mainstream delivery carriers currently.

When screening a large number of compounds, the inventors found that it was very difficult to screen out suitable cationic lipid compounds that meet the following requirements: significantly different from the representative cationic lipid structures in the prior art, with high encapsulation rate, drug loading concentration and total RNA concentration, high transfection efficiency and low cytotoxicity, and high expression and sustained expression in mice. The inventors found that some compounds, such as YK-201, YK-202 and YK-209, can deliver nucleic acids with significantly improved encapsulation rate, drug loading concentration and total RNA concentration, significantly improved intracellular transfection efficiency, significantly reduced cytotoxicity, and significantly improved expression and duration in animals compared with cationic lipids with very different chemical structures in the prior art. The present disclosure is based on at least the following findings:
1. The designed series of compounds, including YK-201, YK-202 and YK-209, as well as YK-206 and YK-207, have significant differences in chemical structure from representative cationic lipids in the prior art, such as SM-102 (compound 25 disclosed in WO2017049245A2), compound 21 and compound 23 disclosed in WO2021055833A1, HHMA (compound 1 disclosed in CN112979483B), ALC-0315 (compound 3 disclosed in CN108368028B) and compound YK-009 disclosed in CN114044741B. Wherein, the G₃ groups are completely different, and other parts are also different, so there are also great differences in polarity, acidity and alkalinity, and hydrophilicity. Therefore, it is impossible to infer the cell transfection efficiency, cytotoxicity, and in vivo expression in animals of the LNP formulations prepared from this series of compounds based on the above-mentioned cationic lipid compounds disclosed in the prior art.

The chemical structures of SM-102, ALC-0315, compound 21, compound 23 and HHMA are as follows: 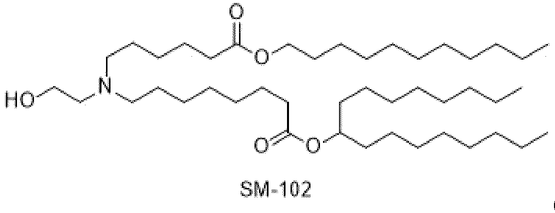 (WO2017049245A2, page 29 of the specification); 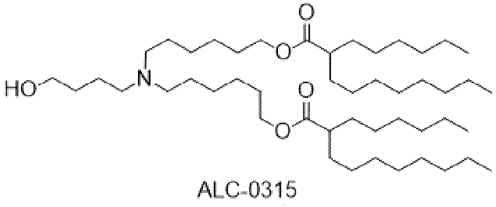 (CN108368028B, page 24 of the specification); 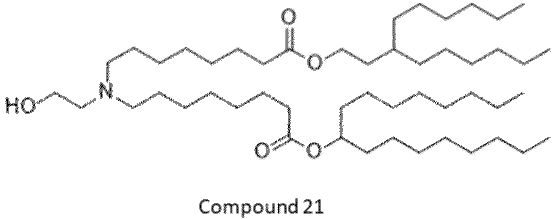 (WO2021055833A1, page 22 of the specification); 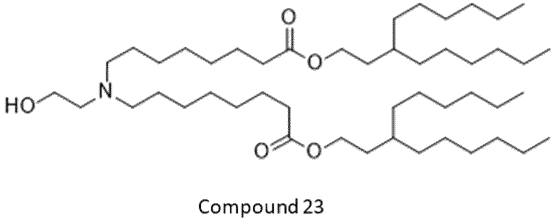 (WO2021055833A1, page 22 of the specification); 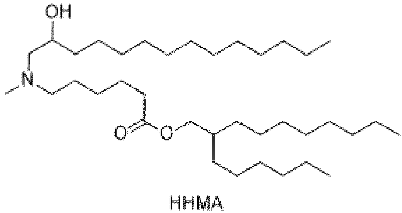 (CN112979483B, page 12 of the specification).

2. Among this series of designed compounds, the LNP formulation prepared from YK-201, YK-202 and YK-209 have significantly improved encapsulation rate, drug loading concentration and total RNA concentration, significantly improved cell transfection activity, significantly reduced cytotoxicity, and significantly increased mRNA expression and duration in mice compared with representative cationic lipids in the prior art.

For example, the encapsulation rate of YK-209 can be increased by 41% compared with compound 23, the drug loading concentration of YK-209 can reach 2 times that of compound 23, and the total RNA concentration of YK-201 can reach 1.5 times that of compound 21; the cell transfection activity of YK-202 can reach 18 times that of SM-102, 21 times that of compound 21, and 22 times that of compound 23; the cell survival rate of YK-202 can be 26.85% higher than that of ALC-0315, 8.26% higher than that of SM-102, and 11.25% higher than that of HHMA; the mRNA expression level in mice of YK-202 can reach 24 times that of SM-102, 25 times that of compound 21, and 23 times that of compound 23.

Among this series of compounds designed with very small differences in chemical structure, the LNP formulations prepared from YK-201, YK-202 and YK-209 showed significantly improved cell transfection activity, significantly reduced cytotoxicity, and significantly increased mRNA expression level and duration in mice compared with other compounds.

Structurally, this series of compounds differ from YK-201, YK-202 and YK-209 only in 1 to 2 C atoms in individual groups, including 2 C atoms being reduced in G₁, 1 C atom being reduced in R₁, an ether bond, an ester bond or a sulfur atom being introduced in G₃, or a hydroxyl group being changed into a methylamino group in the G₃ group, or the hydroxyl group and the methyl group connected to N being removed from the G₃ group. However, the cell transfection activity of YK-201, YK-202 and YK-209 can reach more than 1000 times that of YK-221 and YK-225, and the cytotoxicity can be reduced by 55% compared with YK-221. The expression level of mRNA in mice can reach more than 1000 times that of YK-223.

3. There is no obvious correspondence between the structure of cationic lipid compounds and the intracellular transfection efficiency, toxicity to cells, and high and sustained expression of mRNA in LNP formulations prepared from them in animals. Compounds with small structural differences are very likely to have very large differences in transfection efficiency and/or toxicity to cells, and intracellular expression.

For example, compared with YK-201, YK-204 only has one more C in the G₃ group connected to N, and the other structures are exactly the same. However, the cell transfection efficiency of YK-201 is 78 times that of YK-204, and the toxicity of YK-201 to transfected cells is 30% lower than that of YK-204. The mRNA expression of YK-201 in mice can reach 380 times that of YK-204. Compared with YK-209, YK-225 only has methylamino changed from hydroxyl in the G₃ group, the single chains of R₁ and R₂ groups each have one more C, and each single chain in the double chain has 2 less C, and the other structures are exactly the same. However, the cell transfection activity of YK-209 reaches 800 times that of YK-225, and the toxicity of YK-209 to transfected cells is 30% lower than that of YK-225.

Therefore, screening suitable cationic lipid compounds that can simultaneously have high encapsulation rate, drug loading concentration and total RNA concentration, high transfection efficiency and low toxicity to cells, and high and sustained expression of mRNA in mice is very difficult, which requires a lot of inventive work.

4. Through unique design and extensive screening, it was found in the present disclosure that some compounds, such as YK-201, YK-202, YK-209, YK-206 and YK-207, can deliver nucleic acids with significantly improved encapsulation rate, drug loading concentration and total RNA concentration, significantly improved cell transfection efficiency, significantly reduced cytotoxicity, and significantly increased expression level and duration in animals compared to other compounds in the prior art, achieving unexpected technical effects.

The details are as follows:

### 1. Compared with the representative cationic lipids in the prior art, such as SM-102, ALC-0315, compound 21, compound 23, YK-009 and HHMA, the chemical structures are significantly different

Representative cationic lipids in the prior art, such as SM-102, ALC-0315, compound 21, compound 23, YK-009 and HHMA, are compared with this series of designed compounds:
a. The structure of HHMA is the most different. The group connected to the central N atom of HHMA has only one side chain similar to one side chain of this series of structures, and the other parts are completely different.
b. Other cationic lipids in the prior art, such as SM-102, ALC-0315, compound 21, compound 23 and YK-009, have completely different G₃ groups. Due to the different structures of the G3 groups, they also have great differences in polarity, acidity and alkalinity, and hydrophilicity.
c. The G₁, R₁, G₂ and R₂ groups of SM-102, ALC-0315, compound 21, compound 23 and YK-009 are also significantly different.

### 2. The encapsulation rate, drug loading concentration and total RNA concentration are significantly higher than those of representative cationic lipids in the prior art.

1) Compared with the cationic lipids in the prior art, the LNP formulations prepared by YK-201, YK-202, YK-206, YK-207 and YK-209 have significantly improved encapsulation rate, drug loading concentration and total RNA concentration. For example, the encapsulation rate of YK-209 can be increased by 41% compared with compound 23, and the drug loading concentration can reach 2 times that of compound 23; the total RNA concentration of YK-201 can reach 1.5 times that of compound 21.
2) LNP formulations prepared by different designed compounds vary greatly in terms of encapsulation rate and drug loading amount. The encapsulation rate of different compounds ranges from 55% to 99%, the drug loading concentration is between 25 and 45 µg/mL, and the total RNA concentration is between 25 and 50 µg/mL.

### 3. The in vitro cell transfection efficiency is significantly improved compared with the representative cationic lipids and structurally similar compounds in the prior art

1) The LNP formulations prepared from YK-201, YK-202 and YK-209 have the highest cell transfection efficiency and are significantly more active than representative cationic lipids in the prior art. For example, YK-202 can reach 18 times that of SM-102, 21 times that of compound 21, and 22 times that of compound 23.
2) YK-201, YK-202 and YK-209 have the highest cell transfection efficiency among the series of compounds designed, which are most similar in structure, and only differ by 1-2 carbons in G₁, G₂, G₃, R₁ or R₂ groups. The cell transfection efficiency of YK-202 can reach 80 times that of other compounds, such as YK-204.
3) YK-201, YK-202 and YK-209 have the highest transfection efficiency compared to the compounds with only minor differences in individual groups, such as 2 C less in G₁, 1 C less in R₁, and ether, ester or sulfur atoms introduced in G₃. The transfection efficiency of YK-202 is more than 400 times higher than that of other compounds, such as YK-217.
4) YK-201, YK-202 and YK-209 have the highest cell transfection efficiency compared with the compounds in which only the hydroxyl group in the G₃ group is changed into a methylamino group, or the hydroxyl group and the methyl group connected to N are removed. For example, compared with YK-221 and YK-225, the cell transfection efficiency of YK-201 and YK-202 can be increased by more than 1000 times.
5) There is no corresponding relationship between the structure and the intracellular transfection efficiency of the compound. Compounds with small structural differences are likely to have very large differences in transfection efficiency. Therefore, screening out cationic lipid compounds with high transfection efficiency requires multiple designs and a lot of inventive work.

### 4. The cytotoxicity is significantly lower than that of representative cationic lipids and structurally similar compounds in the prior art

1) The LNP formulations prepared by YK-201, YK-202 and YK-209 have the lowest cytotoxicity and significantly improved cell survival rate compared with the representative cationic lipids in the prior art. For example, the cell survival rate of YK-202 is 26.85% higher than that of ALC-0315, 8.26% higher than that of SM-102, and 11.25% higher than that of HHMA.
2) YK-201, YK-202 and YK-209 have the lowest cytotoxicity among the series of compounds designed, which are most similar in structure, and only differ by 1 to 2 carbons in G₁, G₂, R₁ or R₂ groups. The cell survival rate of the three compounds can be increased by 40% compared with other compounds, such as YK-203.
3) YK-201, YK-202 and YK-209 have the lowest cytotoxicity compared to the compounds with only minor differences in individual groups in their structures. The cell survival rates of all three compounds can be increased by 50% compared to other compounds, such as YK-214.
4) YK-201, YK-202 and YK-209 show the lowest cytotoxicity compared with the compounds in which only the hydroxyl group in the G₃ group is changed into a methylamino group, or the hydroxyl group and the methyl group connected to N are removed. For example, the cell survival rates of YK-201, YK-202 and YK-209 were all 55% higher than that of YK-221.
5) There is no correspondence between the structure and cytotoxicity of a compound. Even compounds with small structural differences are likely to have very different cytotoxicity. Therefore, it is impossible to predict cytotoxicity based on chemical structure. Screening out cationic lipid compounds with low cytotoxicity is very difficult and requires a lot of inventive work.

### 5. The expression level and duration of mRNA in animals are significantly higher than those of representative cationic lipids and structurally similar compounds in the prior art.

1) The LNP formulations prepared by YK-201, YK-202 and YK-209 show high and sustained expression of mRNA in mice, which is significantly higher than that of representative cationic lipids in the prior art. For example, YK-202 was 24 times that of SM-102, 25 times that of compound 21 and 23 times that of compound 23.
2) YK-201, YK-202 and YK-209 have the highest mRNA expression in mice and the longest duration among the series of compounds designed, which are most similar in structure, and only differ by 1 to 2 carbons in G₁, G₂, G₃, R₁ or R₂ groups. For example, the mRNA expression of YK-202 can reach more than 400 times that of YK-204 in 24 hours and still reach 40 times in 7 days.
3) YK-201, YK-202 and YK-209 show the highest mRNA expression in mice and the longest duration among a series of compounds that differ only slightly in the G₁, G₂, G₃, R₁ or R₂ groups. For example, the mRNA expression of YK-202 is 800 times higher than that of YK-217.
4) YK-201, YK-202 and YK-209 show the highest mRNA expression in mice and the longest duration compared with the compounds in which only the hydroxyl group in the G₃ group is changed into a methylamino group, or the hydroxyl group and the methyl group connected to N are removed. For example, the mRNA expression level of the three compounds is more than 1,000 times higher than that of YK-223.
5) There is no correspondence between the structure of cationic lipids and the high and sustained expression of delivered mRNA in mice. Even for the LNP formulations prepared from cationic lipid compounds with small structural differences, the mRNA therein is likely to have very different expressions in animals. It is impossible to predict whether mRNA is highly and sustainably expressed in animals based on the chemical structure of cationic lipids. It is very difficult to screen out cationic lipid compounds with high and sustained mRNA expression, which requires a lot of inventive work.

In an aspect, the present disclosure provides a novel cationic lipid compound for delivering a therapeutic agent or a prophylactic agent. The cationic lipid compound of the present disclosure can be used to deliver nucleic acid molecules, small molecule compounds, polypeptides or proteins. Compared with known cationic lipid compounds, the cationic lipid compounds of the present disclosure exhibit higher transfection efficiency and less cytotoxicity, thereby improving delivery efficiency and safety.

The present disclosure provides a cationic lipid, which is a compound of Formula (I) 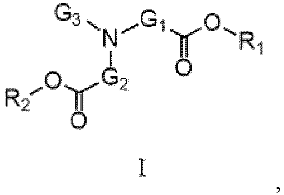 or a N-oxide, solvate, pharmaceutically acceptable salt or stereoisomer thereof, wherein
G₁ is C₁₋₆ alkylene, preferably unsubstituted C₂₋₅ alkylene, more preferably unsubstituted C₅ alkylene or unsubstituted C₃ alkylene;
G₂ is C₂₋₈ alkylene, preferably unsubstituted C₄₋₇ alkylene, more preferably unsubstituted C₇ alkylene or unsubstituted C₅ alkylene or unsubstituted C₄ alkylene;
G₃ is HO(CH₂)₂N(CH₃)(CH₂)₂-, HO(CH₂)₂N(CH₂CH₃)(CH₂)₂-, (HO(CH₂)₂) ₂N(CH₂)₂-, CH₃O(CH₂)₂N(CH₃)(CH₂)₂-, (CH₃)₂N(CH₂)₃SC(O)O(CH₂)₂-, (CH₃)₂N(CH₂)₃SC(O)-, CH₃NH(CH₂)₂N(CH₃)(CH₂)₂- or CH₃CH₂NH(CH₂)₂-, preferably HO(CH₂)₂N(CH₃)(CH₂)₂-.
R₁ is C₆₋₂₀ straight chain or branched alkyl, preferably unsubstituted C₈₋₁₂ straight chain alkyl, or unsubstituted C₁₈ branched alkyl, unsubstituted C₁₇ branched alkyl, or unsubstituted C₁₅ branched alkyl, more preferably unsubstituted C₁₁ straight chain alkyl or unsubstituted C₁₀ straight chain alkyl;
R₂ is C₁₂₋₂₅ branched alkyl, preferably unsubstituted C₁₄₋₂₂ branched alkyl, more preferably unsubstituted C₁₇ branched alkyl, unsubstituted C₁₈ branched alkyl, or unsubstituted C₁₅ branched alkyl;

In an embodiment, G₁ is unsubstituted C₅ alkylene, for example, -(CH₂)₅-.

In an embodiment, G₁ is unsubstituted C₃ alkylene, for example, -(CH₂)₃-.

In an embodiment, G₂ is unsubstituted C₇ alkylene, for example, -(CH₂)₇-.

In an embodiment, G₂ is unsubstituted C₅ alkylene, for example, -(CH₂)₅-.

In an embodiment, G₂ is unsubstituted C₄ alkylene, for example, -(CH₂)₄-.

In an embodiment, G₃ is HO(CH₂)₂N(CH₃)(CH₂)₂-.

In an embodiment, G₃ is HO(CH₂)₂N(CH₂CH₃)(CH₂)₂-.

In an embodiment, G₃ is (HO(CH₂)₂)₂N(CH₂)₂-.

In an embodiment, G₃ is CH₃O(CH₂)₂N(CH₃)(CH₂)₂-.

In an embodiment, G₃ is (CH₃)₂N(CH₂)₃SC(O)O(CH₂)₂-.

In an embodiment, G₃ is (CH₃)₂N(CH₂)₃SC(O)-.

In an embodiment, G₃ is CH₃NH(CH₂)₂N(CH₃)(CH₂)₂-.

In an embodiment, G₃ is CH₃CH₂NH(CH₂)₂-.

In an embodiment, R₁ is unsubstituted C₈₋₁₂ straight chain alkyl, preferably unsubstituted C₁₁ straight chain alkyl, i.e., -(CH₂)₁₀CH₃.

In an embodiment, R₁ is unsubstituted C₈₋₁₂ straight chain alkyl, preferably unsubstituted C₁₀ straight chain alkyl, i.e., -(CH₂)₉CH₃.

In an embodiment, R₁ is unsubstituted C₁₈ branched alkyl, unsubstituted C₁₇ branched alkyl, or unsubstituted C₁₅ branched alkyl. For example, R₁ is:

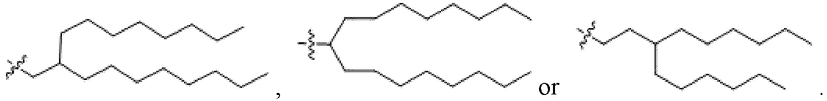

In an embodiment, R₂ is unsubstituted C₁₄₋₂₂ branched alkyl, preferably unsubstituted C₁₇ branched alkyl, unsubstituted C₁₈ branched alkyl, or unsubstituted C₁₅ branched alkyl. For example, R₂ is:

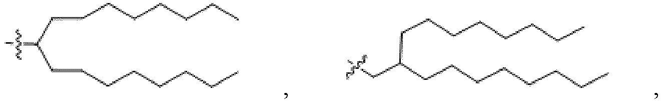

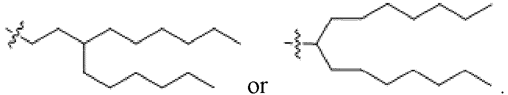

In an embodiment, G₁ is -(CH₂)₅-, G₂ is -(CH₂)₇-, G₃ is HO(CH₂)₂N(CH₃)(CH₂)₂-, R₁ is -(CH₂)₁₀CH₃, and R₂ is:

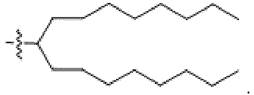

In an embodiment, G₁ is -(CH₂)₅-, G₂ is -(CH₂)₇-, G₃ is HO(CH₂)₂N(CH₃)(CH₂)₂-, R₁ is -(CH₂)₁₀CH₃, and R₂ is:

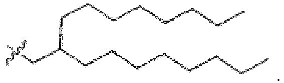

In an embodiment, G₁ is -(CH₂)₅-, G₂ is -(CH₂)₅-, G₃ is HO(CH₂)₂N(CH₃)(CH₂)₂-, R₁ is: 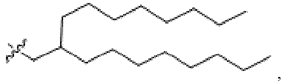 and R₂ is:

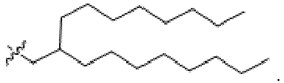

In an embodiment, G₁ is -(CH₂)₅-, G₂ is -(CH₂)₇-, G₃ is HO(CH₂)₂N(CH₃)(CH₂)₂-, R₁ is -(CH₂)₁₀CH₃, and R₂ is:

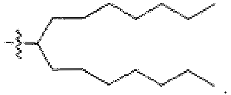

In an embodiment, G₁ is -(CH₂)₃-, G₂ is -(CH₂)₅-, G₃ is HO(CH₂)₂N(CH₃)(CH₂)₂-, R₁ is -(CH₂)₉CH₃, and R₂ is:

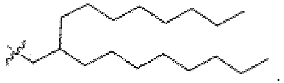

In an exemplary embodiment, the compound is selected from the group consisting of the following compounds or N-oxides, solvates, pharmaceutically acceptable salts or stereoisomers thereof: 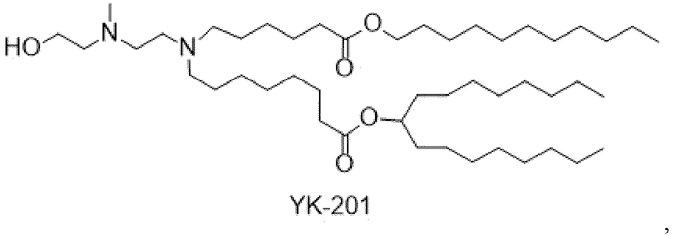 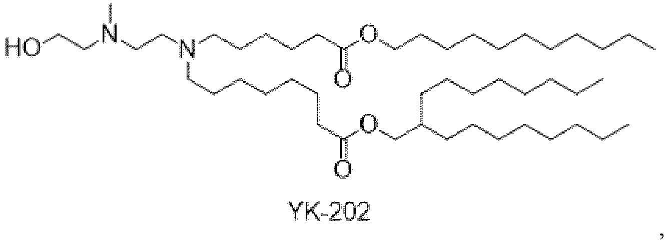 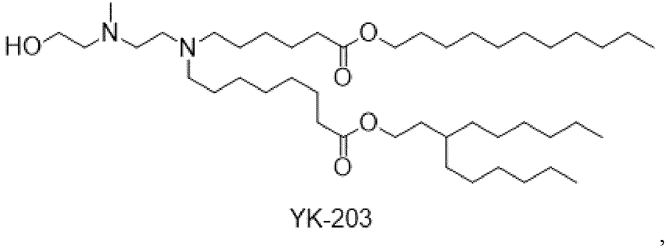 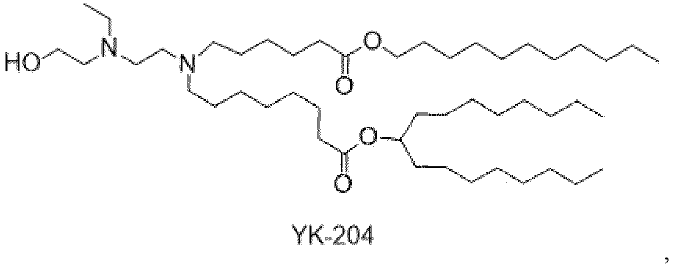 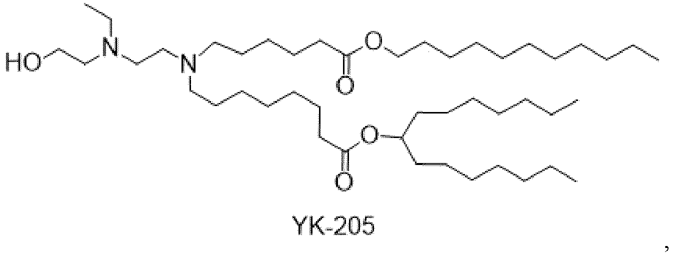 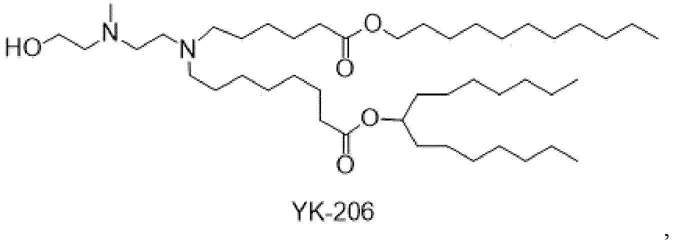 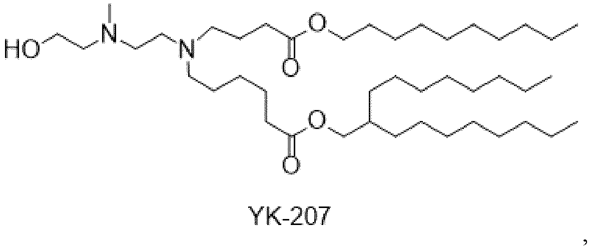 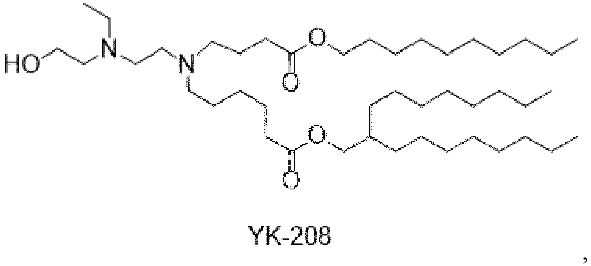 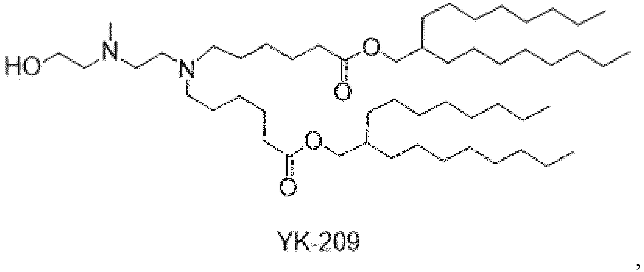 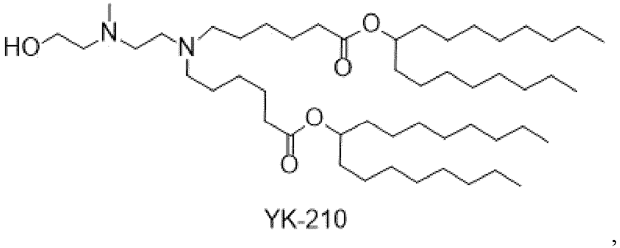 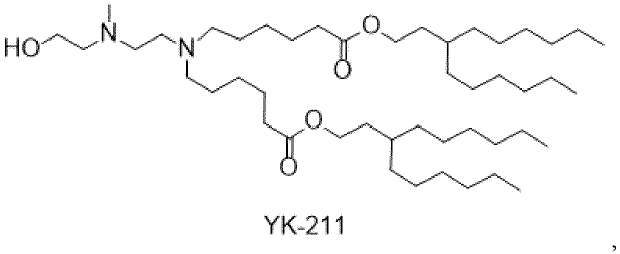 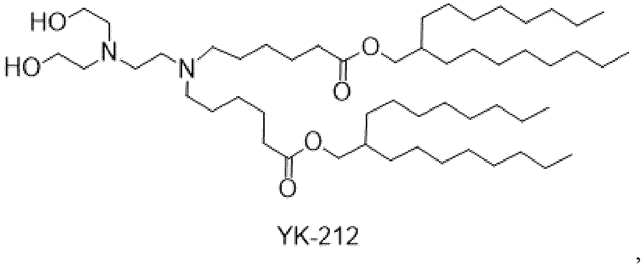 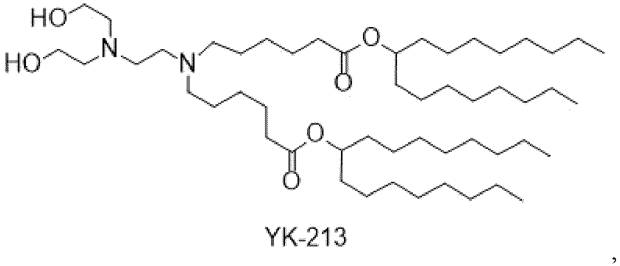 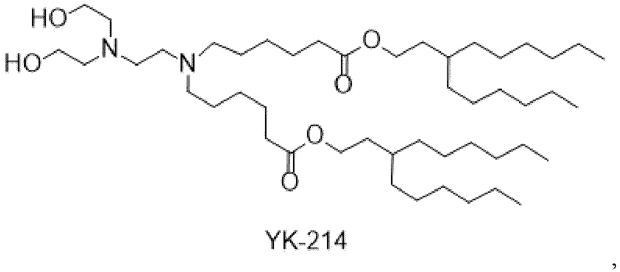 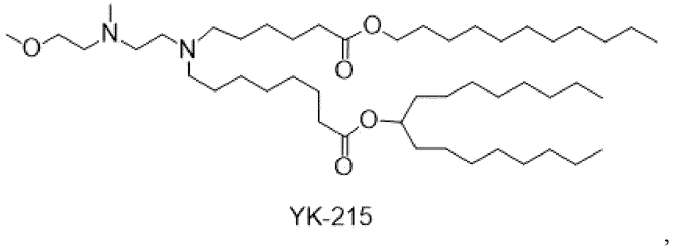 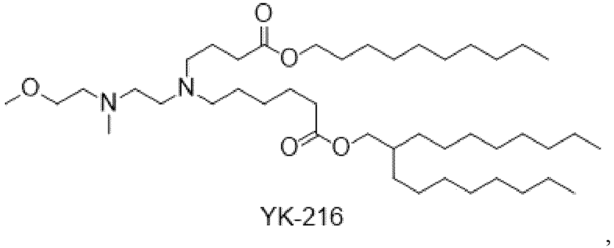 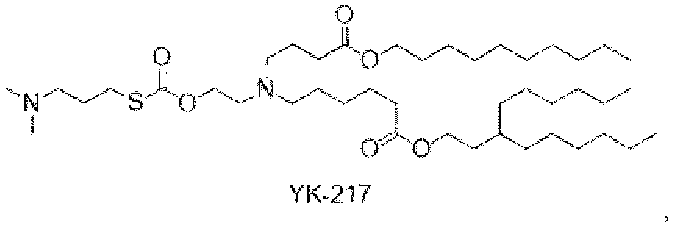 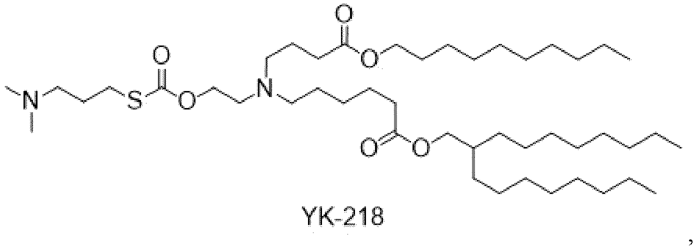 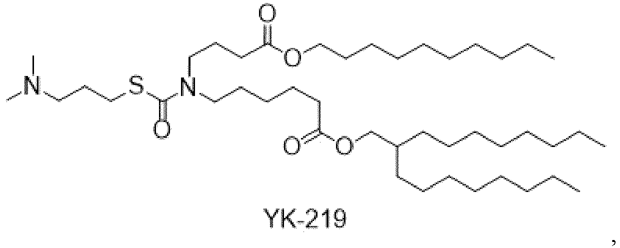 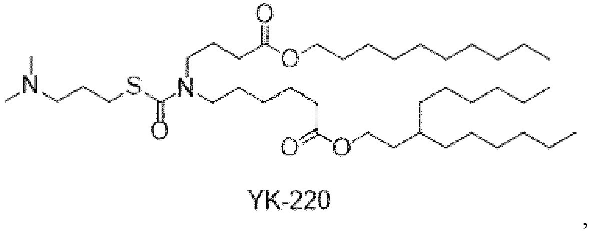 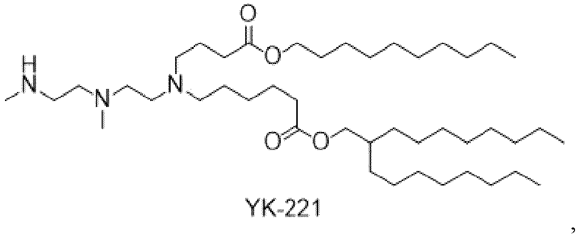 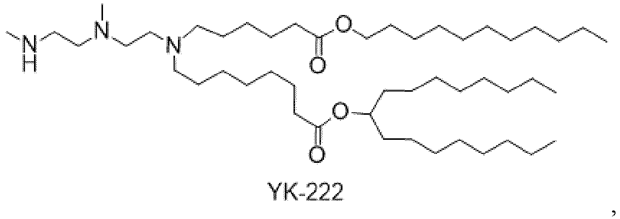 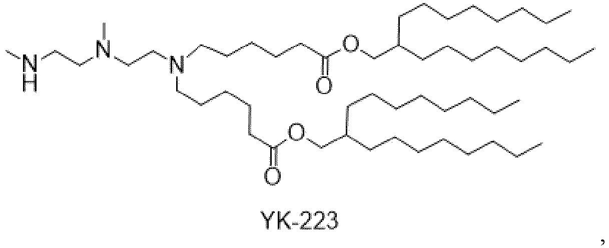 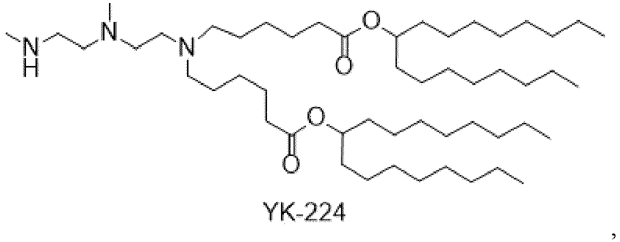 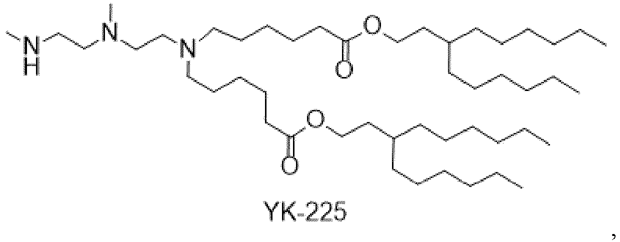 and

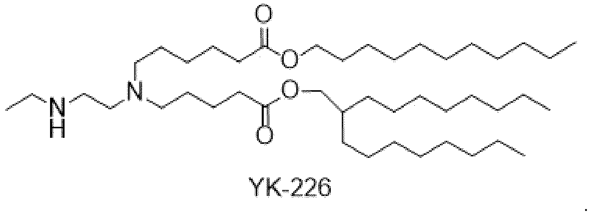

Another aspect of the present disclosure provides a composition comprising a carrier, wherein the carrier comprises a cationic lipid, and the cationic lipid comprises the above-mentioned compound of Formula (I) or an N-oxide, a solvate, a pharmaceutically acceptable salt or a stereoisomer thereof.

In an embodiment, the composition is a nanoparticle formualtion. The average size of the nanoparticle formulation is 10nm to 300nm, preferably 90nm to 260nm; and the polydispersity coefficient of the nanoparticle formulation is ≤50%, preferably ≤40%, and more preferably ≤30%.

### Cationic lipids

In an embodiment of the composition/carrier of the present disclosure, the cationic lipid is selected from the group consisting of a compound of Formula (I), a N-oxide, solvate, pharmaceutically acceptable salt, stereoisomer thereof and a combination thereof. In an embodiment, the cationic lipid is selected from a compound of Formula (I) above. For example, the cationic lipids is selected from the group consisting of compounds YK-201, YK-202, YK-203, YK-204, YK-205, YK-206, YK-207, YK-208, YK-209, YK-210, YK-211, YK-212, YK-213, YK-214, YK-215. YK-216, YK-217, YK-218, YK-219, YK-220, YK-221, YK-222, YK-223, YK-224, YK-225 and YK-226. In a preferred embodiment, the cationic lipid is compound YK-201; in another preferred embodiment, the cationic lipid is compound YK-202; in another preferred embodiment, the cationic lipid is compound YK-209; in another preferred embodiment, the cationic lipid is compound YK-206; in another preferred embodiment, the cationic lipid is compound YK-207.

In another embodiment of the composition/carrier of the present disclosure, the cationic lipid comprises: (a) a compound selected from the group consisting of the above-mentioned compound of Formula (I), or an N-oxide, a solvate, a pharmaceutically acceptable salt, a stereoisomer thereof and a combination thereof; (b) one or more other ionizable lipid compounds different from (a). The cationic lipid compound (b) can be a commercially available cationic lipid, or a cationic lipid compound reported in the literature. For example, the cationic lipid compound (b) can be SM-102 (compound 25 in WO2017049245A2), or ALC-0315 (compound 3 in CN108368028B), or compound 21 or compound 23 in WO2021055833, or HHMA (compound 1 in CN112979483B).

In an embodiment, a molar ratio of the cationic lipid to the carrier is 25% to 75%, such as 30%, 40%, 49%, 55%, 60%, 65%, or 70%.

The carrier can be used to deliver active ingredients such as therapeutic or prophylactic agents. The active ingredient can be encapsulated in the carrier or combined with the carrier.

For example, the therapeutic agent or prophylactic agent is selected from the group consisting of a nucleic acid molecule, a small molecule compound, a polypeptide, a protein and a combination thereof. The nucleic acid includes, but is not limited to, single-stranded DNA, double-stranded DNA and RNA. Suitable RNA includes, but is not limited to, small interfering RNA (siRNA), asymmetric interfering RNA (aiRNA), microRNA (miRNA), Dicer-substrate RNA (dsRNA), small hairpin RNA (shRNA), messenger RNA (mRNA) and a mixture thereof.

### Neutral lipids

The carrier may comprise a neutral lipid. In the present disclosure, a neutral lipid refers to a lipid that is uncharged or exists in the form of a zwitterion at a selected pH value and plays an auxiliary role. The neutral lipid may adjust the fluidity of the nanoparticles to a lipid bilayer structure and improve efficiency by promoting lipid phase transition, and may also affect the specificity to the target organ.

In an embodiment, a molar ratio of the cationic lipid to the neutral lipid is about 1:1 to 15:1, such as about 14: 1, 13:1, 12:1, 11:1, 10:1, 9:1, 8:1, 7:1, 6:1, 5:1, 4:1, 3:1 or 2:1. In a preferred embodiment, a molar ratio of the cationic lipid to the neutral lipid is about 4.5:1. In another preferred embodiment, a molar ratio of the cationic lipid to the neutral lipid is about 4.9:1.

For example, the neutral lipids may comprise one or more of phosphatidylcholine, phosphatidylethanolamine, sphingomyelin, ceramide, sterols, and derivatives thereof.

The carrier component of the composition comprising cationic lipids can include one or more neutral lipids-phospholipids, such as one or more (poly) unsaturated lipids. Phospholipids can be assembled into one or more lipid bilayers. In general, phospholipids can comprise a phospholipid moiety and one or more fatty acid moieties.

The neutral lipid moiety can be selected from the non-limiting group consisting of: phosphatidylcholine, phosphatidylethanolamine, phosphatidylglycerol, phosphatidylserine, phosphatidic acid, 2-lysophosphatidylcholine and sphingomyelin. The fatty acid moiety can be selected from the non-limiting group consisting of: lauric acid, myristic acid, myristoleic acid, palmitic acid, palmitoleic acid, stearic acid, oleic acid, linoleic acid, α-linolenic acid, erucic acid, phytanic acid, arachidic acid, arachidonic acid, eicosapentaenoic acid, behenic acid, docosapentaenoic acid and docosahexaenoic acid. Also encompassed are non-natural species including natural species with modification and substitution, the modification and substitution include branching, oxidation, cyclization and alkynes. For example, phospholipids can be functionalized with one or more alkynes (e.g., one or more double bonds are replaced by triple bond in alkenyl) or cross-linked with the one or more alkynes. Under appropriate reaction conditions, alkynyl may undergo copper-catalyzed cycloaddition reaction when exposed to azide. These reactions can be used to functionalize the lipid bilayer of the composition to facilitate membrane permeation or cellular recognition, or to couple the composition to useful components such as targeting or imaging moieties (e.g., dyes).

The neutral lipid useful in these compositions can be selected from the following non-limiting group consisting of 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesteryl hemisuccinyl-sn-glycero-3-phosphocholine (OChemsPC), 1-hexadecyl-sn-glycero-3-phosphocholine (C16 Lyso PC), 1,2-dialinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dialinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoylphosphatidylglycerol (DPPG), palmitoyloleoylphosphatidylethanolamine (POPE), distearoyl-phosphatidyl-ethanolamine (DSPE), dipalmitoylphosphatidylethanolamine (DPPE), dimyristoylphosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyloleoylphosphatidylcholine, lysophosphatidylcholine, lysophosphatidylethanolamine (LPE) and a mixture thereof.

In some embodiments, the neutral lipid includes DSPC. In certain embodiments, the neutral lipid includes DOPE. In some embodiments, the neutral lipid includes both DSPC and DOPE.

### Structured lipids

The carrier of the composition comprising cationic lipids may further include one or more structured lipids. The structured lipids in the present disclosure refer to lipids that enhance the stability of nanoparticles by filling the gaps between lipids.

In an embodiment, a molar ratio of the cationic lipid to the structured lipid is about 0.6:1 to 3:1, for example, about 1.0:1, 1.1:1, 1.2:1, 1.3:1, 1.4:1, 1.5: 1, 1.6:1, 1.7:1, 1.8:1, 1.9:1, 2.0:1.

The structured lipid can be selected from, but is not limited to, the group consisting of cholesterol, non-sterols, sitosterol, ergosterol, campesterol, stigmasterol, brassicasterol, tomatine, tomatine, ursolic acid, α-tocopherol, corticosteroids, and a mixture thereof. In some embodiments, the structured lipid is cholesterol. In some embodiments, the structured lipid includes cholesterol and corticosteroids (such as prednisolone, dexamethasone, prednisone, and hydrocortisone) or a combination thereof.

### Polymer-conjugated lipids

The carrier of the composition comprising cationic lipids can also comprise one or more polymer-conjugated lipids. The molar ratio of the polymer-conjugated lipid to the carrier is 0.5% to 10%, such as 1%, 2%, 3%, 4%, 5%, preferably 1.5%. Polymer-conjugated lipids mainly refer to lipids modified by polyethylene glycol (PEG). Hydrophilic PEG stabilizes LNP, regulates nanoparticle size by limiting lipid fusion, and increases the half-life of nanoparticles by reducing nonspecific interactions with macrophages.

In an embodiment, the polymer-conjugated lipid is selected from the group consisting of: PEG-modified phosphatidylethanolamine, PEG-modified phosphatidic acid, PEG-modified ceramide, PEG-modified dialkylamine, PEG-modified diacylglycerol, PEG-modified dialkylglycerol, and a combination thereof. The molecular weight of PEG for PEG modification is usually 350-5000Da.

For example, the polymer-conjugated lipid is selected from the group consisting of: distearoylphosphatidylethanolamine polyethylene glycol 2000 (DSPE-PEG2000), dimyristoylglycerol-3-methoxy polyethylene glycol 2000 (DMG-PEG2000) and methoxy polyethylene glycol ditetradecanoyl acetamide (ALC-0159).

In an embodiment of the composition/carrier of the present disclosure, the polymer-conjugated lipid is DMG-PEG2000.

In an embodiment of the composition/carrier of the present disclosure, the carrier comprises a neutral lipid, a structured lipid and a polymer-conjugated lipid, and a molar ratio of the cationic lipid, the neutral lipid, the structured lipid and the polymer-conjugated lipid is (25 to 75): (5 to 25): (15 to 65): (0.5 to 10), for example (35 to 49): (7.5 to 15): (35 to 55): (1 to 5).

In an embodiment of the composition/carrier of the present disclosure, the carrier comprises a neutral lipid, a structured lipid and a polymer-conjugated lipid, and a molar ratio of the cationic lipid, the neutral lipid, the structured lipid and the polymer-conjugated lipid is 49: 10: 39.5: 1.5 or 45:10:43.5:1.5, preferably 45:10:43.5:1.5.

### Therapeutic and/or prophylactic agents

The composition may comprise one or more therapeutic and/or prophylactic agents. In an embodiment, a mass ratio of the carrier to the therapeutic or prophylactic agent is 10:1 to 30:1, for example, 12: 1, 13:1, 14:1, 15:1, 16:1, 17: 1, 18:1, 19:1, 20:1, 21:1, 22:1, 23:1, 24:1, 25:1.

In an embodiment, a mass ratio of the carrier to the therapeutic agent or prophylactic agent is 12.5: 1 to 20: 1, preferably 15: 1.

The therapeutic agent or prophylactic agent includes, but is not limited to, one or more of a nucleic acid molecule, a small molecule compound, a polypeptide or a protein.

For example, the therapeutic or prophylactic agent is a vaccine or a compound capable of eliciting an immune response.

The carrier of the present disclosure can deliver therapeutic agents and/or prophylactic agents to mammalian cells or organs. Therefore, the present disclosure also provides a method for treating a disease or disorder in mammals in need thereof, comprising administering a composition comprising a therapeutic agent and/or prophylactic agent to a mammal and/or contacting mammalian cells with the composition.

Therapeutic and/or prophylactic agents include biologically active substances and are alternatively referred to as "active agents". Therapeutic and/or prophylactic agents can be substances that cause desired changes in cells or organs or other body tissues or systems after delivery to cells or organs. Such substances can be used to treat one or more diseases, disorders or conditions. In some embodiments, therapeutic and/or prophylactic agents are small molecule drugs that can be used to treat specific diseases, disorders or conditions. Examples of drugs that can be used for compositions include, but are not limited to, anti-neoplastic agents (e.g., vincristine, doxorubicin, mitoxantrone, camptothecin, cisplatin, bleomycin, cyclophosphamide, methotrexate and streptozotocin), anti-tumor agents (e.g., actinomycin D, vincristine, vinblastine, cytosine arabinoside, anthracycline, alkylating agents, platinum compounds, antimetabolites, and nucleoside analogs, such as methotrexate and purine and pyrimidine analogs), anti-infectives, local anesthetics (e.g., dibucaine and chlorpromazine), β-adrenergic blocking agents (e.g., propranolol, timolol, and labetalol), antihypertensives (e.g., clonidine and hydralazine), antidepressants (e.g., imipramine, amitriptyline, and doxepin), anticonvulsants (e.g., phenytoin), antihistamines (e.g., diphenhydramine, chlorpheniramine, and promethazine), antibiotics/antibacterials (e.g., gentamycin, ciprofloxacin, and cefoxitin), antifungals (e.g., miconazole, terconazole, econazole, isoconazole, butaconazole, clotrimazole, itraconazole, nystatin, naftifine, and amphotericin B), antiparasitics, hormones, hormone antagonists, immunomodulators, neurotransmitter antagonists, antiglaucoma agents, vitamins, sedatives, and imaging agents.

In some embodiments, the therapeutic and/or prophylactic agent is a cytotoxin, a radioactive ion, a chemotherapeutic agent, a vaccine, a compound that causes an immune response, and/or another therapeutic and/or prophylactic agent. Cytotoxins or cytotoxic agents include any agent that is harmful to cells. Examples include, but are not limited to, taxol, cytochalasin B, gramicidin D, ethidium bromide, emetine, mitomycin, etoposide, teniposide, vincristine, vinblastine, colchicine, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, puromycin, maytansinoids such as maytansinol, rachelmycin (CC-1065), and analogs or homologues thereof. Radioactive ions include, but are not limited to, iodine (e.g., iodine-125 or iodine-131), strontium-89, phosphorus, palladium, cesium, iridium, phosphate, cobalt, yttrium-90, samarium-153, and praseodymium. Vaccines include compounds and formulations that can provide immunity for one or more conditions related to infectious diseases such as influenza, measles, human papillomavirus (HPV), rabies, meningitis, pertussis, tetanus, plague, hepatitis and tuberculosis, and can include mRNA encoding infectious disease-derived antigens and/or epitopes. Vaccines can also include compounds and formulations that guide the immune response for cancer cells and can include mRNA encoding tumor cell-derived antigens, epitopes and/or new epitopes. Compounds that cause immune response can include vaccines, corticosteroids (e.g., dexamethasone) and other substances. In some embodiments, the therapeutic and/or prophylactic agent are vaccines and/or compounds that are capable of eliciting an immune response by the intramuscular administration of a composition comprising compounds of Formula (I), (IA), (IB), (II), (IIa), (IIb), (IIc), (IId), (IIe), (IIf), (IIg) or (III) (e.g., compounds 3, 18, 20, 25, 26, 29, 30, 60, 108-112 or 122). Other therapeutic and/or prophylactic agents include, but are not limited to, antimetabolites (e.g., methotrexate, 6-mercaptopurine, 6-thioguanine, cytarabine, and 5-fluorouracil dacarbazine), alkylating agents (e.g., mechlorethamine, thiotepa, chlorambucil, razithromycin (CC-1065), melphalan, carmustine (BSNU), lomustine (CCNU), cyclophosphamide, busulfan, dibromomannitol, streptozotocin, mitomycin C, and cis-dichlorodiamine platinum (II) (DDP), cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and antimitotic agents (e.g., vincristine, vinblastine, taxol, and maytansine).

In other embodiments, the therapeutic and/or prophylactic agent is a protein. The therapeutic proteins that can be used in the nanoparticles of the present disclosure include, but are not limited to, gentamicin, amikacin, insulin, erythropoietin (EPO), granulocyte colony stimulating factor (G-CSF), granulocyte-macrophage colony stimulating factor (GM-CSF), factor VIR, luteinizing hormone releasing hormone (LHRH) analogs, interferon, heparin, hepatitis B surface antigen, typhoid vaccine, and cholera vaccine.

In some embodiments, the therapeutic agent is a polynucleotide or nucleic acid (e.g., ribonucleic acid or deoxyribonucleic acid). The broadest meaning of the term "polynucleotide" includes any compound and/or substance that is an oligonucleotide chain or can be incorporated into an oligonucleotide chain. Exemplary polynucleotides used according to the present disclosure include, but are not limited to, one or more of the following: deoxyribonucleic acid (DNA); ribonucleic acid (RNA), including messenger mRNA (mRNA), a hybrid thereof; RNAi inducing factor; RNAi factor; siRNA; shRNA; miRNA; antisense RNA; ribozyme; catalytic DNA; RNA that induces triple helix formation; aptamer, etc. In some embodiments, the therapeutic agent and/or prophylactic agent is RNA. The RNA that can be used in the compositions and methods described herein can be selected from, but is not limited to, the group consisting of: shortmer, antagomir, antisense RNA, ribozyme, small interfering RNA (siRNA), asymmetric interfering RNA (aiRNA), microRNA (miRNA), Dicer-substrate RNA (dsRNA), small hairpin RNA (shRNA), transfer RNA (tRNA), messenger RNA (mRNA) and a mixture thereof. In certain embodiments, the RNA is mRNA.

In certain embodiments, the therapeutic and/or prophylactic agent is mRNA. mRNA can encode any polypeptide of interest, including any natural or non-natural polypeptide or otherwise modified polypeptide. The polypeptide encoded by mRNA can have any size and can have any secondary structure or activity. In some embodiments, the polypeptide encoded by mRNA can have a therapeutic effect when expressed in a cell.

In other embodiments, the therapeutic agent and/or prophylactic agent is siRNA. siRNA can selectively reduce the expression of a gene of interest or down-regulate the expression of the gene. For example, the siRNA selected can silence the gene relevant to a specific disease, disorder or condition after a composition comprising the siRNA is administered to a subject in need thereof. siRNA can include a sequence complementary to the mRNA sequence of a gene or protein of interest to encode. In some embodiments, the siRNA can be an immunomodulatory siRNA.

In certain embodiments, the therapeutic and/or prophylactic agent is sgRNA and/or cas9 mRNA. sgRNA and/or cas9 mRNA can be used as a gene editing tool. For example, the sgRNA-cas9 complex can affect the mRNA translation of a cell gene.

In some embodiments, the therapeutic agent and/or prophylactic agent is shRNA or its encoding carrier or plasmid. shRNA can be produced inside the target cell after the appropriate construct is delivered to the nucleus. The construct and mechanism associated with shRNA are well-known in the relevant field.

### Disease or condition

The composition/carrier of the present disclosure can deliver a therapeutic agent or a prophylactic agent to a subject or patient. The therapeutic agent or prophylactic agent includes, but is not limited to, one or more of a nucleic acid molecule, a small molecule compound, a polypeptide or a protein. Therefore, the composition of the present disclosure can be used to prepare nucleic acid drugs, gene vaccines, small molecule drugs, polypeptides or protein drugs. Due to the wide variety of the above-mentioned therapeutic agents or prophylactic agents, the composition of the present disclosure can be used to treat or prevent a variety of diseases or conditions.

In an embodiment, the disease or condition is characterized by dysfunctional or abnormal protein or polypeptide activity.

For example, the disease or disorder is selected from the group consisting of infectious diseases, cancer and proliferative diseases, genetic diseases, autoimmune diseases, diabetes, neurodegenerative diseases, cardiovascular and renal vascular diseases, and metabolic diseases.

In an embodiment, the infectious disease is selected from the group consisting of diseases caused by coronavirus, influenza virus, or HIV virus, pediatric pneumonia, Rift Valley fever, yellow fever, rabies, and various herpes.

### Other components

The composition may comprise one or more components other than those described in the preceding section. For example, the composition may comprise one or more hydrophobic small molecules, such as vitamins (eg, vitamin A or vitamin E) or sterols.

The composition may also include one or more permeability enhancing molecules, carbohydrates, polymers, surface modifiers or other components. The permeability enhancing molecules may be, for example, molecules described in U.S. Patent Application Publication No. 2005/0222064. The carbohydrates may include simple sugars (e.g., glucose) and polysaccharides (e.g., glycogen and its derivatives and analogs).

Surface modifiers may include, but are not limited to, anionic proteins (e.g., bovine serum albumin), surfactants (e.g., cationic surfactants such as dimethyldioctadecyl ammonium bromide), sugars or sugar derivatives (e.g., cyclodextrins), nucleic acids, polymers (e.g., heparin, polyethylene glycol, and poloxamer), mucolytic agents (e.g., acetylcysteine, mugwort, bromelain, papain, clerodendrum, bromhexine, carbocisteine, eprazinone, mesna, ambroxol, sobrerol, domiodol, letosteine, stepronin, tiopronin, gelsolin, thymosin β4, dornase α, neltenexine and erdosteine) and DNA enzymes (such as rhDNA enzyme). The surface modifier can be disposed within and/or on the surface of the nanoparticles of the composition (e.g., by coating, adsorption, covalent attachment or other methods).

The composition can also comprise one or more functionalized lipids. For example, lipids can be functionalized with alkynyl, which may undergo cycloaddition reaction when exposed to an azide under appropriate reaction conditions. Specifically, the lipid bilayer can be functionalized with one or more groups that can effectively promote membrane penetration, cell recognition or imaging in this way. The surface of the composition can also be coupled to one or more useful antibodies. Functional groups and conjugates that can be used for targeted cell delivery, imaging and membrane penetration are well known in the art.

In addition to these components, the composition can comprise any substance that can be used in the pharmaceutical composition. For example, the composition can comprise one or more pharmaceutically acceptable excipients or auxiliary ingredients, such as but not limited to one or more solvents, dispersion media, diluents, dispersing aids, suspension aids, granulation aids, disintegrants, fillers, glidants, liquid vehicles, adhesives, surfactants, isotonic agents, thickeners or emulsifiers, buffering agents, lubricants, oils, preservatives, flavoring agents, coloring agents, etc. Excipients include such as starch, lactose or dextrin. Pharmaceutically acceptable excipients are well known in the art (see, for example, Remington's The Science and Practice of Pharmacy, 21st edition, A. R. Gennaro; Lippincott, Williams & Wilkins, Baltimore, MD, 2006).

Examples of diluents may include, but are not limited to, calcium carbonate, sodium carbonate, calcium phosphate, dicalcium phosphate, calcium sulfate, calcium hydrogen phosphate, sodium phosphate, lactose, sucrose, cellulose, microcrystalline cellulose, kaolin, mannitol, sorbitol, inositol, sodium chloride, dry starch, corn starch, powdered sugar, and/or combinations thereof.

In some embodiments, a composition comprising one or more lipids described herein may also comprise one or more adjuvants, such as glucopyranosyl lipid adjuvant (GLA), CpG oligodeoxyribonucleotides (e.g., class A or class B), poly (I:C), aluminum hydroxide, and Pam3CSK4.

The composition of the present disclosure can be made into a formulation in the form of solid, semisolid, liquid or gas, such as tablets, capsules, ointments, elixirs, syrups, solutions, emulsions, suspensions, injections, aerosols. The composition of the present disclosure can be prepared by methods well known in the pharmaceutical field. For example, a sterile injection solution can be prepared by mixing the required amount of therapeutic agent or prophylactic agent with the required various other ingredients mentioned above into a suitable solvent such as sterile distilled water, and then filtering and sterilizing. Surfactants can also be added to promote the formation of uniform solutions or suspensions.

For example, the compositions of the invention can be administered intravenously, intramuscularly, intradermally, subcutaneously, intranasally, or by inhalation. In an embodiment, the composition is administered subcutaneously.

The compositions of the present disclosure are administered in a therapeutically effective amount, which may vary not only with the specific agent selected, but also with the route of administration, the nature of the disease being treated, and the age and condition of the patient, and may ultimately be determined at the discretion of the attending physician or clinician. For example, a therapeutic or prophylactic agent may be administered to a mammal (e.g., a human) at an amount of about 0.001 mg/kg to about 10 mg/kg.

The present disclosure includes but is not limited to the following embodiments:
1. A compound of Formula (I) 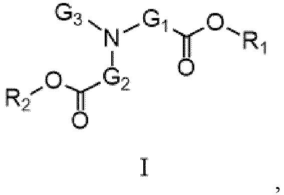 or an N-oxide, a solvate, a pharmaceutically acceptable salt or a stereoisomer thereof, wherein
   G₁ is C₁₋₆ alkylene;
   G₂ is C₂₋₈ alkylene;
   R₁ is C₆₋₂₀ straight chain or branched alkyl;
   R₂ is C₁₂₋₂₅ branched alkyl;
   G₃ is HO(CH₂)₂N(CH₃)(CH₂)₂-, HO(CH₂)₂N(CH₂CH₃)(CH₂)₂-, (HO(CH₂)₂)₂N(CH₂)₂-, CH₃O(CH₂)₂N(CH₃)(CH₂)₂-, (CH₃)₂N(CH₂)₃SC(O)O(CH₂)₂-, (CH₃)₂N(CH₂)₃SC(O)-, CH₃NH(CH₂)₂N(CH₃)(CH₂)₂- or CH₃CH₂NH(CH₂)₂-.
2. The compound of Formula (I), or the N-oxide, solvate, pharmaceutically acceptable salt or stereoisomer thereof according to embodiment 1, wherein G₁ is unsubstituted C₂₋₅ alkylene.
3. The compound of Formula (I), or the N-oxide, solvate, pharmaceutically acceptable salt or stereoisomer thereof according to embodiment 2, wherein G₁ is unsubstituted C₅ alkylene or unsubstituted C₃ alkylene.
4. The compound of Formula (I), or the N-oxide, solvate, pharmaceutically acceptable salt or stereoisomer thereof according to any one of the preceding embodiments, wherein G₂ is unsubstituted C₄₋₇ alkylene.
5. The compound of Formula (I), or the N-oxide, solvate, pharmaceutically acceptable salt or stereoisomer thereof according to embodiment 4, wherein G₂ is unsubstituted C₇ alkylene or unsubstituted C₅ alkylene or unsubstituted C₄ alkylene.
6. The compound of Formula (I), or the N-oxide, solvate, pharmaceutically acceptable salt or stereoisomer thereof according to any one of the preceding embodiments, wherein R₁ is unsubstituted C₈₋₁₂ straight chain alkyl.
7. The compound of Formula (I), or the N-oxide, solvate, pharmaceutically acceptable salt or stereoisomer thereof according to embodiment 6, wherein R₁ is unsubstituted C₁₁ straight chain alkyl or unsubstituted C₁₀ straight chain alkyl.
8. The compound of Formula (I), or the N-oxide, solvate, pharmaceutically acceptable salt or stereoisomer thereof according to any one of embodiments 1 to 5, wherein R₁ is unsubstituted C₁₈ branched alkyl, unsubstituted C₁₇ branched alkyl or unsubstituted C₁₅ branched alkyl.
9. The compound of Formula (I), or the N-oxide, solvate, pharmaceutically acceptable salt or stereoisomer thereof according to embodiment 8, wherein R₁ is 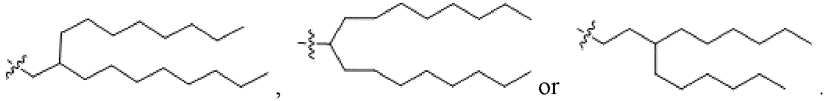
10. The compound of Formula (I), or the N-oxide, solvate, pharmaceutically acceptable salt or stereoisomer thereof according to any one of the preceding embodiments, wherein R₂ is unsubstituted C₁₄₋₂₂ branched alkyl.
11. The compound of Formula (I), or the N-oxide, solvate, pharmaceutically acceptable salt or stereoisomer thereof according to embodiment 10, wherein R₂ is unsubstituted C₁₇ branched alkyl or unsubstituted C₁₈ branched alkyl or unsubstituted C₁₅ branched alkyl.
12. The compound of Formula (I), or the N-oxide, solvate, pharmaceutically acceptable salt or stereoisomer thereof according to embodiment 11, wherein R₂ is 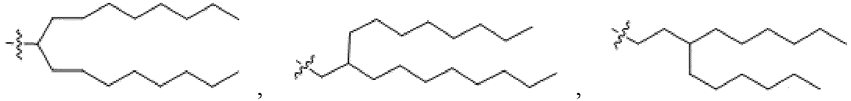 or 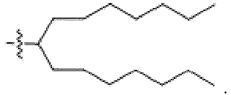
13. The compound of Formula (I), or the N-oxide, solvate, pharmaceutically acceptable salt or stereoisomer thereof according to embodiment 1, wherein the compound of Formula (I) has a structure selected from the group consisting of: 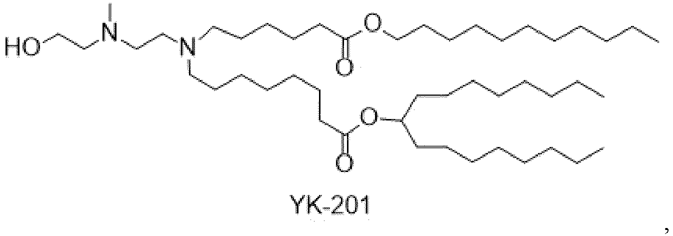 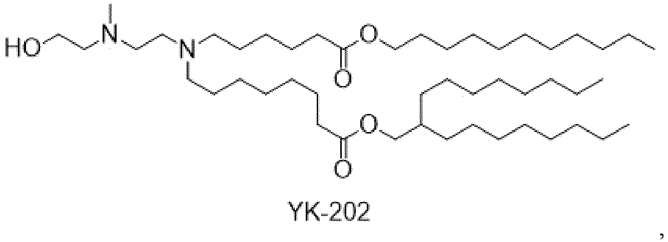 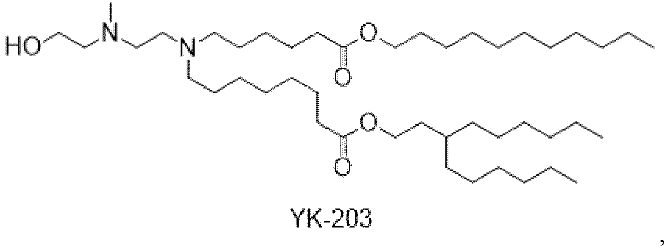 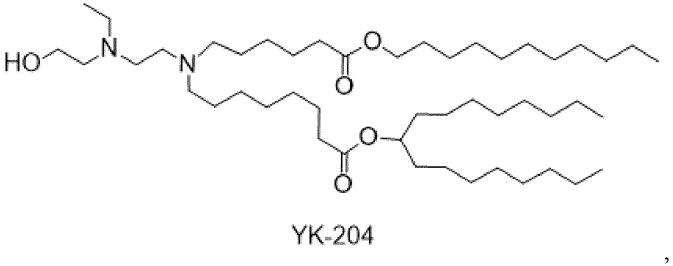 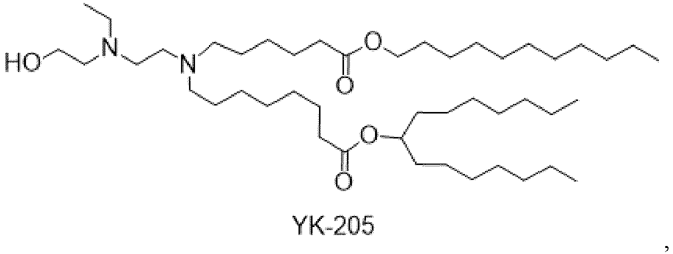 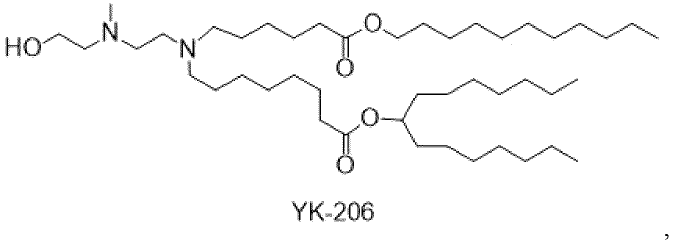 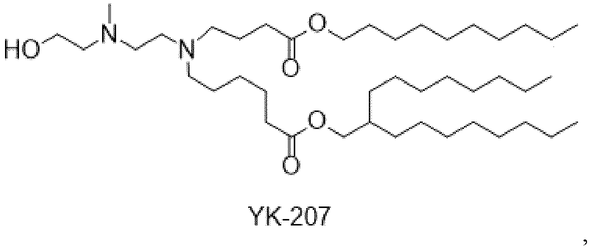 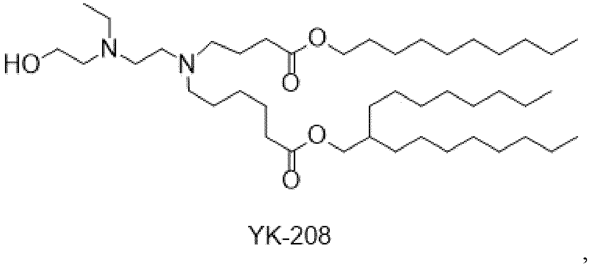 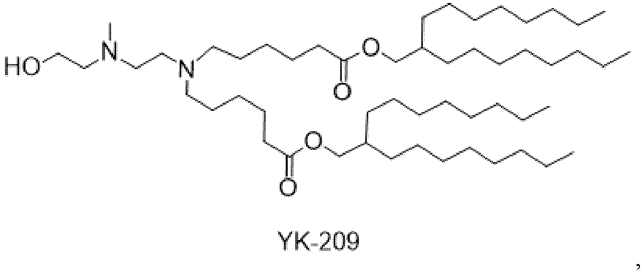 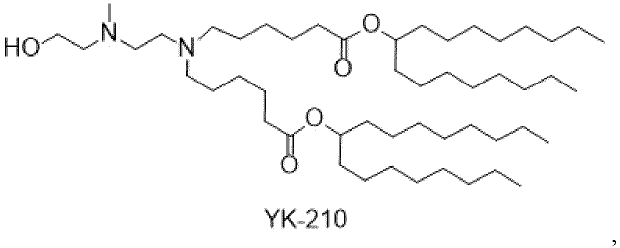 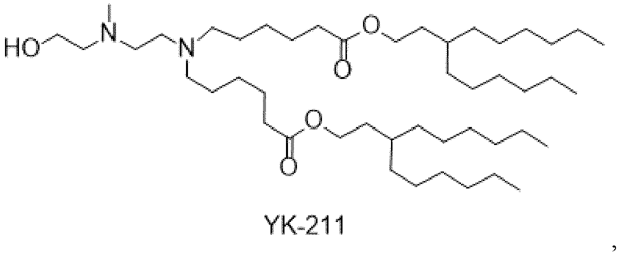 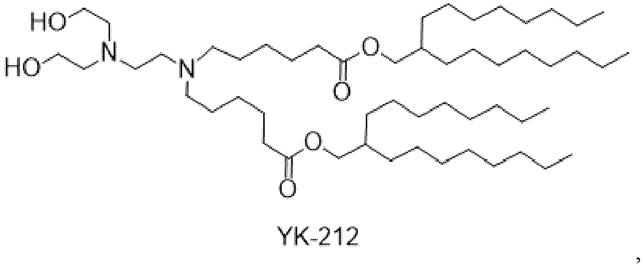 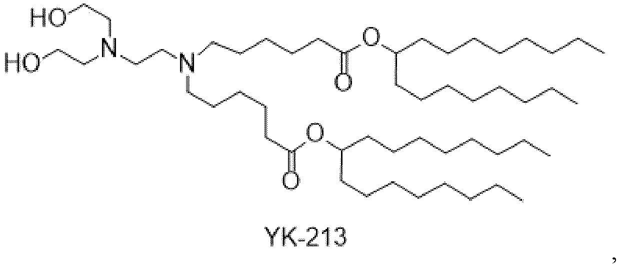 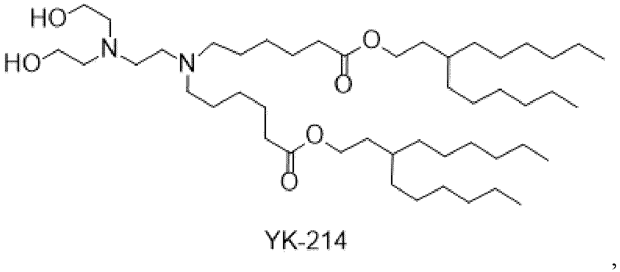 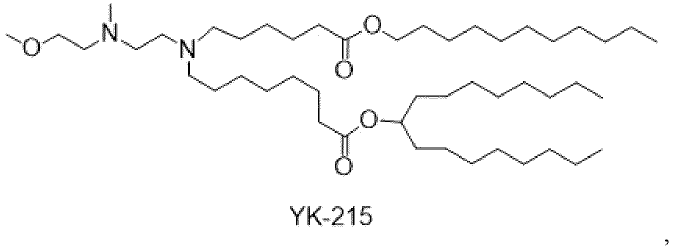 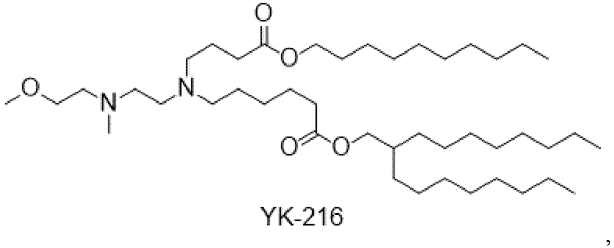 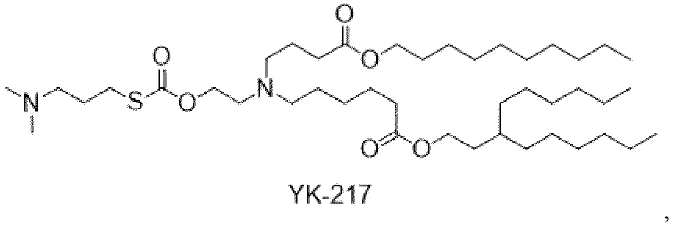 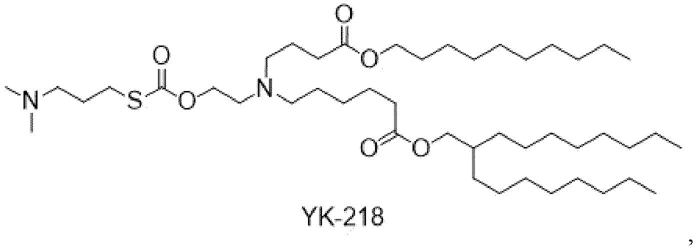 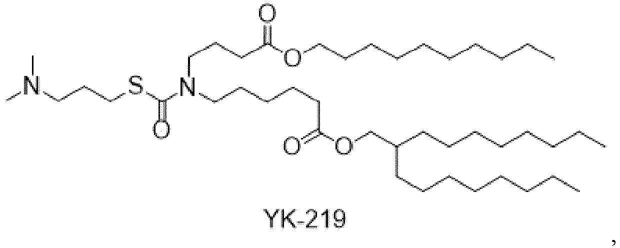 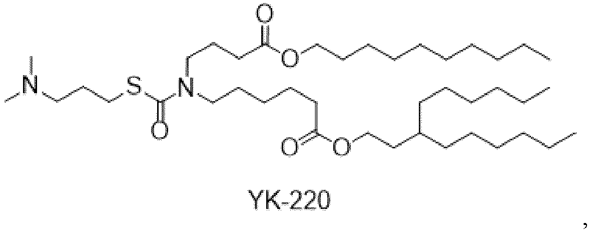 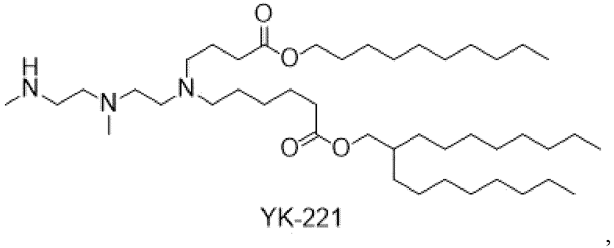 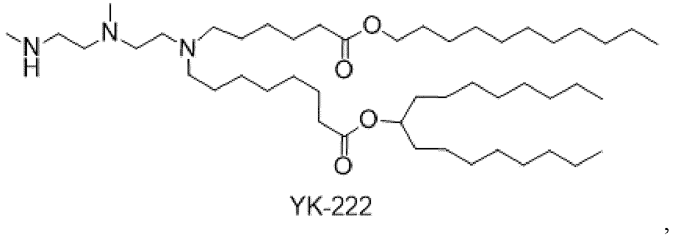 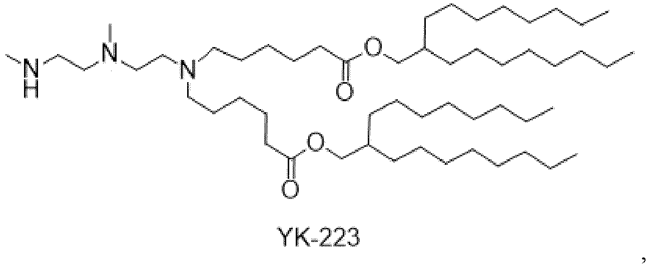 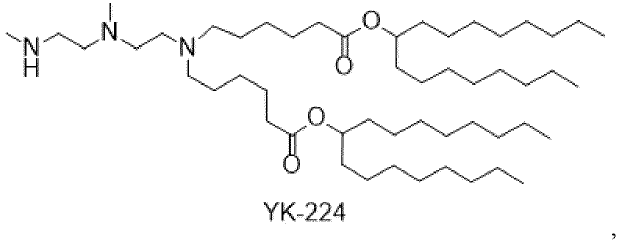 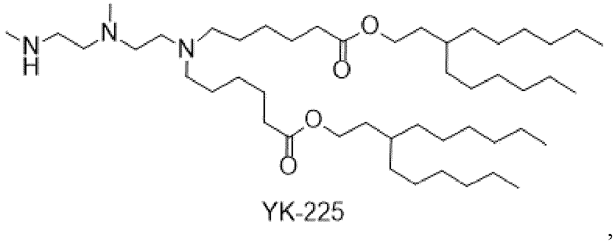 and 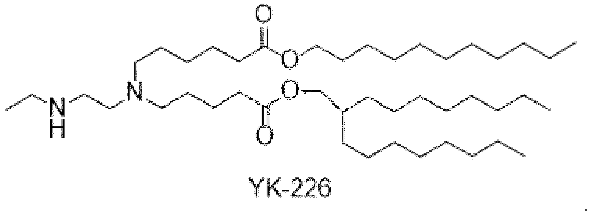
14. The compound of Formula (I), or the N-oxide, solvate, pharmaceutically acceptable salt or stereoisomer thereof according to embodiment 1, wherein the compound of Formula (I) is compound YK-201 having the following structure: 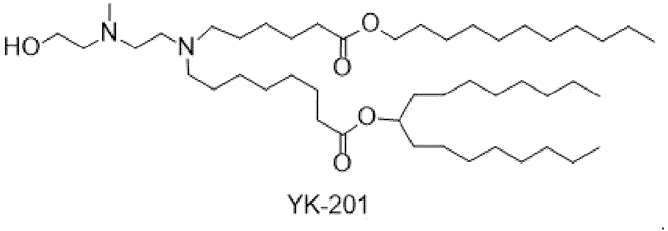
15. The compound of Formula (I), or the N-oxide, solvate, pharmaceutically acceptable salt or stereoisomer thereof according to embodiment 1, wherein the compound of Formula (I) is compound YK-202 having the following structure: 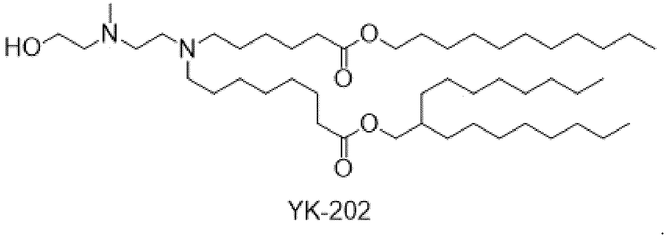
16. The compound of Formula (I), or the N-oxide, solvate, pharmaceutically acceptable salt or stereoisomer thereof according to embodiment 1, wherein the compound of Formula (I) is compound YK-209 having the following structure: 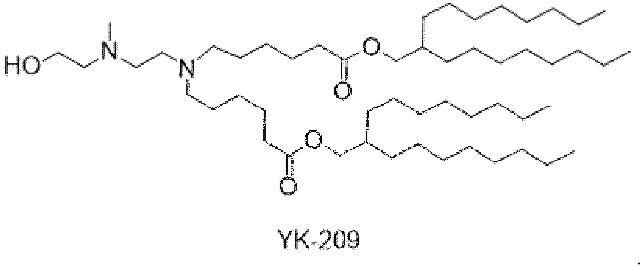
17. The compound of Formula (I), or the N-oxide, solvate, pharmaceutically acceptable salt or stereoisomer thereof according to embodiment 1, wherein the compound of Formula (I) is compound YK-206 having the following structure: 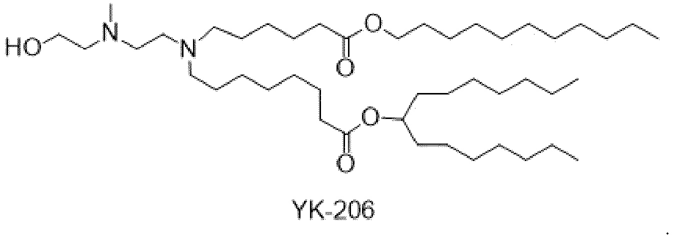
18. The compound of Formula (I), or the N-oxide, solvate, pharmaceutically acceptable salt or stereoisomer thereof according to embodiment 1, wherein the compound of Formula (I) is compound YK-207 having the following structure: 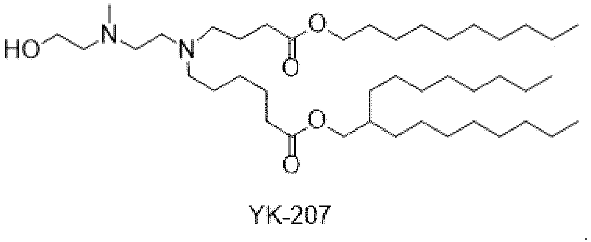
19. A composition comprising a carrier, wherein the carrier comprises a cationic lipid, and the cationic lipid comprises the compound of Formula (I) or the N-oxide, solvate, pharmaceutically acceptable salt or stereoisomer thereof according to any one of the preceding embodiments.
20. The composition according to embodiment 19, wherein a molar ratio of the cationic lipid to the carrier is 25% to 75%.
21. The composition according to any one of embodiments 19 to 20, wherein the carrier further comprises a neutral lipid.
22. The composition according to embodiment 21, wherein a molar ratio of the cationic lipid to the neutral lipid is 1: 1 to 15: 1, preferably 4.5:1.
23. The composition according to embodiments 21 to 22, wherein the neutral lipid is selected from the group consisting of phosphatidylcholine, phosphatidylethanolamine, sphingomyelin, ceramide, sterol, a derivative thereof and a combination thereof.
24. The composition according to embodiment 23, wherein the neutral lipid is selected from one or more of the group consisting of: 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesteryl hemisuccinyl-sn-glycero-3-phosphocholine (OChemsPC), 1-hexadecyl-sn-glycero-3-phosphocholine (C16 Lyso PC), 1,2-dialinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dialinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoylphosphatidylglycerol (DPPG), palmitoyloleoylphosphatidylethanolamine (POPE), distearoyl-phosphatidyl-ethanolamine (DSPE), dipalmitoylphosphatidylethanolamine (DPPE), dimyristoylphosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyloleoylphosphatidylcholine, lysophosphatidylcholine, lysophosphatidylethanolamine (LPE) and a mixture thereof.
25. The composition according to embodiment 24, wherein the neutral lipid is DOPE and/or DSPC.
26. The composition according to any one of embodiments 19 to 25, wherein the carrier further comprises a structured lipid.
27. The composition according to embodiment 26, wherein a molar ratio of the cationic lipid to the structured lipid is 0.6:1 to 3:1.
28. The composition according to any one of embodiments 26 to 27, wherein the structured lipid is selected from the group consisting of: cholesterol, non-sterols, sitosterol, ergosterol, campesterol, stigmasterol, brassicasterol, tomatine, tomatine, ursolic acid, α-tocopherol, corticosteroids, and a mixture thereof.
29. The composition according to embodiment 28, wherein the structured lipid is cholesterol.
30. The composition according to any one of embodiments 19 to 29, wherein the carrier further comprises a polymer-conjugated lipid.
31. The composition according to embodiment 30, wherein a molar ratio of the polymer-conjugated lipid to the carrier is 0.5% to 10%, preferably 1.5%.
32. The composition according to any one of embodiments 30 to 31, wherein the polymer-conjugated lipid is one or more selected from the group consisting of: PEG-modified phosphatidylethanolamine, PEG-modified phosphatidic acid, PEG-modified ceramide, PEG-modified dialkylamine, PEG-modified diacylglycerol, PEG-modified dialkylglycerol, and a combination thereof.
33. The composition according to embodiment 32, wherein the polymer-conjugated lipid is one or more selected from the group consisting of distearoylphosphatidylethanolamine polyethylene glycol 2000 (DSPE-PEG2000), dimyristoylglycerol-3-methoxypolyethylene glycol 2000 (DMG-PEG2000) and methoxypolyethylene glycol ditetradecanoyl acetamide (ALC-0159).
34. The composition according to any one of embodiments 19 to 33, wherein the carrier comprises a neutral lipid, a structured lipid and a polymer-conjugated lipid, and a molar ratio of the cationic lipid, the neutral lipid, the structured lipid and the polymer-conjugated lipid is (25 to 75):(5 to 25):(15 to 65):(0.5 to 10).
35. The composition according to embodiment 34, wherein a molar ratio of the cationic lipid, the neutral lipid, the structured lipid, and the polymer-conjugated lipid is (35 to 49):(7.5 to 15):(35 to 55):(1 to 5).
36. The composition according to embodiment 35, wherein a molar ratio of the cationic lipid, the neutral lipid, the structured lipid, and the polymer-conjugated lipid is 45:10:43.5:1.5.
37. The composition according to any one of embodiments 19 to 36, wherein the composition is a nanoparticle formulation having an average particle size of 10 nm to 300 nm and a polydispersity coefficient of ≤50%.
38. The composition according to embodiment 37, wherein the nanoparticle formulation has an average particle size of 90nm-260nm and a polydispersity coefficient of ≤40%.
39. The composition according to any one of embodiments 19 to 38, wherein the cationic lipid further comprises one or more other ionizable lipid compounds.
40. The composition according to any one of embodiments 19 to 39, further comprising a therapeutic agent or a prophylactic agent.
41. The composition according to embodiment 40, wherein a mass ratio of the carrier to the therapeutic agent or prophylactic agent is 10:1 to 30:1.
42. The composition according to embodiment 41, wherein a mass ratio of the carrier to the therapeutic agent or prophylactic agent is 12.5:1 to 20: 1.
43. The composition according to embodiment 42, wherein a mass ratio of the carrier to the therapeutic agent or prophylactic agent is 15:1.
44. The composition according to any one of embodiments 40 to 43, wherein the therapeutic agent or prophylactic agent comprises one or more of a nucleic acid molecule, a small molecule compound, a polypeptide or a protein.
45. The composition according to any one of embodiments 40 to 44, wherein the therapeutic agent or prophylactic agent is a vaccine or a compound capable of eliciting an immune response.
46. The composition according to any one of embodiments 40 to 43, wherein the therapeutic agent or prophylactic agent is a nucleic acid.
47. The composition according to any one of embodiments 40 to 43, wherein the therapeutic agent or prophylactic agent is ribonucleic acid (RNA).
48. The composition according to any one of embodiments 40 to 43, wherein the therapeutic or prophylactic agent is deoxyribonucleic acid (DNA).
49. The composition according to embodiment 47, wherein the RNA is selected from the group consisting of small interfering RNA (siRNA), asymmetric interfering RNA (aiRNA), micro RNA (miRNA), Dicer-substrate RNA (dsRNA), small hairpin RNA (shRNA), messenger RNA (mRNA) and a mixture thereof.
50. The composition according to embodiment 49, wherein the RNA is mRNA.
51. The composition according to any one of embodiments 19 to 50, wherein the composition further comprises one or more of pharmaceutically acceptable excipients or diluents.
52. Use of the compound of Formula (I) or the N-oxide, solvate, pharmaceutically acceptable salt or stereoisomer thereof according to any one of embodiments 1 to 18 or the composition according to any one of embodiments 19 to 51 in the manufacture of a nucleic acid drug, a gene vaccine, a small molecule drug, a polypeptide or a protein drug.
53. Use of the compound of Formula (I) or the N-oxide, solvate, pharmaceutically acceptable salt or stereoisomer thereof according to any one of embodiments 1 to 18 or the composition according to any one of embodiments 19 to 51 in the manufacture of a medicament for treating a disease or condition in a mammal in need thereof.
54. The use according to any one of embodiments 52 to 53, wherein the disease or condition is characterized by dysfunctional or abnormal protein or polypeptide activity.
55. The use according to embodiment 54, wherein the disease or condition is selected from the group consisting of infectious diseases, cancer and proliferative diseases, genetic diseases, autoimmune diseases, diabetes, neurodegenerative diseases, cardiovascular and renal vascular diseases, and metabolic diseases.
56. The use according to embodiment 55, wherein the infectious disease is selected from the group consisting of diseases caused by coronavirus, influenza virus, or HIV virus, pediatric pneumonia, Rift Valley fever, yellow fever, rabies, and various herpes.
57. The use according to any one of embodiments 53 to 56, wherein the mammal is a human.
58. The use according to any one of embodiments 52 to 57, wherein the composition is to be administered intravenously, intramuscularly, intradermally, subcutaneously, intranasally or by inhalation.
59. The use according to embodiment 58, wherein the composition is to be administered subcutaneously.
60. The use according to any one of embodiments 53 to 59, wherein the therapeutic or prophylactic agent is to be administered to a mammal at an amount of about 0.001 mg/kg to about 10 mg/kg.

### Example

The present disclosure is further described below in conjunction with examples. However, the present disclosure is not limited to the following examples. The implementation conditions used in the examples can be further adjusted according to the different requirements of specific use, and the implementation conditions not specified are conventional conditions in the industry. In the specific examples of the present disclosure, the raw materials used can all be obtained commercially. Unless otherwise specified, the percentages in the context are weight percentages, and all temperatures are given in degrees Celsius. The technical features involved in each embodiment of the present disclosure can be combined with each other as long as they do not conflict with each other

### Example 1: Synthesis of cationic lipid compound

### 1. Synthesis of YK-201

The synthetic route is as follows:

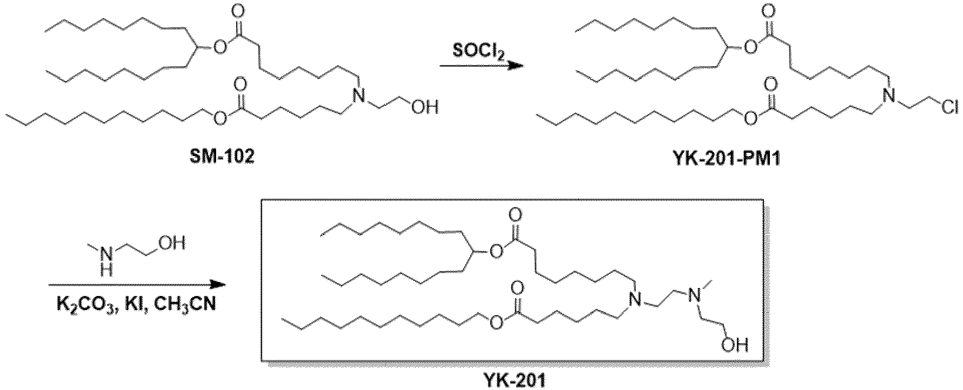

Step 1: Synthesis of heptadecan-9-yl-8-((2-chloroethyl)(6-oxo-6-((undecyloxy)hexyl)amino)octanoate (YK-201-PM1):
SM-102 (10.00 g, 0.014 mol) was added to a single-mouth bottle, and 100 mL of SOCl₂ was added. The mixture was stirred at room temperature for 6 hours. After the reaction was completed, the reaction solution was poured into ice water to quench the reaction, and extracted with ethyl acetate. The organic phase was dried under vacuum to obtain a colorless oily product (9.51 g, 0.013 mol, 93.1%), C₄₄H₈₆ClNO₄, MS (ES): m/z (M+H⁺) 728.5.

### Step 2: Synthesis of heptadecan-9-yl-8-(2-((2-hydroxyethyl)(methylamino))ethyl)(6-oxo-6-((undecanyloxy)hexyl) amino)octanoate (YK-201)

YK-201-PM1 (1.00 g, 1.37 mmol) and 2-methylamino-ethanol (0.31 g, 4.11 mmol) were dissolved in acetonitrile (20 mL). Potassium carbonate (0.61 g, 4.41 mmol) and potassium iodide (38 mg, 0.23 mmol) were added to the above system and heated to 70°C. The mixture was stirred for 7 hours. The reaction solution was cooled to room temperature and filtered. The filtrate was concentrated under vacuum to remove the solvent. The residue was purified by silica gel chromatography (ethyl acetate/n-hexane) to obtain YK-201 (559 mg, 0.73 mmol, 53.3%). C₄₇H₉₄N₂O₅, MS(ES): m/z(M+H⁺)767.6.

¹H NMR (400 MHz, Chloroform-*d*) δ 4.05 (t, J = 6.8 Hz, 2H), 3.57 (t, J = 5.1 Hz, 2H), 2.57 - 2.52 (m, 5H), 2.46 (dt, J = 9.8, 5.1 Hz, 4H), 2.33 - 2.25 (m, 7H), 1.68 - 1.56 (m, 6H), 1.47 (dd, J = 13.4, 6.0 Hz, 8H), 1.28 (d, J = 17.7 Hz, 50H), 0.88 (t, J = 6.8 Hz, 9H).

### 2. Synthesis of YK-202

The synthetic route is as follows:

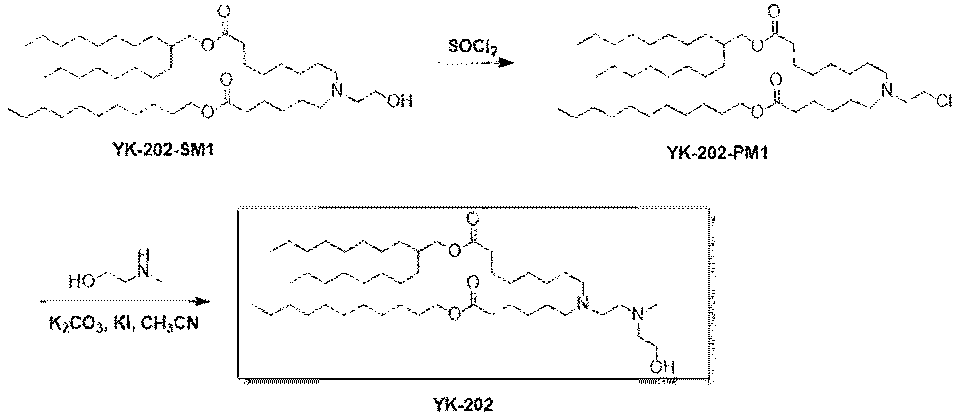

### Step 1: Synthesis of 2-octyldecyl-8-((2-chloroethyl)(6-oxo-6-((undecyloxy)hexyl)amino)octanoate (YK-202-PM1):

YK-202-SM1 (1.50 g, 2.07 mmol) was added to a single-mouth bottle, and 10 mL of SOCl₂ was added. The mixture was stirred at room temperature for 6 hours. After the reaction was completed, the reaction solution was poured into ice water to quench the reaction, and extracted with ethyl acetate. The organic phase was dried under vacuum to obtain a colorless oily product (1.63 g, 2.07 mmol, 100.0%), C₄₅H₈₈ClNO₄, MS(ES): m/z(M+H⁺)742.6.

### Synthesis of 2-octyldecyl-8-((2-(2-hydroxyethyl(methyl)amino)ethyl)(6-oxo-6-((undecanyloxy)hexyl)amin o)octanoate (YK-202)

The target compound (1.01 g, 1.29 mmol, 62.3%) was obtained by using the above-obtained YK-202-PM1 (1.60 g, 2.07 mmol) and 2-methylamino-ethanol (0.47 g, 6.21 mmol) as raw materials according to the method for synthesizing YK-201. C₄₈H₉₆N₂O₅, MS(ES):m/z(M+H⁺)781.3.

¹H NMR (400 MHz, Chloroform-*d*) δ 4.05 (t, J = 6.7 Hz, 2H), 3.96 (d, J = 5.7 Hz, 2H), 3.62 (t, J = 4.8 Hz, 2H), 2.72 (d, J = 5.4 Hz, 2H), 2.64 (dd, J = 14.4, 5.3 Hz, 8H), 2.36 (s, 3H), 2.30 (td, J = 7.4, 4.0 Hz, 4H), 1.60 (qd, J = 15.3, 7.7 Hz, 11H), 1.28 (d, J = 14.9 Hz, 52H), 0.88 (t, J = 6.6 Hz, 9H).

### 3. Synthesis of YK-203

The synthetic route is as follows:

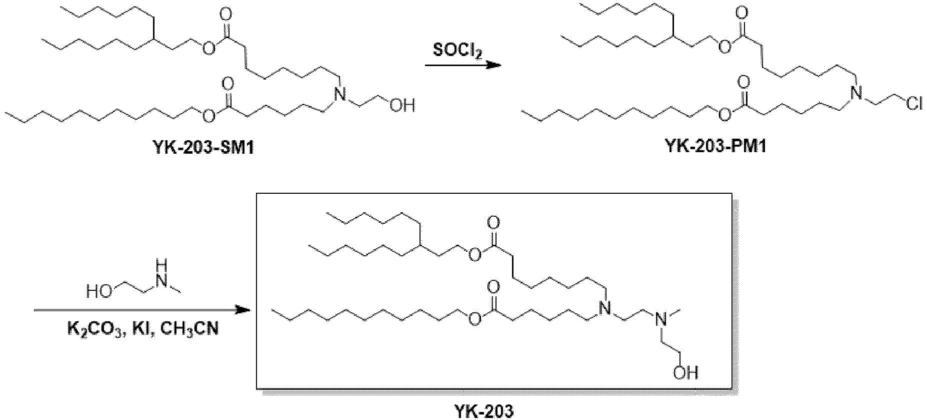

### Step 1: Synthesis of 3-hexylnonyl-8-((2-chloroethyl)(6-oxo-6-((undecanyloxy)hexyl)amino)octanoate (YK-203-PM1):

YK-203-PM1 (250 mg, 0.36 mmol, 87.0%) was obtained by using YK-203-SM1 (280 mg, 0.41 mmol) and SOCl₂ (2 mL) as raw materials according to the method for synthesizing YK-201-PM1. C₄₂H₈₂ClNO₄, MS(ES): m/z(M+H⁺)700.6.

### Step 2: Synthesis of 3-hexylnonyl-8-((2-(2-hydroxyethyl(methyl)amino)ethyl)(6-oxo-6-((undecanyloxy)hexyl)ami no)octanoate (YK-203)

YK-203 (120 mg, 0.16 mmol, 73.8%) was obtained by using YK-203-PM1 (150 mg, 0.22 mmol) and 2-methylamino-ethanol (75 mg, 0.22 mmol) as raw materials according to the method for synthesizing YK-201. C₄₅H₉₀N₂O₅, MS(ES): m/z(M+H⁺)739.7.

¹H NMR (400 MHz, Chloroform-d) δ 4.10 (dt, *J* = 9.2, 7.0 Hz, 4H), 3.73 - 3.63 (m, 2H), 3.51 (s, 3H), 3.11 (s, 4H), 2.96 (t, *J =* 6.1 Hz, 2H), 2.88 (q, *J =* 7.8 Hz, 4H), 2.79 (t, *J =* 6.1 Hz, 2H), 2.70 (t, *J =* 4.9 Hz, 2H), 2.42 (s, 2H), 2.33 (dt, *J =* 13.2, 7.4 Hz, 4H), 1.70 - 1.59 (m, 10H), 1.37 - 1.27 (m, 41H), 0.94 - 0.88 (m, 9H).

### 4. Synthesis of YK-204

The synthetic route is as follows:

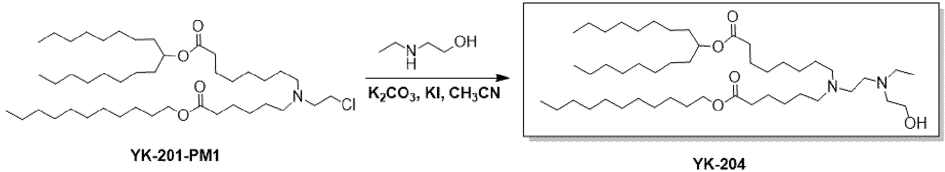

### Step 1: Synthesis of heptadecan-9-yl-8-(2-((2-hydroxyethyl)(ethylamino))ethyl)(6-oxo-6-((undecanyloxy)hexyl)a mino)octanoate (YK-204)

YK-204 (52 mg, 0.07 mmol, 55.5%) was obtained by using YK-201-PM1 (88 mg, 0.12 mmol) and 2-ethylamino-ethanol (36 mg, 0.41 mmol) as raw materials according to the method for synthesizing YK-201. C₄₈H₉₆N₂O₅, MS(ES): m/z(M+H⁺)781.7.

¹H NMR (400 MHz, Chloroform-d) δ 5.41 - 5.24 (m, 1H), 4.85 (t, *J* = 6.2 Hz, 1H), 4.07 (q, *J* = 7.7, 6.7 Hz, 3H), 3.91 - 3.48 (m, 2H), 3.36 (d, *J* = 25.5 Hz, 2H), 3.08 (s, 2H), 2.38 - 2.15 (m, 3H), 2.11 - 1.96 (m, 1H), 1.85 (s, 3H), 1.76 - 1.46 (m, 21H), 1.26 (s, 48H), 0.94 - 0.83 (m, 9H).

### 5. Synthesis of YK-205

The synthetic route is as follows:

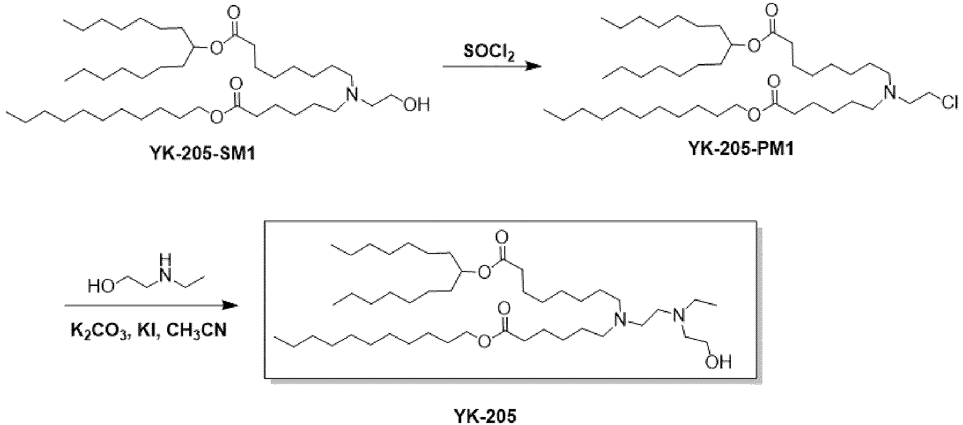

### Step 1: Synthesis of pentadecane-8-yl-8-((2-chloroethyl)(6-oxo-6-((undecyloxy)hexyl)amino)octanoate (YK-205-PM1):

YK-205-PM1 (400 mg, 0.57 mmol, 96.8%) was obtained by using YK-205-SM1 (400 mg, 0.59 mmol) and SOCl₂ (3 mL) as raw materials according to the method for synthesizing YK-201-PM1. C₄₂H₈₂ClNO₄, MS(ES): m/z(M+H⁺)700.5.

### Step 2: Synthesis of pentadecane-8-yl-8-((2-(2-hydroxyethyl(ethyl)amino)ethyl)(6-oxo-6-((undecanyloxy)hexyl)a mino)octanoate (YK-205)

The target compound (160 mg, 0.21 mmol, 73.2%) was obtained by using YK-205-PM1 (200 mg, 0.29 mmol) and 2-ethylamino-ethanol (127 mg, 1.43 mmol) as raw materials according to the method for synthesizing YK-201. C₄₆H₉₂N₂O₅, MS(ES):m/z(M+H⁺)753.7.

¹H NMR (400 MHz, Chloroform-d) δ 4.86 (p, *J* = 6.2 Hz, 1H), 4.05 (t, *J* = 6.8 Hz, 2H), 3.57 (t, *J* = 4.9 Hz, 2H), 2.69 - 2.58 (m, 7H), 2.54 (s, 4H), 2.29 (dt, *J* = 10.4, 7.5 Hz, 4H), 1.62 (dq, *J =* 14.0, 7.3 Hz, 6H), 1.49 (d, *J =* 6.3 Hz, 8H), 1.29 (d, *J =* 21.1 Hz, 46H), 1.05 (t, *J* = 7.1 Hz, 3H), 0.92 - 0.84 (m, 9H).

### 6. Synthesis of YK-206

The synthetic route is as follows:

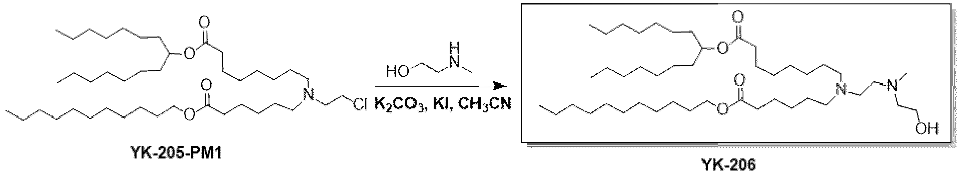

### Synthesis of pentadecane-8-yl 8-((2-(2-hydroxyethyl(methyl)amino)ethyl)(6-oxo-6-((undecanyloxy)hexyl)amino)octanoate (YK-206)

The target compound (127 mg, 0.17 mmol, 58.6%) was obtained by using YK-205-PM1 (200 mg, 0.29 mmol) and 2-methylamino-ethanol (107 mg, 1.43 mmol) as raw materials according to the method for synthesizing YK-201. C₄₅H₉₀N₂O₅ , MS(ES):m/z(M+H⁺)739.7.

¹H NMR (400 MHz, Chloroform-*d*) δ 4.86 (p, *J =* 6.2 Hz, 1H), 4.05 (t, *J =* 6.8 Hz, 2H), 3.56 (t, *J =* 5.1 Hz, 2H), 3.28 (s, 2H), 2.92 - 2.71 (m, 1H), 2.54 (d, *J =* 10.4 Hz, 6H), 2.44 (dt, *J* = 9.9, 4.8 Hz, 4H), 2.37 - 2.21 (m, 6H), 1.62 (dq, *J* = 14.5, 7.6 Hz, 6H), 1.46 (dt, *J* = 27.8, 9.8 Hz, 8H), 1.28 (d, *J =* 15.4 Hz, 43H), 0.88 (t, *J =* 6.7 Hz, 9H).

### 7. Synthesis of YK-207

The synthetic route is as follows:

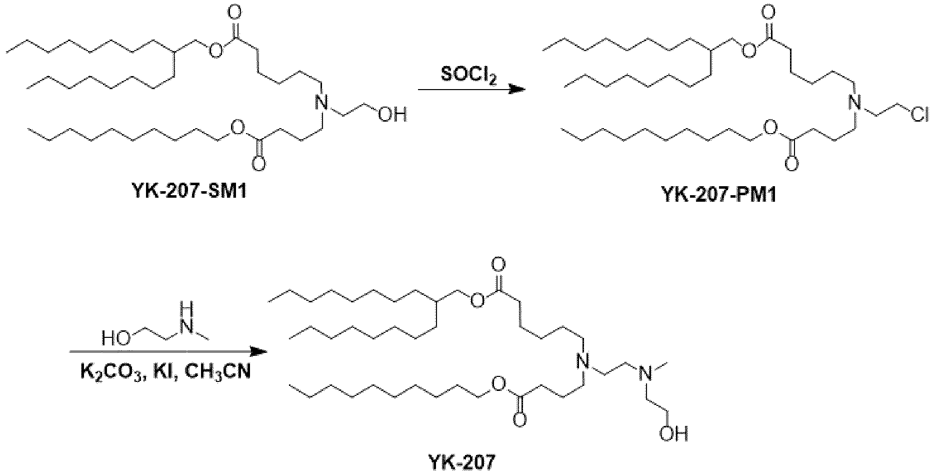

### Step 1: Synthesis of 2-octyldecyl 6-((2-chloroethyl)(4-oxo-4-((decyloxy)butyl)amino)hexanoate (YK-207-PM 1):

YK-207-PM1 (2.9 g, 4.31 mmol, 94.2%) was obtained by using YK-207-SM1 (3.0 g, 4.58 mmol) and SOCl₂ (5 mL) as raw materials according to the method for synthesizing YK-201-PM1. C₄₀H₇₈ClNO₄, MS(ES): m/z(M+H⁺)672.3.

### Step 2: Synthesis of 2-octyldecyl-6-(2-((2-(2-hydroxyethyl(methyl)amino)ethyl)(4-oxo-4-((decyloxy)butyl)amino) hexanoate (YK-207)

YK-207 (50 mg, 0.07 mmol, 23.4%) was obtained by using YK-207-PM1 (200 mg, 0.30 mmol) and 2-methylamino-ethanol (22 mg, 0.29 mmol) as raw materials according to the method for synthesizing YK-201. C₄₃H₈₆N₂O₅, MS(ES): m/z(M+H⁺)711.7.

¹H NMR (400 MHz, Chloroform-d) δ 5.43 - 5.15 (m, 1H), 4.07 (t, *J* = 6.8 Hz, 2H), 3.96 (d, *J =* 5.8 Hz, 2H), 3.87 (t, *J* = 4.6 Hz, 1H), 3.34 (d, *J* = 25.1 Hz, 3H), 3.05 (s, 3H), 2.70 (s, 2H), 2.47 (t, *J* = 6.5 Hz, 2H), 2.34 (t, *J* = 7.3 Hz, 2H), 2.01 (d, *J* = 6.9 Hz, 2H), 1.84 - 1.74 (m, 2H), 1.65 (dp, *J =* 21.2, 7.1 Hz, 5H), 1.46 - 1.37 (m, 2H), 1.37 - 1.17 (m, 44H), 0.94 - 0.85 (m, 9H).

### 8. Synthesis of YK-208

The synthetic route is as follows:

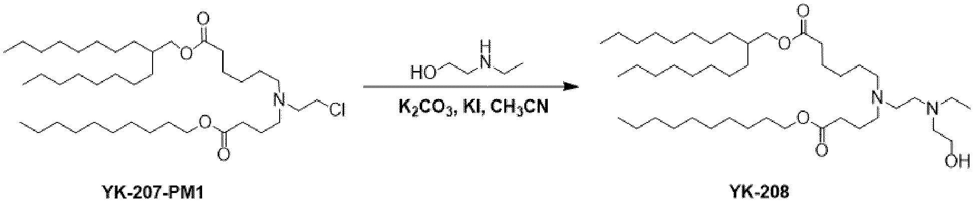

### Synthesis of 2-octyldecyl 6-(2-((2-(2-hydroxyethyl(ethyl)amino)ethyl)(4-oxo-4-((decyloxy)butyl)amino)hexanoate (YK-208)

YK-208 (140 mg, 0.19 mmol, 87.8%) was obtained by using YK-207-PM1 (150 mg, 0.22 mmol) and 2-ethylamino-ethanol (59 mg, 0.66 mmol) as raw materials according to the method for synthesizing YK-201. C₄₄H₈₈N₂O₅, MS(ES): m/z(M+H⁺)725.7.

¹H NMR (400 MHz, Chloroform-d) δ 5.33 (s, 1H), 4.60 (s, 2H), 4.09 (t, *J* = 6.8 Hz, 2H), 3.99 (d, *J* = 5.8 Hz, 2H), 3.68 (t, *J* = 4.9 Hz, 2H), 2.93 - 2.54 (m, 11H), 2.36 (dt, *J* = 17.0, 7.3 Hz, 4H), 1.93 - 1.78 (m, 2H), 1.76 - 1.48 (m, 7H), 1.45 - 1.21 (m, 43H), 1.15 (t, *J =* 7.1 Hz, 3H), 1.00 - 0.78 (m, 9H).

### 9. Synthesis of YK-209

The synthetic route is as follows:

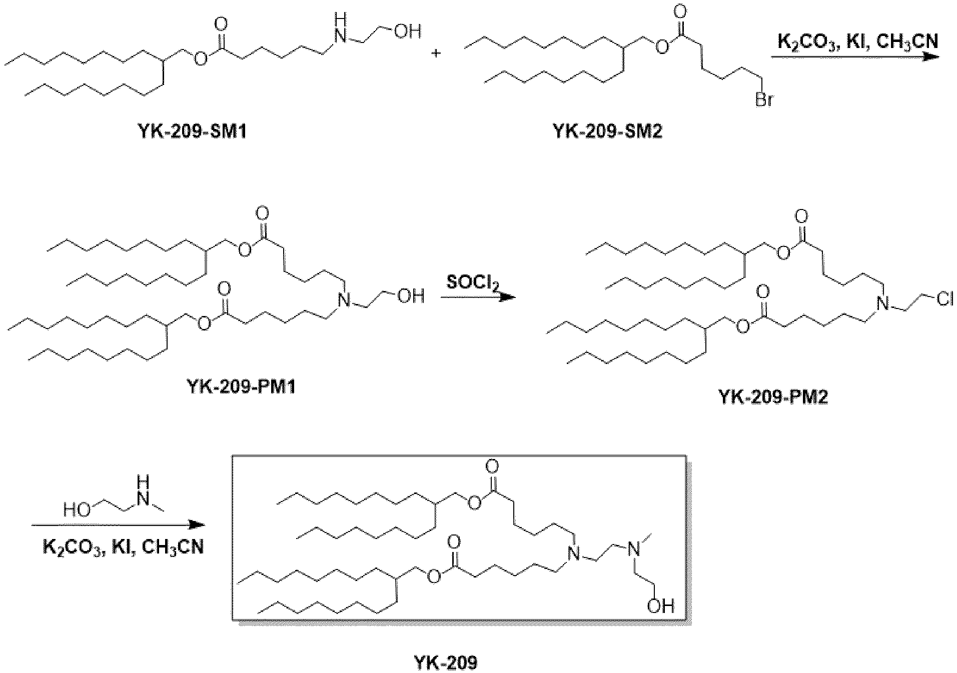

### Step 1: Synthesis of bis(2-octyldecyl)-6,6'-((2-hydroxyethyl)azadialkyl) dihexanoate (YK-209-PM1)

YK-209-SM1 (1.0 g, 2.34 mmol) and YK-209-SM2 (1.15 g, 2.58 mmol) were dissolved in acetonitrile (7 mL), potassium carbonate (0.97 g, 7.02 mmol) and potassium iodide (38 mg, 0.23 mmol) were added to the above system, and the mixture was heated to 70°C and stirred for 8 hours. After the reaction was completed, the reaction solution was cooled to room temperature and filtered, and the filtrate was concentrated under vacuum to remove the solvent. The residue was purified by silica gel chromatography (methanol/dichloromethane) to obtain YK-209-PM1 (780 mg, 0.98 mmol, 41.9%), C₅₀H₉₉NO₅, MS(ES): m/z(M+H⁺)794.8.

### Step 2: Synthesis of bis(2-octyldecyl)-6,6'-((2-chloroethyl)azadialkyl)dihexanoate (YK-209-PM2):

YK-209-PM2 (800 mg, 0.98 mmol, 102.5%) was obtained by using YK-209-PM1 (760 mg, 0.96 mmol) and SOCl₂ (5 mL) as raw materials according to the method for synthesizing YK-201-PM1. C₅₀H₉₈ClNO₄, MS(ES): m/z(M+H⁺)812.7.

### Step 3: Synthesis of bis(2-octyldecyl)-6,6'-((2-(2-hydroxyethyl(methyl)amino)ethyl)azadialkyl)dihexanoate (YK-209)

YK-209 (120 mg, 0.14 mmol, 78.3%) was obtained by using YK-209-PM2 (150 mg, 0.18 mmol) and 2-methoxy-ethanol (27 mg, 0.36 mmol) as raw materials according to the method for synthesizing YK-201. C₅₃H₁₀₆N₂O₅, MS(ES): m/z(M+H⁺) 851.4.

¹H NMR (400 MHz, Chloroform-*d*) δ 3.96 (d, J = 5.8 Hz, 4H), 3.69 - 3.65 (m, 2H), 2.86 (s, 2H), 2.76 (s, 4H), 2.72 - 2.65 (m, 2H), 2.41 (s, 3H), 2.32 (t, J = 7.4 Hz, 4H), 1.66 (dt, J = 15.0, 7.4 Hz, 10H), 1.26 (s, 62H), 0.88 (t, J = 6.8 Hz, 12H).

### 10. Synthesis of YK-210

The synthetic route is as follows:

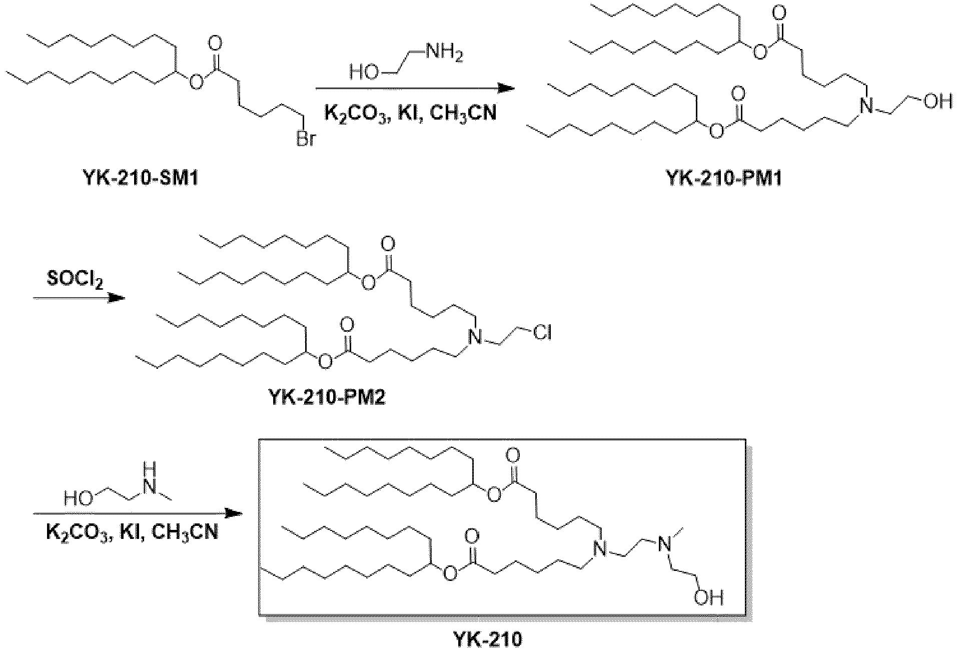

### Step 1: Synthesis of bis(heptadecan-9-yl)-6,6'-((2-hydroxyethyl)azadialkyl) dihexanoate (YK-210-PM1)

Heptadecan-9-yl-6-bromohexanoate (700 mg, 1.62 mmol) and ethanolamine (55 mg, 0.74 mmol) were dissolved in acetonitrile (5 mL), and potassium carbonate (307 mg, 2.22 mmol) and potassium iodide (12 mg, 0.07 mmol) were added to the above system. The mixture was heated to 70°C and stirred to react for 8 hours. After the reaction was completed, the reaction solution was cooled to room temperature and filtered, and the filtrate was concentrated under vacuum to remove the solvent. The residue was purified by silica gel chromatography (methanol/dichloromethane) to obtain YK-210-PM1 (450 mg, 0.66 mmol, 79.4%), C₄₈H₉₅NO₅, MS(ES): m/z(M+H⁺) 766.7.

### Step 2: Synthesis of bis(heptadecan-9-yl)-6,6'-((2-chloroethyl)azadialkyl)dihexanoate (YK-210-PM2):

YK-210-PM2 (460 mg, 0.59 mmol, 100.0%) was obtained by using YK-210-PM1 (450 mg, 0.59 mmol) and SOCl₂ (3 mL) as raw materials according to the method for synthesizing YK-201-PM1. C₄₈H₉₄ClNO₄, MS(ES): m/z(M+H⁺)784.6.

### Step 3: Synthesis of bis(heptadecan-9-yl)-6,6'-((2-(2-hydroxyethyl(methyl)amino)ethyl)azadialkyl)dihexanoate (YK-210)

YK-210 (136 mg, 0.17 mmol, 86.9%) was obtained by using YK-210-PM2 (150 mg, 0.19 mmol) and 2-methoxy-ethanol (17 mg, 0.23 mmol) as raw materials according to the method for synthesizing YK-201. C₅₁H₁₀₂N₂O₅, MS(ES): m/z(M+H⁺) 823.8.

¹H NMR (400 MHz, Chloroform-*d*) δ 5.30 (s, 1H), 4.85 (p, *J* = 6.2 Hz, 2H), 3.96 (s, 2H), 3.72 - 3.65 (m, 2H), 2.97 (s, 1H), 2.90 (s, 3H), 2.81 (s, 2H), 2.75 - 2.67 (m, 2H), 2.42 (s, 2H), 2.30 (t, *J* = 7.3 Hz, 4H), 1.72 - 1.62 (m, 7H), 1.50 (d, *J* = 5.6 Hz, 8H), 1.26 (s, 54H), 0.88 (t, *J =* 6.8 Hz, 12H).

### 11. Synthesis of YK-211

The synthetic route is as follows:

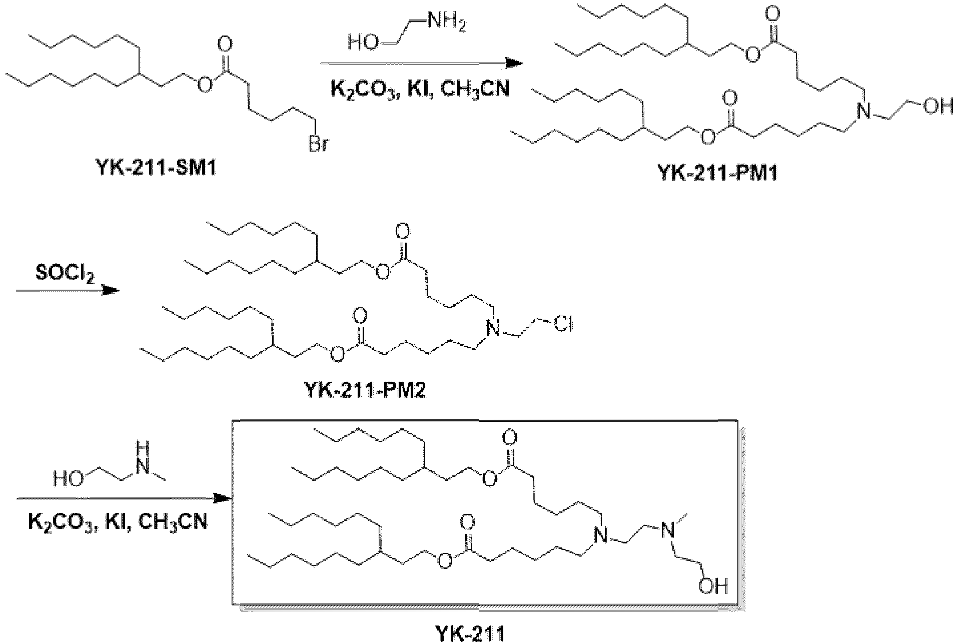

### Step 1: Synthesis of bis(3-hexylnonyl)-6,6'-((2-hydroxyethyl)azadialkyl) dihexanoate (YK-211-PM1)

YK-211-PM1 (470 mg, 0.66 mmol, 80.8%) was obtained by using 3-hexylnonyl 6-bromohexanoate (828 mg, 2.05 mmol) and ethanolamine (50 mg, 0.82 mmol) as raw materials according to the method for synthesizing YK-210-PM1. C₄₄H₈₇NO₅, MS(ES): m/z(M+H⁺)710.5.

### Step 2: Synthesis of bis(3-hexylnonyl)-6,6'-((2-chloroethyl)azadialkyl)dihexanoate (YK-211)-PM2:

YK-211-PM2 (420 mg, 0.58 mmol, 87.5%) was obtained by using YK-211-PM1 (470 mg, 0.66 mmol) and SOCl₂ (3 mL) as raw materials according to the method for synthesizing YK-201-PM1. C₄₄H₈₆ClNO₄, MS(ES): m/z(M+H⁺)728.6.

### Step 3: Synthesis of bis(3-hexylnonyl)-6,6'-((2-(2-hydroxyethyl(methyl)amino)ethyl)azadialkyl)dihexanoate (YK-211)

YK-211 (57 mg, 0.07 mmol, 53.1%) was obtained by using YK-211-PM2 (100 mg, 0.14 mmol) and 2-methylamino-ethanol (12 mg, 0.16 mmol) as raw materials according to the method for synthesizing YK-201. C₄₇H₉₄N₂O₅, MS(ES): m/z(M+H⁺)767.7.

¹H NMR (400 MHz, Chloroform-d) δ 4.08 (t, *J* = 7.2 Hz, 4H), 3.82 - 3.75 (m, 2H), 3.31 (d, *J = 5.6* Hz, 4H), 3.13 (dd, *J* = 18.9, 10.6 Hz, 5H), 2.87 (s, 2H), 2.56 (s, 2H), 2.33 (t, *J* = 7.2 Hz, 4H), 1.78 (p, *J* = 7.9 Hz, 3H), 1.73 - 1.62 (m, 4H), 1.57 (q, *J* = 7.0 Hz, 4H), 1.43 (dt, *J =* 14.4, 7.5 Hz, 6H), 1.25 (s, 42H), 0.88 (t, *J* = 6.7 Hz, 12H).

### 12. Synthesis of YK-212

The synthetic route is as follows:

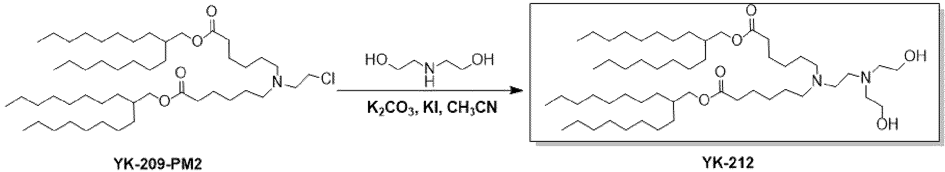

### Synthesis of bis(2-octyldecyl)-6,6'-((2-(bis(2-hydroxyethyl)amino)ethyl)azadialkyl)dihexanoate (YK-212)

YK-212 (80 mg, 0.09 mmol, 75.6%) was obtained by using YK-209-PM2 (100 mg, 0.12 mmol) and diethanolamine (26 mg, 0.24 mmol) as raw materials according to the method for synthesizing YK-201. C₅₄H₁₀₈N₂O₆, MS(ES): m/z(M+H⁺)881.8.

¹H NMR (400 MHz, Chloroform-d) δ 4.00 (d, *J =* 5.8 Hz, 4H), 3.69 (dd, *J =* 5.6, 4.0 Hz, 4H), 3.04 - 2.93 (m, 5H), 2.85 (t, *J =* 5.4 Hz, 2H), 2.75 (t, *J =* 4.8 Hz, 4H), 2.36 (t, *J =* 7.3 Hz, 4H), 1.70 (tq, *J* = 11.0, 6.7, 5.6 Hz, 10H), 1.30 (s, 63H), 0.95 - 0.89 (m, 12H).

### 13. Synthesis of YK-213

The synthetic route is as follows:

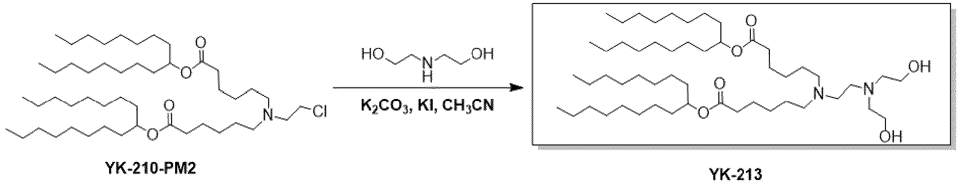

### Synthesis of bis(heptadecan-9-yl)-6,6'-((2-(bis(2-hydroxyethyl)amino)ethyl)azadialkyl)dihexanoate (YK-213)

YK-213 (50 mg, 0.06 mmol, 30.8%) was obtained by using YK-210-PM2 (150 mg, 0.19 mmol) and diethanolamine (21 mg, 0.20 mmol) as raw materials according to the method for synthesizing YK-201. C₅₂H₁₀₄N₂O₆, MS(ES): m/z(M+H⁺)853.8.

¹H NMR (400 MHz, Chloroform-d) δ 4.00 (d, *J =* 5.8 Hz, 4H), 3.69 (dd, *J =* 5.6, 4.0 Hz, 4H), 3.04 - 2.93 (m, 5H), 2.85 (t, *J =* 5.4 Hz, 2H), 2.75 (t, *J =* 4.8 Hz, 4H), 2.36 (t, *J =* 7.3 Hz, 4H), 1.70 (tq, *J* = 11.0, 6.7, 5.6 Hz, 10H), 1.30 (s, 63H), 0.95 - 0.89 (m, 12H).

### 14. Synthesis of YK-214

The synthetic route is as follows:

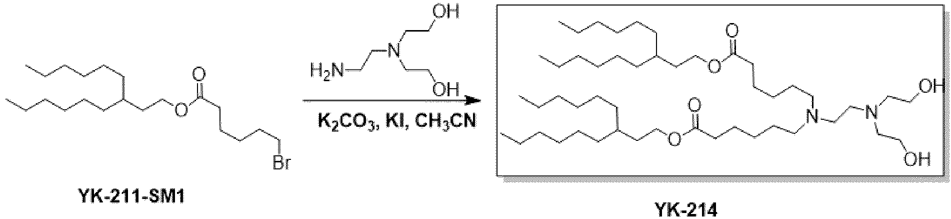

### Synthesis of bis(3-hexylnonyl)-6,6'-((2-(bis(2-hydroxyethyl)amino)ethyl)azadialkyl)dihexanoate (YK-214)

3-hexylnonyl 6-bromohexanoate (303 mg, 0.75 mmol) and N,N-bis(2-hydroxyethyl)ethylenediamine (50 mg, 0.34 mmol) were dissolved in acetonitrile (2 mL), potassium carbonate (140 mg, 1.02 mmol) and potassium iodide (5.6 mg, 0.034 mmol) were added to the above system, and the mixture was heated to 70°C and stirred for 24 hours. After the reaction was completed, the reaction solution was cooled to room temperature and filtered, and the filtrate was concentrated under vacuum to remove the solvent. The residue was purified by silica gel chromatography (methanol/dichloromethane) to obtain YK-214 (120 mg, 0.15 mmol, 44.3%), C₄₈H₉₆N₂O₆, MS(ES): m/z(M+H⁺)797.8.

¹H NMR (400 MHz, Chloroform-d) δ 4.11 (t, *J =* 7.2 Hz, 4H), 4.02 - 3.74 (m, 1H), 3.65 (t, *J* = 4.9 Hz, 4H), 2.74 (dd, *J* = 12.4, 7.4 Hz, 11H), 2.34 (t, *J* = 7.4 Hz, 4H), 1.64 (dq, *J* = 29.0, 7.4 Hz, 11H), 1.50 - 1.20 (m, 49H), 0.94 - 0.89 (m, 12H).

### 15. Synthesis of YK-215

The synthetic route is as follows:

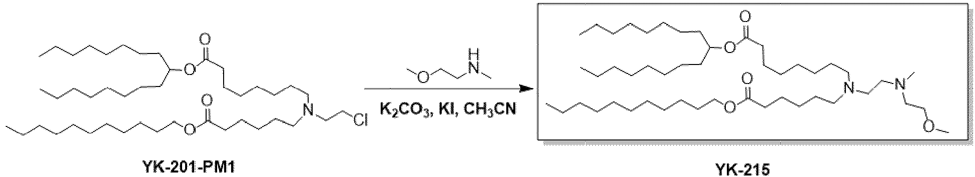

### Synthesis of heptadecan-9-yl-8-((2-(2-methoxyethyl(methyl)amino)ethyl)(6-oxo-6-((undecyloxy)hexyl)am ino)octanoate (YK-215)

The target compound (98 mg, 0.12 mmol, 96.5%) was obtained by using YK-201-PM1 (95 mg, 0.13 mmol) and 2-methoxy-N-methylethane-1-amine (14 mg, 0.16 mmol) as raw materials according to the method for synthesizing YK-201. C₄₈H₉₆N₂O₅, MS(ES):m/z(M+H⁺)781.2.

¹H NMR (400 MHz, Chloroform-*d*) δ 4.86 (t, *J =* 6.2 Hz, 1H), 4.05 (t, *J =* 6.8 Hz, 2H), 3.36 (s, 3H), 3.15 (t, *J* = 6.3 Hz, 3H), 3.01 (dq, *J* = 11.7, 7.2, 5.6 Hz, 6H), 2.77 (t, *J* = 5.0 Hz, 2H), 2.45 (s, 2H), 2.30 (dt, *J* = 17.9, 7.4 Hz, 4H), 1.85 - 1.57 (m, 10H), 1.50 (d, *J* = 6.3 Hz, 4H), 1.41 - 1.18 (m, 50H), 0.88 (t, *J* = 6.6 Hz, 9H).

### 16. Synthesis of YK-216

The synthetic route is as follows:

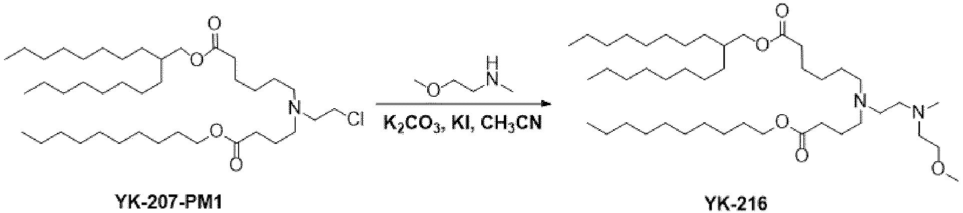

### Synthesis of 2-octyldecyl-6-(2-((2-(2-methoxyethyl(methyl)amino)ethyl)(4-oxo-4-((decyloxy)butyl)amino )hexanoate (YK-216)

YK-216 (140 mg, 0.19 mmol, 77.2%) was obtained by using YK-207-PM1 (170 mg, 0.25 mmol) and 2-methoxy-N-methylethane-1-amine (89 mg, 0.45 mmol) as raw materials according to the method for synthesizing YK-201. C₄₄H₈₈N₂O₅, MS(ES): m/z(M+H⁺)725.6.

¹H NMR (400 MHz, Chloroform-d) δ 4.09 (t, *J =* 6.7 Hz, 2H), 4.00 (d, *J =* 5.8 Hz, 2H), 3.58 (t, *J* = 5.3 Hz, 2H), 3.39 (s, 3H), 2.94 - 2.50 (m, 11H), 2.51 - 2.25 (m, 7H), 1.88 (d, *J =* 8.2 Hz, 2H), 1.75 - 1.48 (m, 7H), 1.50 - 1.20 (m, 43H), 0.98 - 0.83 (m, 9H).

### 17. Synthesis of YK-217

The synthetic route is as follows:

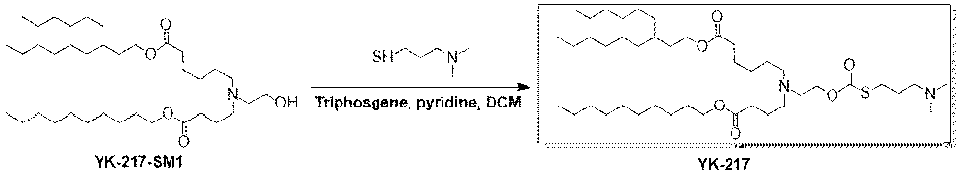

### Synthesis of 3-hexylnonyl-11-(4-(decyloxy)-4-oxobutyl)-2-methyl-7-oxo-8-oxo-6-thio-2,11-diazaheptane-17-carboxylate (YK-217)

YK-217-SM1 (100 mg, 0.16 mmol), dimethylaminopropanethiol (20 mg, 0.32 mmol) and pyridine (1.2 ml) were dissolved in dichloromethane (5 mL). Under nitrogen protection, triphosgene (77 mg, 0.26 mmol) was added to the above system in batches under ice bath. The ice bath was removed, the temperature was naturally raised to room temperature, and the reaction was stirred for 8 h. After the reaction was completed, saturated sodium bicarbonate aqueous solution (5 mL) was added to the reaction solution, 20 mL of dichloromethane was added, the liquid was separated, the organic phase was washed with saturated saline solution, and the organic phase was concentrated under vacuum to remove the solvent. The residue was purified by silica gel chromatography (methanol/dichloromethane) to obtain YK-217 (60 mg, 0.03 mmol, 16.5%), C₄₃H₈₄N₂O₆S, MS(ES): m/z(M+H⁺)757.2.

¹H NMR (400 MHz, Chloroform-d) δ 4.14 - 4.00 (m, 2H), 3.96 (t, *J =* 4.7 Hz, 2H), 3.34 (s, 4H), 2.91 (td, *J* = 7.2, 3.3 Hz, 2H), 2.47 (d, *J* = 7.9 Hz, 2H), 2.31 (t, *J* = 7.0 Hz, 8H), 1.95 - 1.78 (m, 4H), 1.62 (d, *J* = 11.7 Hz, 6H), 1.31 - 1.23 (m, 45H), 0.87 (dt, *J* = 7.0, 3.4 Hz, 9H).

### 18. Synthesis of YK-218

The synthetic route is as follows:

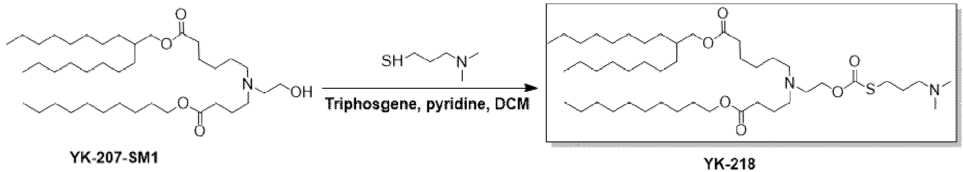

### Synthesis of 2-octyldecyl-11-(4-(decyloxy)-4-oxobutyl)-2-methyl-7-oxo-8-oxo-6-thio-2,11-diazaheptane-1 7-carboxylate (YK-218)

YK-218 (60 mg, 0.08 mmol, 50.0%) was obtained by using YK-207-SM1 (100 mg, 0.15 mmol) and dimethylaminopropylthiol (36.5 mg, 0.31 mmol) as raw materials according to the method for synthesizing YK-217. C₄₆H₉₀N₂O₆S, MS(ES): m/z(M+H⁺)799.7.

¹H NMR (400 MHz, Chloroform-*d*) δ 4.23 (t, *J* = 6.2 Hz, 2H), 4.05 (t, *J =* 6.8 Hz, 2H), 3.96 (d, *J* = 5.8 Hz, 2H), 2.89 (t, *J* = 7.2 Hz, 2H), 2.70 (t, *J* = 6.2 Hz, 2H), 2.46 (ddd, *J* = 12.4, 8.0, 6.1 Hz, 5H), 2.31 (d, *J* = 7.4 Hz, 9H), 1.91 - 1.83 (m, 2H), 1.77 - 1.68 (m, 2H), 1.62 (tt, *J =* 7.8, 4.5 Hz, 5H), 1.46 - 1.38 (m, 2H), 1.28 (d, *J =* 14.4 Hz, 46H), 0.88 (t, *J =* 6.7 Hz, 9H).

### 19. Synthesis of YK-219

The synthetic route is as follows:

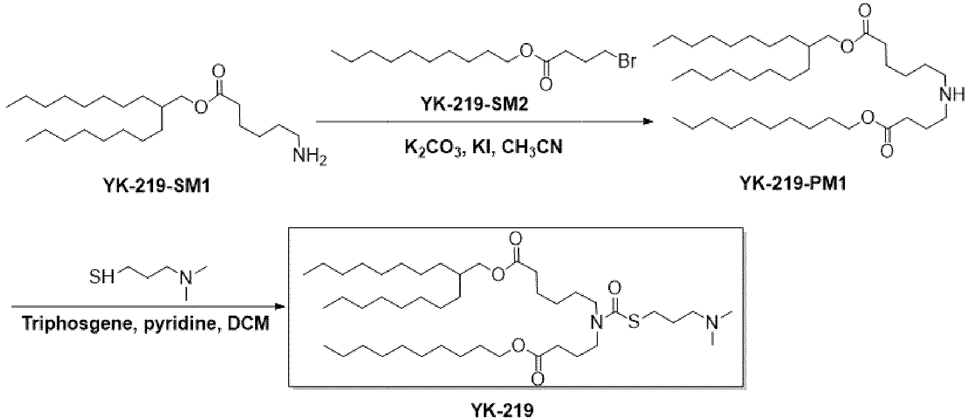

### Step 1: Synthesis of 2-octyldecyl 6-(4-(decyloxy)-4-oxobutyl)-aminohexanoate (YK-219-PM1)

YK-219-PM1 (310 mg, 0.51 mmol, 51.3%) was obtained by using YK-219-SM1 (459 mg, 1.19 mmol) and YK-219-SM2 (304 mg, 0.99 mmol) as raw materials according to the method for synthesizing YK-207-PM1. C₃₈H₇₅NO₄, MS(ES): m/z(M+H⁺) 610.5.

### Step 2: Synthesis of 2-octyldecyl-6-(4-(decyloxy)-4-oxobutyl)(((3-(dimethylamino)propyl)thio)carbonyl)amino)he xanoate (YK-219)

YK-219 (82mg, 0.11mmol, 21.3%) was obtained by using YK-219-PM1 (310mg, 0.51mmol) and dimethylaminopropylthiol (303mg, 2.54mmol) as raw materials according to the method for synthesizing YK-217. C₄₄H₈₆N₂O₅S, MS(ES): m/z(M+H⁺)755.5.

¹H NMR (400 MHz, Chloroform-*d*) δ 4.10 (q, *J =* 4.9, 3.5 Hz, 2H), 3.99 (t, *J =* 4.6 Hz, 2H), 3.37 (s, 3H), 2.95 (td, *J =* 7.2, 3.4 Hz, 2H), 2.50 (d, *J =* 7.8 Hz, 2H), 2.36 (d, *J =* 3.6 Hz, 9H), 2.07 - 1.84 (m, 4H), 1.70 - 1.59 (m, 6H), 1.43 - 1.26 (m, 47H), 0.91 (dt, *J =* 7.0, 3.4 Hz, 9H).

### 20. Synthesis of YK-220

The synthetic route is as follows:

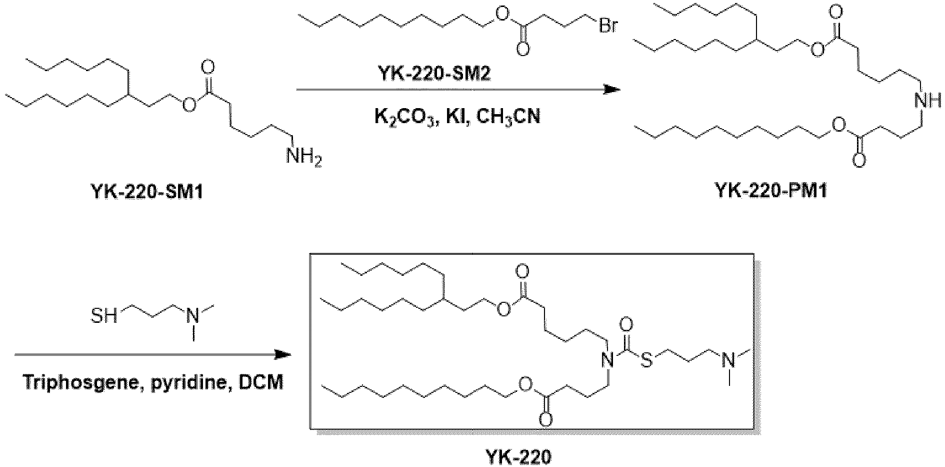

### Step 1: Synthesis of 3-hexylnonyl 6-(4-(decyloxy)-4-oxobutyl)-aminohexanoate (YK-220-PM1)

YK-220-PM1 (249 mg, 0.44 mmol, 46.2%) was obtained by using YK-220-SM1 (391 mg, 1.15 mmol) and YK-220-SM2 (292 mg, 0.95 mmol) as raw materials according to the method for synthesizing YK-210-PM1. C₃₅H₆₉NO₄, MS(ES): m/z(M+H⁺) 568.5.

### Step 2: Synthesis of 3-hexylnonyl 6-(4-(decyloxy)-4-oxobutyl)(((3-(dimethylamino)propyl)thio)carbonyl)amino)hexanoate (YK-220)

YK-220 (76 mg, 0.11 mmol, 30.4%) was obtained by using YK-220-PM1 (200 mg, 0.35 mmol) and dimethylaminopropyl mercaptan (209 mg, 1.75 mmol) as raw materials according to the method for synthesizing YK-217. C₄₁H₈₀N₂O₅S, MS(ES): m/z(M+H⁺)713.6.

¹H NMR (400 MHz, Chloroform-*d*) δ 4.12 - 4.02 (m, 4H), 3.35 (s, 4H), 2.92 (t, *J =* 7.2 Hz, 2H), 2.49 - 2.40 (m, 2H), 2.30 (s, 8H), 1.60 (dp, *J =* 20.8, 7.2 Hz, 8H), 1.44 - 1.18 (m, 40H), 0.88 (t, *J =* 6.8 Hz, 9H).

### 21. Synthesis of YK-221

The synthetic route is as follows:

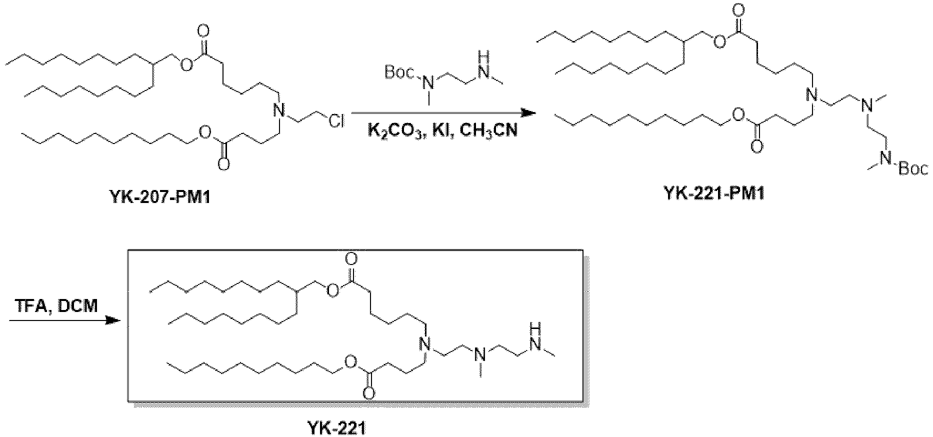

### Step 1: Synthesis of 2-octyldecyl-6-(2-((2-(N-tert-butyloxycarbonyl-N-methylamino) ethyl)(methylamino) ethyl)(4-oxo-4-((decyloxy) butyl) amino) hexanoate (YK-221-PM1)

YK-221-PM1 (120 mg, 0.15 mmol, 66.2%) was obtained by using YK-207-PM1 (150 mg, 0.22 mmol) and tert-butyl methyl (2-(methylamino)ethyl)carbamate(45 mg, 0.22 mmol) as raw materials according to the method for synthesizing YK-201. C₄₉H₉₇N₃O₆, MS(ES): m/z(M+H⁺)824.7.

### Step 2: Synthesis of 2-octyldecyl-6-(2-((2-(methylamino) ethyl)(methylamino)ethyl)(4-oxo-4-((decyloxy)butyl)amino)hexanoate (YK-221)

YK-221-PM1 (120 mg, 0.15 mmol) was dissolved in 3 mL of dichloromethane, 0.5 mL of trifluoroacetic acid was added dropwise, and the mixture was stirred at room temperature for 2 h. After the reaction was completed, the mixture was dried under vacuum, 10 mL of ethyl acetate was added to dissolve the mixture, and saturated sodium bicarbonate aqueous solution was added to extract the mixture. The organic phase was concentrated under vacuum to remove the solvent. The residue was purified by silica gel chromatography (ethyl acetate/n-hexane) to obtain the target compound (80 mg, 0.11 mmol, 73.6%). C₄₄H₈₉N₃O₄, MS(ES): m/z(M+H⁺)724.6.

¹H NMR (400 MHz, Chloroform-*d*) δ 4.06 (t, *J =* 6.8 Hz, 2H), 3.96 (d, *J =* 5.8 Hz, 1H), 3.21 (s, 3H), 3.05 (s, 2H), 2.91 (s, 1H), 2.73 (s, 2H), 2.54 (s, 2H), 2.49 - 2.17 (m, 11H), 2.03 (d, *J =* 11.0 Hz, 3H), 1.82 (s, 2H), 1.61 (d, *J =* 7.3 Hz, 4H), 1.53 - 1.40 (m, 2H), 1.26 (d, *J* = 3.9 Hz, 42H), 0.88 (t, *J =* 6.7 Hz, 9H).

### 2 2. Synthesis of YK-222

The synthetic route is as follows:

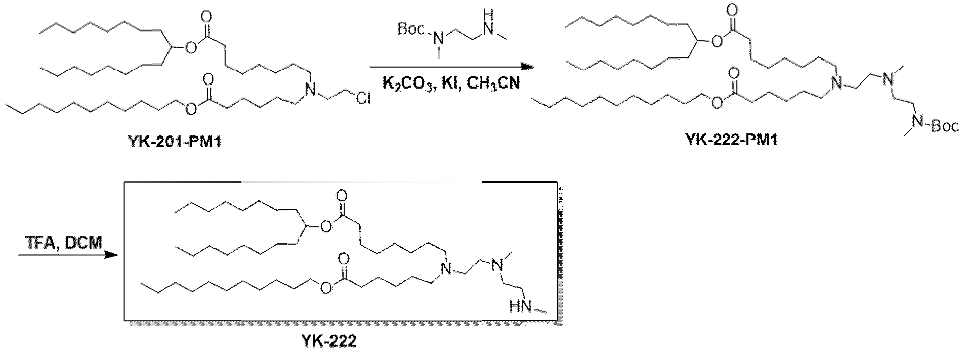

### Step 1: Synthesis of heptadecan-9-yl-8-(2-((2-(N-tert-butyloxycarbonyl-N-methylamino) ethyl)(methylamino)ethyl)(6-oxo-6-((undecanyloxy)hexyl)amino) octanoate (YK-222-PM1)

YK-222-PM1 (120 mg, 0.14 mmol, 97.4%) was obtained by using YK-201-PM1 (100 mg, 0.14 mmol) and tert-butyl methyl (2-(methylamino)ethyl) carbamate (28 mg, 0.15 mmol) as raw materials according to the method for synthesizing YK-201. C₄₉H₉₇N₃O₆, MS(ES): m/z(M+H⁺)880.3.

### Step 2: Synthesis of heptadecan-9-yl-8-(2-((2-(methylamino) ethyl)(methylamino)ethyl)(6-oxo-6-((undecanyloxy)hexyl)amino)octanoate (YK-222)

YK-222-PM1 (120 mg, 0.14 mmol) was dissolved in 1 mL of dichloromethane, 1 mL of trifluoroacetic acid was added dropwise, and the target compound (67 mg, 0.09 mmol, 61.3%) was obtained according to the method for synthesizing YK-221. C₄₈H₉₇N₃O₄, MS(ES): m/z(M+H⁺)780.4.

¹H NMR (400 MHz, Chloroform-d) δ 4.93 - 4.80 (m, 1H), 4.05 (t, *J =* 6.8 Hz, 2H), 3.15 (t, *J =* 5.4 Hz, 4H), 3.02 (s, 3H), 2.83 (s, 1H), 2.73 (s, 2H), 2.37 - 2.21 (m, 7H), 2.01 (d, *J =* 5.5 Hz, 6H), 1.63 (tt, *J =* 13.9, 7.4 Hz, 10H), 1.50 (d, *J =* 6.4 Hz, 4H), 1.46 - 1.15 (m, 49H), 0.93 - 0.85 (m, 9H).

### 23. Synthesis of YK-223

The synthetic route is as follows:

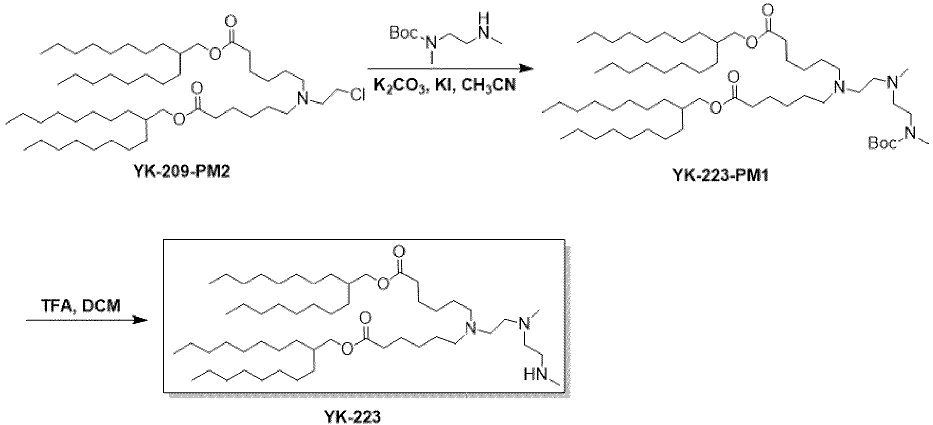

### Step 1: Synthesis of bis(2-octyldecyl)-6,6'-((2-((2-(N-Boc-N-methylamino)ethyl)(methylamino)ethyl)azadialkyl)di hexanoate (YK-223-PM1)

YK-223-PM1 (160 mg, 0.17 mmol, 92.2%) was obtained by using YK-209-PM2 (150 mg, 0.18 mmol) and tert-butyl methyl (2-(methylamino)ethyl)carbamate (45 mg, 0.24 mmol) as raw materials according to the method for synthesizing YK-201. C₅₉H₁₁₇N₃O₆, MS(ES): m/z(M+H⁺)964.9.

### Step 2: Synthesis of bis(2-octyldecyl)-6,6'-((2-((2-(methylamino) ethyl)(methylamino)ethyl)azadialkyl)dihexanoate (YK-223)

YK-223-PM1 (160 mg, 0.17 mmol) was dissolved in 1 mL of dichloromethane, 0.5 mL of trifluoroacetic acid was added dropwise, and the target compound (120 mg, 0.14 mmol, 81.7%) was obtained according to the method for synthesizing YK-221. C₅₄H₁₀₉N₃O₄, MS(ES): m/z(M+H⁺)864.9.

¹H NMR (400 MHz, Chloroform-*d*) δ 4.12 (q, *J =* 7.1 Hz, 1H), 3.97 (d, *J =* 5.8 Hz, 3H), 2.95 (d, *J =* 4.8 Hz, 1H), 2.88 - 2.40 (m, 10H), 2.38 - 2.25 (m, 6H), 2.05 (s, 2H), 1.74 - 1.12 (m, 74H), 0.88 (d, *J =* 6.9 Hz, 12H).

### 24. Synthesis of YK-224

The synthetic route is as follows:

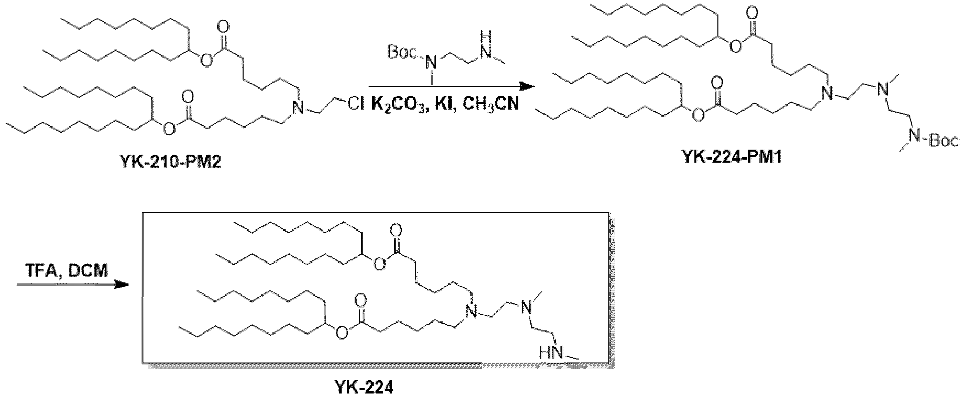

### Step 1: Synthesis of bis(heptadecan-9-yl)-6,6'-((2-((2-(N-Boc-N-methylamino)ethyl)(methylamino)ethyl)azadialk yl)dihexanoate (YK-224-PM1)

YK-224-PM1 (120 mg, 0.13 mmol, 55.7%) was obtained by using YK-210-PM2 (150 mg, 0.19 mmol) and tert-butyl methyl (2-(methylamino)ethyl)carbamate (43 mg, 0.23 mmol) as raw materials according to the method for synthesizing YK-201. C₅₇H₁₁₃N₃O₆, MS(ES): m/z(M+H⁺)936.8.

### Step 2: Synthesis of bis(heptadecan-9-yl)-6,6'-((2-((2-(methylamino) ethyl)(methylamino)ethyl)azadialkyl)dihexanoate (YK-224)

YK-224-PM1 (100 mg, 0.11 mmol) was dissolved in 2 mL of dichloromethane, 1 mL of trifluoroacetic acid was added dropwise, and the target compound (70 mg, 0.08 mmol, 76.1%) was obtained according to the method for synthesizing YK-221. C₅₂H₁₀₅N₃O₄, MS(ES): m/z(M+H⁺)836.7.

¹H NMR (400 MHz, Chloroform-*d*) δ 4.86 (q, *J =* 6.1 Hz, 2H), 4.12 (q, *J =* 7.1 Hz, 1H), 2.90 - 2.82 (m, 2H), 2.76 - 2.41 (m, 9H), 2.30 (q, *J =* 8.3, 7.4 Hz, 6H), 2.04 (s, 1H), 1.63 (q, *J* = 7.4 Hz, 4H), 1.57 - 1.39 (m, 12H), 1.28 (d, *J* = 15.2 Hz, 56H), 0.88 (t, *J* = 6.8 Hz, 12H).

### 25. Synthesis of YK-225

The synthetic route is as follows:

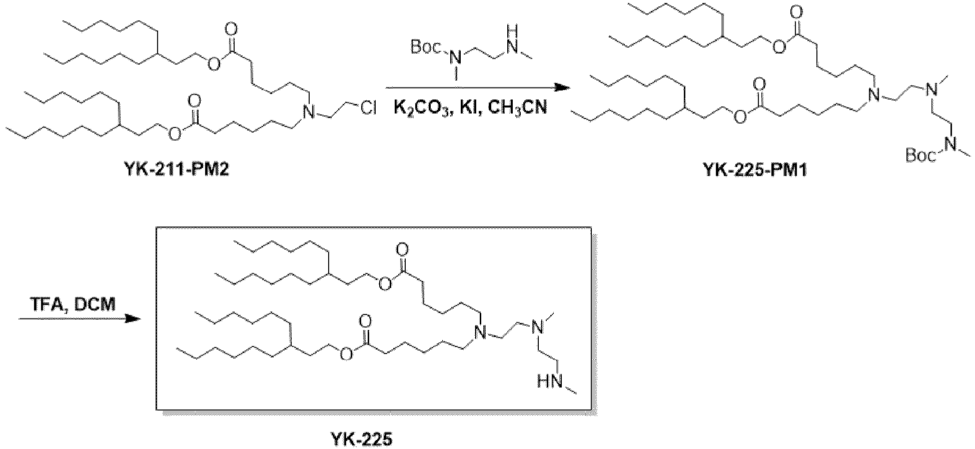

### Step 1: Synthesis of bis(3-hexylnonyl)-6,6'-((2-((2-(N-Boc-N-methylamino) ethyl)(methylamino)ethyl)azadialkyl)dihexanoate (YK-225-PM1)

YK-225-PM1 (120mg, 0.14mmol, 64.9%) was obtained by using YK-211-PM2 (170 mg, 0.21 mmol) and tert-butyl methyl (2-(methylamino)ethyl)carbamate (43 mg, 0.23 mmol) as raw materials according to the method for synthesizing YK-201. C₅₃H₁₀₅N₃O₆, MS(ES): m/z(M+H⁺)880.8.

### Step 2: Synthesis of bis(3-hexylnonyl)-6,6'-((2-((2-(methylamino) ethyl)(methylamino)ethyl)azadialkyl)dihexanoate (YK-225)

YK-225-PM1 (120 mg, 0.14 mmol) was dissolved in 2 mL of dichloromethane, 0.5 mL of trifluoroacetic acid was added dropwise, and the target compound (80 mg, 0.10 mmol, 73.2%) was obtained according to the method for synthesizing YK-221. C₄₈H₉₇N₃O₄, MS(ES): m/z(M+H⁺)780.7.

¹H NMR (400 MHz, Chloroform-*d*) δ 4.08 (t, *J =* 7.1 Hz, 4H), 3.48 (s, 3H), 3.04 - 2.86 (m, 5H), 2.29 (dd, *J =* 17.2, 9.6 Hz, 5H), 1.61 (ddd, *J =* 26.8, 14.3, 7.3 Hz, 10H), 1.47 - 1.35 (m, 6H), 1.25 (s, 45H), 0.88 (t, *J* = 6.1 Hz, 12H).

### 26. Synthesis of YK-226

The synthetic route is as follows:

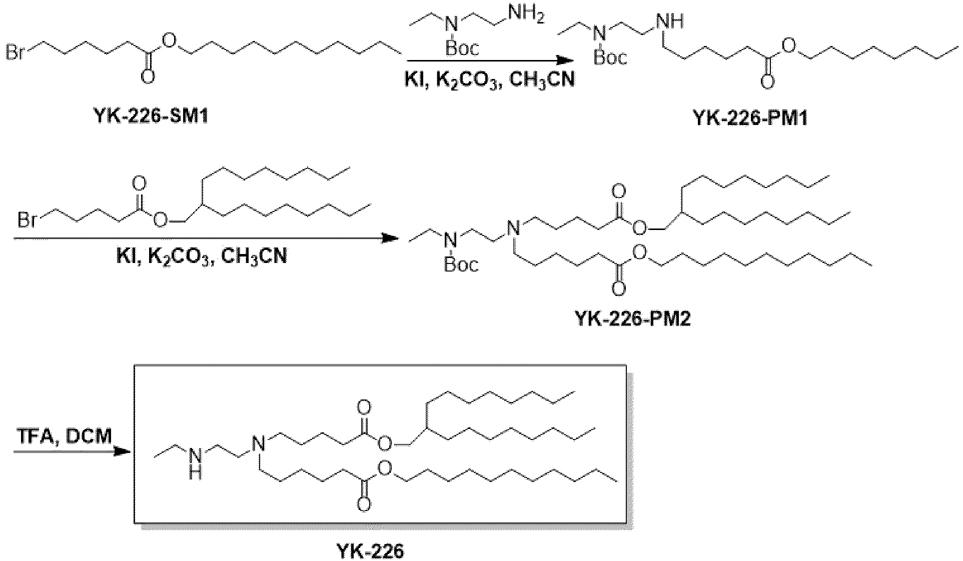

### Step 1: Synthesis of undecyl 6-((2-(N-Boc-N-ethylamino) ethyl) amino)hexanoate (YK-226-PM1):

YK-226-SM1 (278mg, 0.80mmol) and tert-butyl (2-aminoethyl)(ethyl)carbamate (150mg, 0.80mmol) were dissolved in acetonitrile (3mL), potassium carbonate (330mg, 2.38mmol) and potassium iodide (13mg, 0.08mmol) were added to the above system, and the mixture was heated to 70°C and stirred for 4 hours. After the reaction was completed, the reaction solution was cooled to room temperature and filtered, and the filtrate was concentrated under vacuum to remove the solvent. The residue was purified by silica gel chromatography (methanol/dichloromethane) to obtain decyl 4-((2-hydroxybutyl)amino)butyrate (190mg, 0.46mmol, 57.3%). C₂₃H₄₆N₂O₄, MS(ES): m/z(M+H⁺)415.4.

### Step 2: Synthesis of 2-octyldecyl-6-(6-(undecyloxy)-6-oxohexyl)((2-(N-Boc-N-ethylamino)ethyl)amino)hexanoat e (YK-226-PM2)

The purified YK-226-PM1 (190 mg, 0.42 mmol) and 2-octyldecyl 6-bromohexanoate (180 mg, 0.46 mmol) were dissolved in acetonitrile (3 mL), potassium carbonate (173 mg, 1.26 mmol) and potassium iodide (7 mg, 0.04 mmol) were added to the above system, and the mixture was heated to 70°C and stirred for 24 hours. The reaction solution was cooled to room temperature and filtered, and the filtrate was concentrated under vacuum to remove the solvent. The residue was purified by silica gel chromatography (ethyl acetate/n-hexane) to obtain the target compound (281 mg, 0.35 mmol, 82.7%). C₄₉H₉₆N₂O₆, MS(ES):m/z(M+H⁺)809.7.

### Step 3: Synthesis of 2-octyldecyl-6-(6-(undecyloxy)-6-oxohexyl)((2-(ethylamino) ethyl)amino)hexanoate (YK-226)

YK-226-PM2 (281 mg, 0.35 mmol) was dissolved in 2 mL of dichloromethane, 1 mL of trifluoroacetic acid was added dropwise, and the target compound (210 mg, 0.30 mmol, 84.6%) was obtained according to the method for synthesizing YK-221. C₄₄H₈₈N₂O₄, MS(ES): m/z(M+H⁺)709.6.

¹H NMR (400 MHz, Chloroform-*d*) δ 4.09 (t, *J =* 6.8 Hz, 2H), 4.00 (d, *J =* 5.8 Hz, 2H), 3.40 - 3.18 (m, 1H), 2.86 - 2.73 (m, 4H), 2.64 (t, *J =* 6.0 Hz, 2H), 2.46 (q, *J =* 7.1 Hz, 4H), 2.34 (q, *J =* 7.7 Hz, 4H), 1.73 - 1.58 (m, 7H), 1.54 - 1.42 (m, 4H), 1.37 - 1.20 (m, 49H), 0.97 - 0.89 (m, 9H).

### 27. Synthesis of YK-009

According to the method in CN114044741B, 159 mg of YK-009 was obtained.

### 28. Synthesis of 9-heptadecyl-8-(8-((3-hexylnonyl)oxy)-8-oxooctyl)-((2-hydroxyethyl)amino)octanoate (Compound 21)

The synthetic route is as follows:

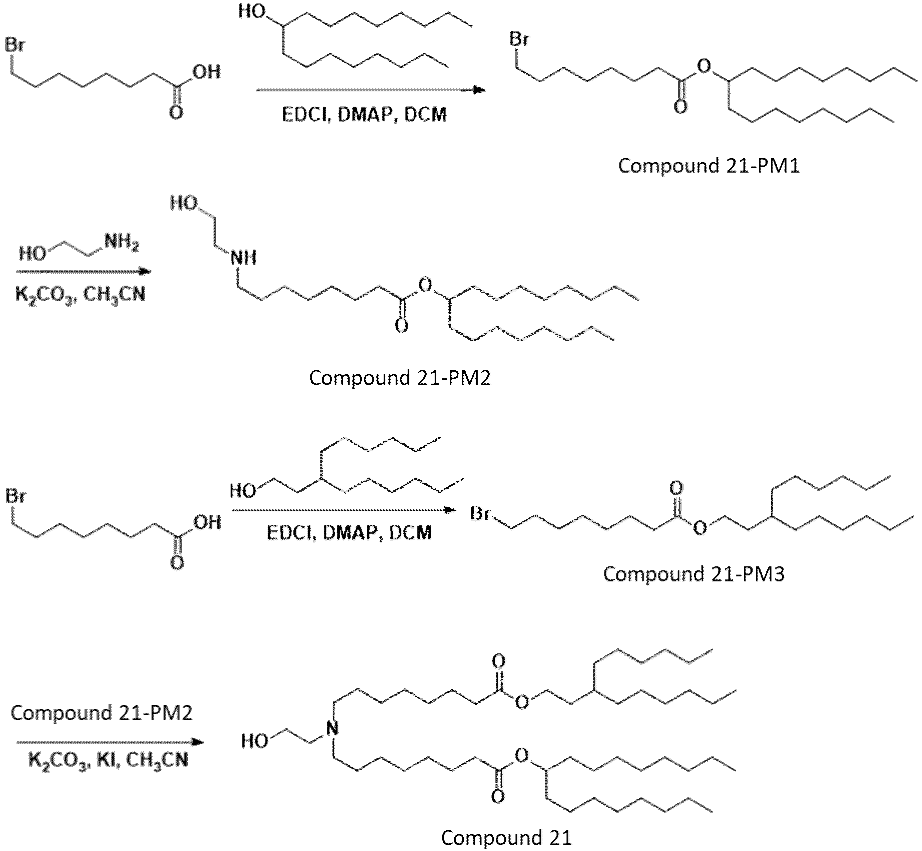

### Step 1: Synthesis of 9-heptadecanyl 8-bromooctanoate (Compound 21-PM1)

9-Heptadecanol (1.00 g, 3.90 mmol) and 8-bromooctanoic acid (1.04 g, 4.66 mmol) were dissolved in dichloromethane (10 mL), 1-(3-dimethylaminopropyl)-3-ethylcarbodiimide hydrochloride (0.90 g, 4.68 mmol) and 4-dimethylaminopyridine (24 mg, 0.20 mmol) were added, and the mixture was stirred at 30-35°C for 8 hours. After the reaction was completed, the reaction solution was washed with saturated sodium carbonate and saturated brine, and dried over Na₂SO₄. The mixture was filtered, and the filtrate was concentrated under vacuum and purified by silica gel chromatography (ethyl acetate/n-hexane) to obtain 9-heptadecanyl 8-bromooctanoate (1.20 g, 2.60 mmol, 66.7%).

### Step 2: Synthesis of 9-heptadecanyl 8-((2-hydroxyethyl)amino)octanoate (Compound 21-PM2)

9-heptadecanyl 8-bromooctanoate (500 mg, 1.08 mmol) and ethanolamine (119 mg, 3.25 mmol) were dissolved in acetonitrile (5 mL), potassium carbonate (149 mg, 1.08 mmol) was added, and the mixture was heated to 70°C and stirred for 2 hours. After the reaction was completed, the reaction solution was cooled to room temperature and filtered, and the filtrate was concentrated under vacuum to remove the solvent. The residue was purified by silica gel chromatography (methanol/dichloromethane) to obtain 9-heptadecanyl-8-((2-hydroxyethyl)amino)octanoate (372 mg, 0.84 mmol, 78.0%), C₂₇H₅₅NO₃, MS(ES): m/z(M+H⁺)442.3.

### Step 3: Synthesis of 8-bromooctanoic acid-3-hexylnonyl ester (Compound 21-PM3)

By using 3-hexylnonanol (1.00 g, 4.38 mmol) and 8-bromooctanoic acid (1.17 g, 5.25 mmol) as raw materials according to the method for preparing compound 21-PM1, a product was prepared and purified by silica gel chromatography (ethyl acetate/n-hexane) to obtain 3-hexylnonyl 8-bromooctanoate (1.62 g, 3.74 mmol, 85.3%).

### Step 4: Synthesis of 9-heptadecyl 8-(8-((3-hexylnonyl)oxy)-8-oxooctyl)-((2-hydroxyethyl)amino)octanoate (Compound 21)

9-Heptadecanyl 8-((2-hydroxyethyl)amino)octanoate (200 mg, 0.46 mmol) and 3-hexylnonyl 8-bromooctanoate (336 mg, 0.82 mmol) were dissolved in acetonitrile (6 mL), potassium carbonate (254 mg, 1.84 mmol) and potassium iodide (8.3 mg, 0.05 mmol) were added, and the mixture was heated to 70°C and stirred for 20 hours. The reaction solution was cooled to room temperature and filtered, and the filtrate was concentrated under vacuum to remove the solvent. The residue was purified by silica gel chromatography (ethyl acetate/n-hexane) to obtain the target compound (213 mg, 0.27 mmol, 58.4%). C₅₀H₉₉NO₅, MS(ES): m/z(M+H⁺)794.8.

¹H NMR (400 MHz, CDCl₃) δ 4.90 (p, *J =* 6.3 Hz, 1H), 4.21 - 4.02 (m, 2H), 3.66 (s, 2H), 2.73 (s, 2H), 2.60 (s, 4H), 2.43 - 2.20 (m, 4H), 2.12 - 1.99 (m, 1H), 1.75 - 1.49 (m, 13H), 1.48 - 1.39 (m, 2H), 1.42 - 1.15 (m, 56H), 0.92 (td, *J =* 6.8, 2.2 Hz, 12H).

### 29. Synthesis of bis(3-hexylnonyl)8,8'-((2-hydroxyethyl)azadialkyl)dioctanoate (Compound 23)

The synthetic route is as follows:

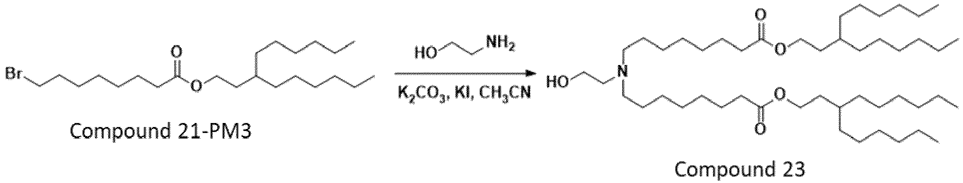

3-Hexylnonyl 8-bromooctanoate (710 mg, 1.64 mmol) and ethanolamine (40 mg, 0.66 mmol) were dissolved in acetonitrile (10 mL), potassium carbonate (1.09 g, 7.92 mmol) and potassium iodide (66 mg, 0.39 mmol) were added to the above system, and the mixture was heated to 70°C and stirred for 20 hours. After the reaction was completed, the reaction solution was cooled to room temperature and filtered, and the filtrate was concentrated under vacuum to remove the solvent. The residue was purified by silica gel chromatography (methanol/ dichloromethane) to obtain bis(3-hexylnonyl)-8,8'-((2-hydroxyethyl)azadialkyl) dioctanoate (150 mg, 0.20 mmol, 29.7%), C₄₈H₉₅NO₅, MS(ES): m/z(M+H⁺)766.5.

¹H NMR (400 MHz, CDCl₃) δ 4.12 (t, *J =* 7.1 Hz, 4H), 3.62 (s, 2H), 2.68 (s, 2H), 2.51 (d, *J =* 25.8 Hz, 4H), 2.32 (t, *J =* 7.5 Hz, 4H), 1.72 - 1.57 (m, 8H), 1.55 - 1.40 (m, 6H), 1.40 - 1.17 (m, 55H), 0.92 (t, *J =* 6.8 Hz, 12H).

### Example 2: Optimization of preparation conditions of nanolipid particles (LNP formulation)

### 1. Optimization of ratio of carrier (liposome) to mRNA

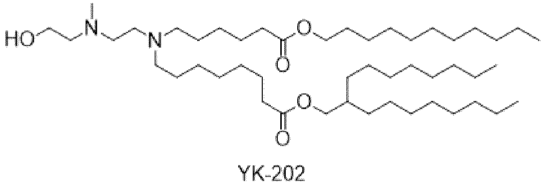

The cationic lipid compound YK-202 synthesized in Example 1, DSPC (AVT (Shanghai) Pharmaceutical Technology Co., Ltd.), cholesterol (AVT (Shanghai) Pharmaceutical Technology Co., Ltd.) and DMG-PEG2000 were dissolved in ethanol at a molar ratio of 49:10:39.5:1.5 to prepare an ethanol lipid solution. The ethanol lipid solution was quickly added to a citrate buffer (pH = 4 to 5) by ethanol injection method and vortexed for 30 seconds for use. eGFP-mRNA was diluted in a citrate buffer (pH = 4 to 5) to obtain an mRNA aqueous solution. Liposomes were prepared using a certain volume of liposome solution and an mRNA aqueous solution at a weight ratio of total lipid to mRNA of 5:1, 10:1, 15:1, 20:1, 30:1 and 35:1 at 25 °C and under ultrasound for 15 min (ultrasonic frequency 40 kHz, ultrasonic power 800W). After the obtained liposomes were diluted with PBS to 10 times the volume, 300KDa ultrafiltration tubes were used for ultrafiltration to remove ethanol. The mixture was then diluted to a certain volume with PBS to obtain an LNP formulation encapsulating eGFP-mRNA using cationic lipid YK-202/DSPC/cholesterol/DMG-PEG2000 (molar percentage of 49:10:39.5:1.5).

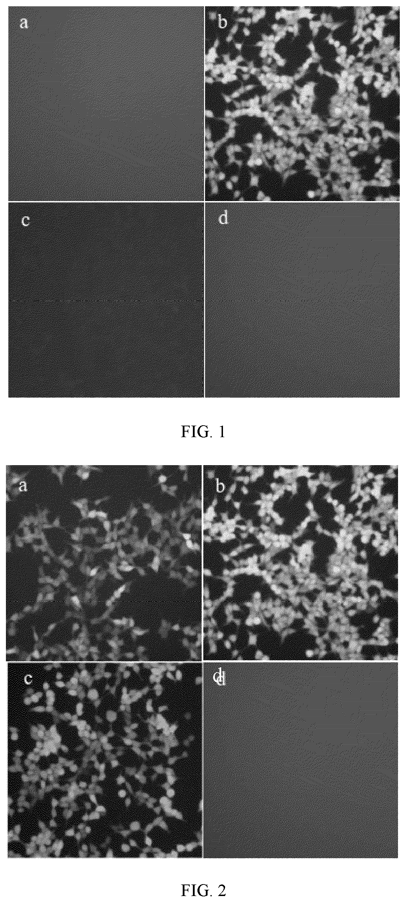

The results of the cell transfection experiment showed that the weight ratio of the carrier to mRNA in the range of 10:1 to 30:1 all had a good transfection effect, among which the best transfection effect was 15:1. The ratios of 5:1 and 35:1 had poor transfection effects and should not be used to deliver mRNA. (FIG. 1)

The same results were obtained with LNP formulation prepared with YK-201 and YK-209, which are not shown in the figure.

### 2. Optimization of ratio of cationic lipids to neutral lipids

In the LNP formulations encapsulating eGFP-mRNA prepared according to the method in 1, the molar ratios of cationic lipid YK-202 to neutral lipid DSPC were 1:1, 3:1, 3.5:1, 4:1, 4.5:1, 4.9: 1, 10:1, 15:1 and 20:1, respectively.

Through the cell transfection experiment, it can be seen that the molar ratio of cationic lipid to neutral lipid of 1:1 to 15:1 all had good transfection effect. Among them, the highest transfection efficiency was achieved at 4.5:1, and the ratios of 3.5:1 and 4.9:1 also had good transfection effects. (FIG. 2)

The same results were obtained with LNP formulations prepared with YK-201 and YK-209, which are not shown in the figure.

### 3. Optimization of ratio of polymer-conjugated lipid to carrier (liposome)

In the LNP formulations encapsulating eGFP-mRNA prepared according to the method in 1, the cationic lipid in the carrier was YK-202, and the molar ratios of the polymer-conjugated lipid DMG-PEG2000 in the carrier were 0.5%, 1.5%, 3.5%, 5%, 10% and 15%, respectively.

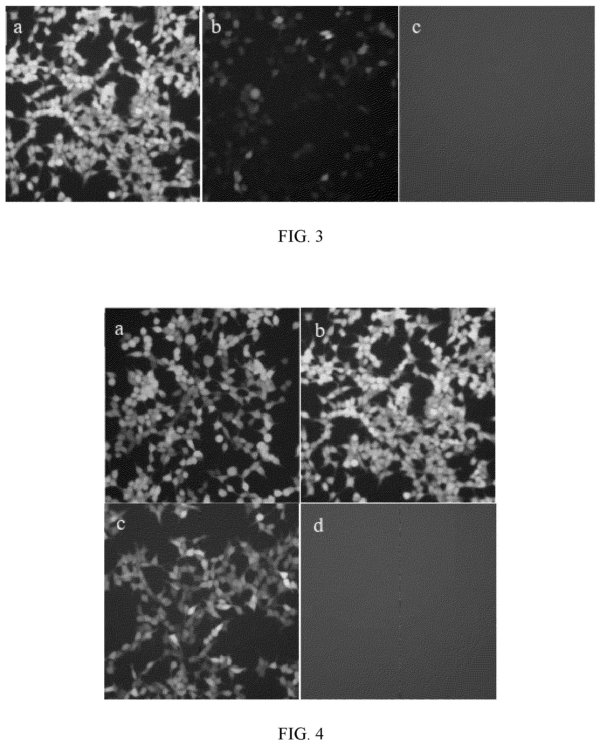

The results of the cell transfection experiment showed that the molar ratios of the polymer-conjugated lipid to the carrier in the range of 0.5% to 10% all had a transfection effect. The transfection efficiency was highest at 1.5% and lowest at 10%. (FIG. 3)

The same results were obtained with LNP formulations prepared with YK-201 and YK-209, which are not shown in the figure.

### 4. Optimization of ratio of each component in the carrier (liposome)

In the LNP formulations encapsulating eGFP-mRNA prepared according to the method in 1, the molar ratios of cationic lipid YK-202, neutral lipid DSPC, structured lipid cholesterol and polymer-conjugated lipid DMG-PEG2000 were 75:5:15:5, 49:10:39.5:1.5, 45:10: 43.5: 1.5, 45: 25: 20: 10, 40:10:48.5:1.5, 35:10: 53.5:1.5 and 25: 5: 65: 5, respectively.

The cell transfection experiments showed that the transfection could be performed when the molar ratios of cationic lipids, neutral lipids, structured lipids and polymer-conjugated lipids were 75:5:15:5, 49:10:39.5:1.5, 45:10: 43.5: 1.5, 45: 25: 20: 10, 40:10:48.5:1.5, 35:10: 53.5:1.5 and 25: 5: 65: 5, among which the ratio of 45:10: 43.5: 1.5 had the best transfection effect (FIG. 4). It shows that cationic lipids, neutral lipids, structured lipids and polymer-conjugated lipids can be used to prepare LNP formulation in the molar ratio range of (25 to 75):(5 to 25):(15 to 65):(0.5 to 10), and the optimal ratio is 45: 10: 43.5: 1.5.

The same results were obtained with LNP formulations prepared with YK-201 and YK-209, which are not shown in the figure.

### Example 3: Cell transfection experiment of LNP formulation of eGFP-mRNA

Cell recovery and passaging: 293T cells were recovered and cultured in culture dishes to the required cell number.

Plating: The cells in the culture dish were digested, counted, plated in a 96-well plate at 10,000 cells per well, or in a 12-well plate at 150,000 cells per well, and cultured overnight until the cells adhered to the wall.

Cell transfection experiment: The LNP formulation containing 1.5 µg of eGFP-mRNA prepared in Example 2 (the cationic lipid in the carrier was YK-202) and Lipofectamin 3000 formulation of eGFP-mRNA were added to the cell culture medium in a 12-well plate, and the cells were cultured for 24 hours. The transfection efficiency of different samples was investigated by fluorescence microscopy based on the fluorescence intensity.

According to the results of the experiments, the preparation condition of the nano lipid particles (LNP formulation) were finally determined: the ratio of carrier to mRNA was 15:1; the molar ratio of cationic lipid to neutral lipid was 4.9:1; the polymer-conjugated lipid accounted for 1.5% of the liposomes; the molar ratio of cationic lipid, neutral lipid, structured lipid and polymer-conjugated lipid was 49:10:39.5:1.5. At this ratio, the various cationic lipids designed by the present application and the cationic lipids used in the prior art all had good transfection effects (determined by Example 2, some experimental results are not shown), and the subsequent experiments used this condition to prepare nano lipid particles (LNP formulation).

### Example 4: Preparation (Optimal Ratio) of Nanolipid Particles (LNP Preparation)

**Table 1 Cationic lipid structures**

| Name | Structure | Note |
|---|---|---|
| YK-201 | 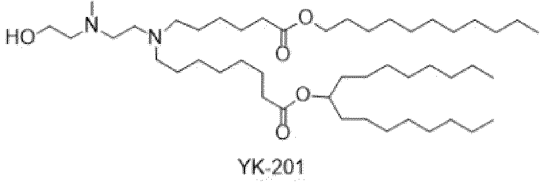 | synthesized in Example 1 |
| YK-202 | 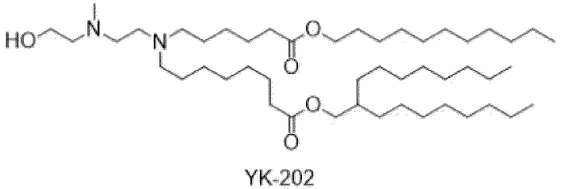 | synthesized in Example 1 |
| YK-203 | 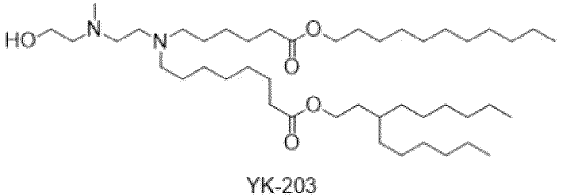 | synthesized in Example 1 |
| YK-204 | 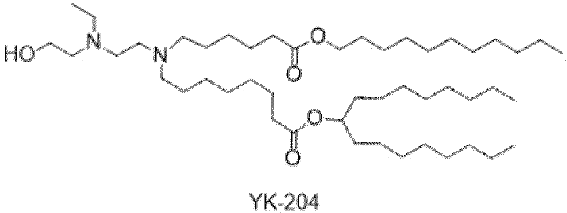 | synth esized in Example 1 |
| YK-205 | 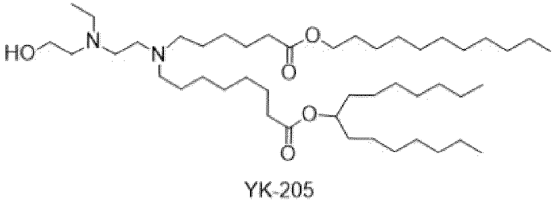 | synthesized in Example 1 |
| YK-206 | 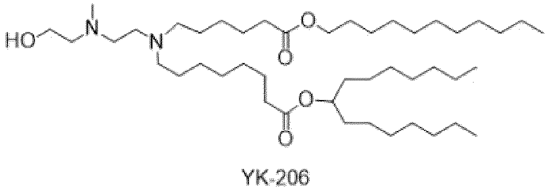 | synthesized in Example 1 |
| YK-207 | 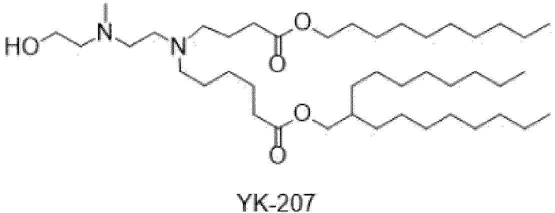 | synthesized in Example 1 |
| YK-208 | 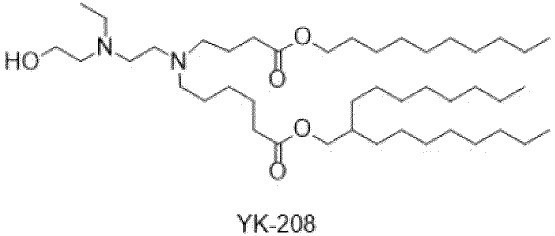 | synthesized in Example 1 |
| YK-209 | 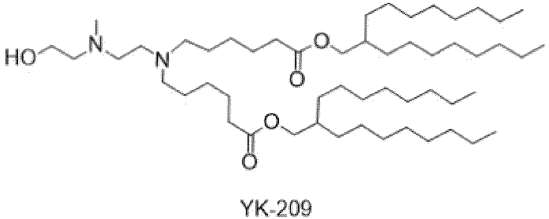 | synth esized in Example 1 |
| YK-210 | 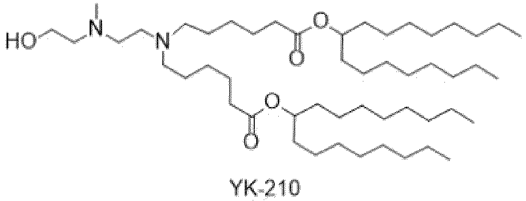 | synthesized in Example 1 |
| YK-211 | 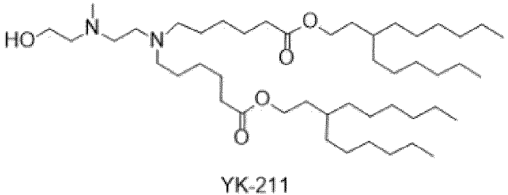 | synthesized in Example 1 |
| YK-212 | 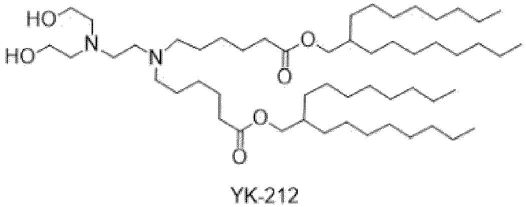 | synthesized in Example 1 |
| YK-213 | 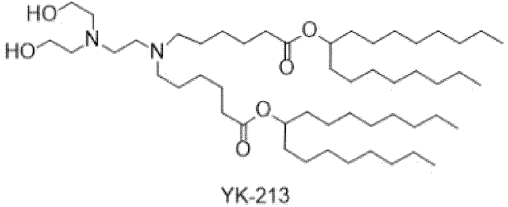 | synthesized in Example 1 |
| YK-214 | 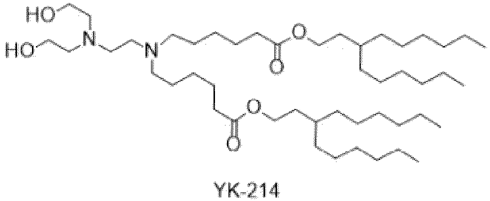 | synthesized in Example 1 |
| YK-215 | 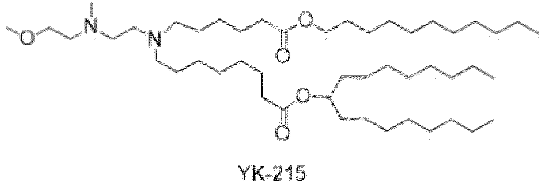 | synthesized in Example 1 |
| YK-216 | 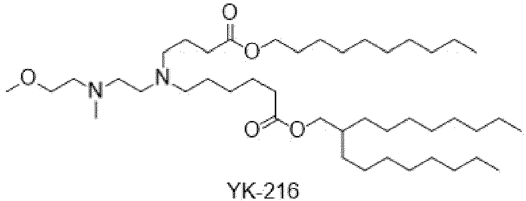 | synthesized in Example 1 |
| YK-217 | 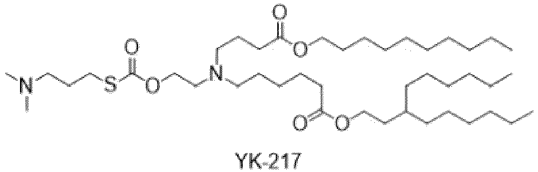 | synthesized in Example 1 |
| YK-218 | 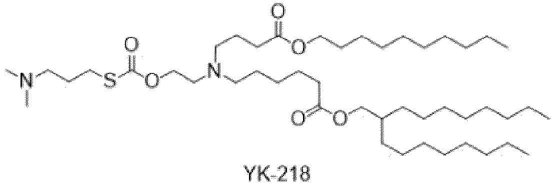 | synthesized in Example 1 |
| YK-219 | 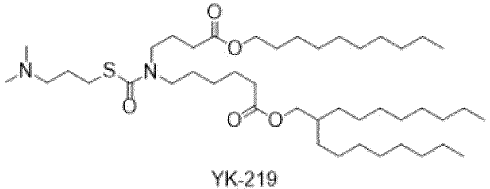 | synthesized in Example 1 |
| YK-220 | 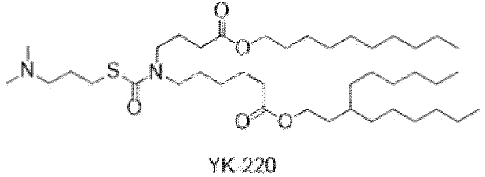 | synth esized in Example 1 |
| YK-221 | 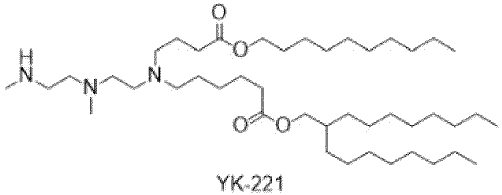 | synthesized in Example 1 |
| YK-222 | 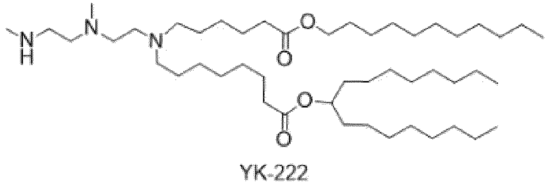 | synthesized in Example 1 |
| YK-223 | 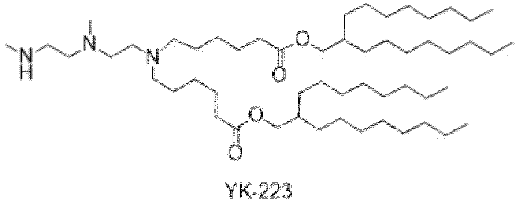 | synthesized in Example 1 |
| YK-224 | 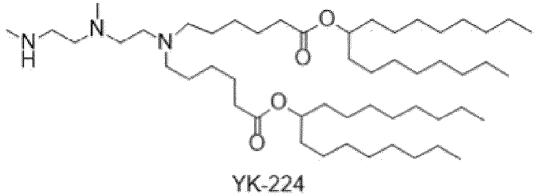 | synthesized in Example 1 |
| YK-225 | 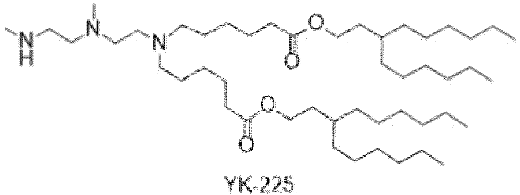 | synth esized in Example 1 |
| YK-226 | 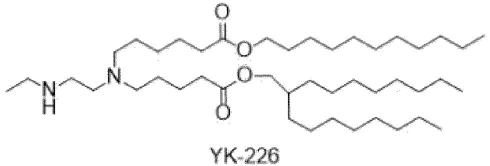 | synthesized in Example 1 |
| YK-009 | 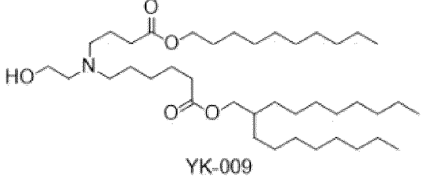 | synthesized in Example 1 YK-009 in CN114044741B |
| SM-102 | 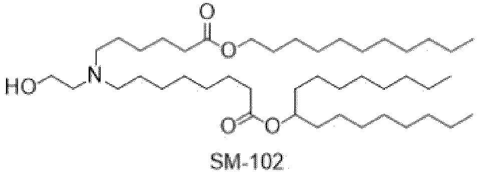 | Purchased from Xiamen Sinopeg Biotech Co., Ltd.; Compound 25 in WO2017049245A2 |
| ALC-0315 | 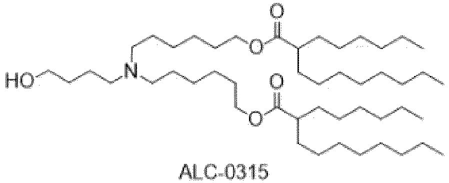 | Purchased from Xiamen Sinopeg Biotech Co., Ltd.: Compound 3 in CN108368028B |
| Compound 21 | 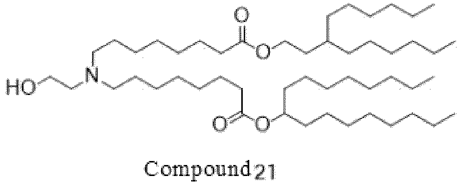 | synthesized in Example 1 Compound 21 in WO2021055833A1 |
| Compound 23 | 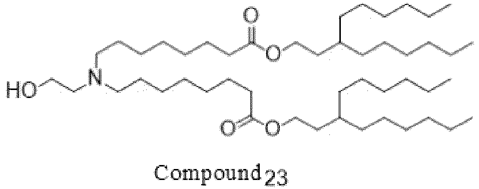 | synthesized in Example 1 Compound 23 in WO2021055833A1 |
| HHMA | 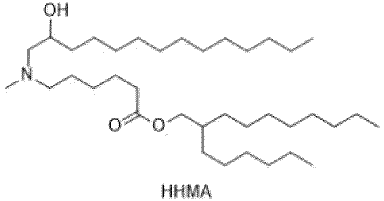 | Purchased from Xiamen Sinopeg Biotech Co., Ltd.; Compound 1 in CN112979483B |

The cationic lipids listed in Table 1 were dissolved in ethanol with DSPC (Aiweituo (Shanghai) Pharmaceutical Technology Co., Ltd.), cholesterol (AVT (Shanghai) Pharmaceutical Technology Co., Ltd.) and DMG-PEG2000 at a molar ratio of 49:10:39.5:1.5 to prepare an ethanol lipid solution. The ethanol lipid solution was quickly added to a citrate buffer (pH = 4 to 5) by ethanol injection method and vortexed for 30 seconds for use. eGFP-mRNA (Shanghai Qifa Experimental Reagent Co., Ltd.) or Fluc-mRNA (Shanghai Qifa Experimental Reagent Co., Ltd.) was diluted in a citrate buffer (pH = 4 to 5) to obtain an mRNA aqueous solution. A certain volume of liposome solution and mRNA aqueous solution were mixed to prepare liposomes at a weight ratio of total lipid to mRNA of 15:1. Ultrasonication was performed at 25 °C for 15 min (ultrasonic frequency 40 kHz, ultrasonic power 800 W). The obtained liposomes were diluted to 10 times the volume with PBS, and then ultrafiltered to remove ethanol using a 300KDa ultrafiltration tube. The mixture was then diluted to a certain volume with PBS to obtain an LNP preparation encapsulating eGFP-mRNA or Fluc-mRNA using cationic lipid/DSPC/cholesterol/DMG-PEG2000 (molar percentage of 49:10:39.5:1.5).

Lipofectamine 3000 transfection reagent is currently widely used for cell transfection. It has very good transfection performance and excellent transfection efficiency, can improve cell activity, and is suitable for difficult-to-transfect cell types. Lipofectamine 3000 transfection reagent was used as a control herein and Lipofectamine 3000 formulations of eGFP-mRNA or Fluc-mRNA were prepared according to the method in the instruction manual of Lipofectamine 3000 (Invitrogen (Shanghai) Trading Co., Ltd.).

### Example 5: Determination of particle size and polydispersity index (PDI) of nanolipid particles

The particle size and polydispersity index (PDI) were determined by dynamic light scattering method using a Malvern laser particle size analyzer. 10 µL of liposome solution was diluted to 1 mL with RNase-free deionized water and added to a sample pool. Each sample was measured three times. The measurement conditions were: 90° scattering angle, 25 °C. The test results are as follows:

**Table 2 Particle size and polydispersity index (PDI)**

| Name | Particle size(nm) | PDI (%) |
|---|---|---|
| YK-201 | 205 | 25.5 |
| YK-202 | 173 | 23.4 |
| YK-203 | 235 | 23.7 |
| YK-204 | 193 | 17.7 |
| YK-205 | 219 | 17.7 |
| YK-206 | 216 | 18.8 |
| YK-207 | 192 | 18.9 |
| YK-208 | 167 | 24.4 |
| YK-209 | 226 | 26.6 |
| YK-2 10 | 224 | 22.9 |
| YK-211 | 222 | 20.7 |
| YK-212 | 172 | 19.2 |
| YK-213 | 207 | 17.6 |
| YK-214 | 203 | 31.5 |
| YK-215 | 219 | 20.8 |
| YK-216 | 219 | 13.7 |
| YK-217 | 210 | 17.9 |
| YK-218 | 222 | 17.2 |
| YK-219 | 217 | 22.4 |
| YK-220 | 221 | 26.6 |
| YK-221 | 237 | 21.9 |
| YK-222 | 218 | 30.4 |
| YK-223 | 211 | 25.8 |
| YK-224 | 185 | 30.3 |
| YK-225 | 222 | 19.3 |
| YK-226 | 246 | 17.0 |
| YK-009 | 202 | 13.0 |
| SM-102 | 145 | 19.2 |
| ALC-0315 | 218 | 15.9 |
| Compound 21 | 155 | 15.5 |
| Compound 23 | 138 | 8.6 |
| HMMA | 135 | 15.1 |

The particle size of the nanolipid particles prepared in Example 4 was between 130 and 250 nm, and can be used to deliver mRNA. The particle size prepared by HMMA was the smallest, which was 135 nm, and the particle size prepared by YK-226 was the largest, which was 246 nm. The polydispersity index of all nanolipid particles was between 5% and 35%, wherein the smallest was that of compound 23, which was 8.6%, and the largest was that of YK-214, which was 31.5%.

### Example 6: Encapsulation rate, drug loading concentration and total RNA concentration detection

Encapsulation rate is a key quality attribute of liposomes. It refers to the percentage of drug content encapsulated in the lipid bilayer to the total amount of drug added, which can reflect the degree of drug encapsulation in liposomes. The Chinese Pharmacopoeia stipulates that the encapsulation rate should usually not be less than 80%.

The drug loading capacity is the ratio of the amount of drug in the liposome to the total amount of drug and carrier in the liposome. The drug loading capacity directly affects the clinical application dose of the drug, so the larger the drug loading capacity, the more it can meet clinical needs. The drug loading concentration is proportional to the drug loading capacity, and the relative proportion of the drug loading concentration can represent the relative proportion of the drug loading capacity. The relative proportion of the total RNA concentration can represent the relative proportion of the amount of mRNA carried by the LNP formulation.

### Reagent preparation:

1×TE buffer, 0.1% Triton X-100 buffer, and RiboGreen reagent (1: 200) and mRNA standard stock solution were prepared.

### Sample detection:

An appropriate amount of sample was taken and added to an appropriate amount of 1×TE buffer, which was diluted to a solution containing about 2.8 µg of the test sample per 1 mL. Then the sample was added to a 96-well plate, where 50 µL of the diluted test sample or mRNA standard stock solution was added to each well, and then added with 1×TE buffer and 0.1% Triton X-100 buffer. The samples were incubated at 37 °C for 10 minutes, and 100 µL of RiboGreen reagent (1: 200) was added to each well of the 96-well plate. The plate was centrifuged, and read using an ELISA reader, and the data was processed. Specific data are shown in Tables 3 and 4.

### Experimental results:

(1) Compared with the cationic lipids in the prior art, the encapsulation rate, drug loading concentration and total RNA concentration of the LNP formulations prepared by YK-201, YK-202, YK-206, YK-207 and YK-209 were significantly improved. For example, the encapsulation rate of YK-209 was 41% higher than that of compound 23, and the drug loading concentration of YK-209 was 2 times that of compound 23; the total RNA concentration of YK-201 was 1.5 times that of compound 21.

**Table 3 Encapsulation rate, drug loading concentration and total RNA concentration detection results-1**

| Number | Name | Encapsulation rate(%) | Drug loading concentration (µg/mL) | Total RNA concentration (µg/mL) |
|---|---|---|---|---|
| 1 | YK-201 | 89.5 | 40.32 | 45.07 |
| 2 | YK-202 | 86.3 | 37.08 | 42.98 |
| 3 | YK-206 | 91.4 | 38.09 | 41.69 |
| 4 | YK-207 | 90.8 | 39.60 | 43.63 |
| 5 | YK-209 | 96.2 | 41.76 | 43.42 |
| 6 | YK-009 | 63.0 | 26.86 | 38.62 |
| 7 | SM-102 | 62.3 | 25.44 | 35.27 |
| 8 | ALC-0315 | 62.8 | 26.05 | 37.12 |
| 9 | 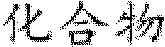 21 | 57.8 | 22.6 | 30.69 |
| 10 | 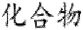 23 | 55.2 | 20.7 | 32.51 |
| 11 | HMMA | 60.5 | 30.6 | 37.84 |

As can be seen from Table 3, the encapsulation rate of LNP formulations prepared from different compounds varies greatly. YK-201, YK-202, YK-206, YK-207 and YK-209 have significantly improved encapsulation rate compared with SM-102, ALC-0315, compound 21, compound 23, HHMA and YK-009.

The encapsulation rate of YK-201 was 89.5%, which was 27.20% higher than that of SM-102, 26.70% higher than that of ALC-0315, 31.70% higher than that of compound 21, 34.30% higher than that of compound 23, 29.00% higher than that of HHMA, and 26.50% higher than that of YK-009.

The encapsulation rate of YK-202 was 86.3%, which was 24.00% higher than that of SM-102, 23.50% higher than that of ALC-0315, 28.50% higher than that of compound 21, 31.10% higher than that of compound 23, 25.80% higher than that of HHMA, and 23.30% higher than that of YK-009.

The encapsulation rate of YK-206 was 91.4%, which was 29.10% higher than that of SM-102, 28.60% higher than that of ALC-0315, 33.60% higher than that of compound 21, 36.20% higher than that of compound 23, 30.90% higher than that of HHMA, and 28.40% higher than that of YK-009.

The encapsulation rate of YK-207 was 90.8%, which was 28.50% higher than that of SM-102, 28.00% higher than that of ALC-0315, 33.00% higher than that of compound 21, 35.60% higher than that of compound 23, 30.30% higher than that of HHMA, and 27.80% higher than that of YK-009.

The encapsulation rate of YK-209 was 96.2%, which was 33.90% higher than that of SM-102, 33.40% higher than that of ALC-0315, 38.40% higher than that of compound 21, 41.00% higher than that of compound 23, 35.70% higher than that of HHMA, and 33.20% higher than that of YK-009.

Moreover, the drug loading concentration and total RNA concentration of LNP formulations prepared from different compounds also varied greatly. Compared with SM-102, ALC-0315, compound 21, compound 23 and YK-009, the drug loading concentration and total RNA concentration of YK-201, YK-202, YK-206, YK-207 and YK-209 were significantly increased.

The drug loading concentration of YK-201 was 40.32µg/mL, and the total RNA concentration of YK-201 was 45.07µg/mL, which were 1.58 and 1.28 times that of SM-102, 1.55 and 1.21 times that of ALC-0315, 1.78 and 1.47 times that of compound 21, 1.95 and 1.39 times that of compound 23, 1.32 and 1.19 times that of HHMA, and 1.50 and 1.17 times that of YK-009.

The drug loading concentration of YK-202 was 37.08µg/mL, and the total RNA concentration was 42.98µg/mL, which were 1.46 and 1.22 times that of SM-102, 1.42 and 1.16 times that of ALC-0315, 1.64 and 1.40 times that of compound 21, 1.79 and 1.32 times that of compound 23, 1.21 and 1.14 times that of HHMA, and 1.38 and 1.11 times that of YK-009.

The drug loading concentration of YK-206 was 38.09µg/mL, and the total RNA concentration was 41.69µg/mL, which were 1.50 and 1.18 times that of SM-102, 1.46 and 1.12 times that of ALC-0315, 1.69 and 1.36 times that of compound 21, 1.84 and 1.28 times that of compound 23, 1.24 and 1.10 times that of HHMA, and 1.42 and 1.08 times that of YK-009.

The drug loading concentration of YK-207 was 39.60µg/mL, and the total RNA concentration was 43.63µg/mL, which were 1.56 and 1.24 times that of SM-102, 1.52 and 1.18 times that of ALC-0315, 1.75 and 1.42 times that of compound 21, 1.91 and 1.34 times that of compound 23, 1.29 and 1.15 times that of HHMA, and 1.47 and 1.13 times that of YK-009.

The drug loading concentration of YK-209 was 41.76µg/mL, and the total RNA concentration was 43.42µg/mL, which were 1.64 and 1.23 times that of SM-102, 1.60 and 1.17 times that of ALC-0315, 1.85 and 1.41 times that of compound 21, 2.02 and 1.34 times that of compound 23, 1.36 and 1.15 times that of HHMA, and 1.55 and 1.12 times that of YK-009.

The data were analyzed using GraphPad Prism software. Any one of YK-201, YK-202, YK-206, YK-207 and YK-209 showed significant differences compared with SM-102, ALC-0315, compound 21, compound 23, HHMA and YK-009, and the encapsulation rate, drug loading concentration and total RNA concentration were significantly improved.

### Summary:

The LNP formulations prepared from some of the designed compounds, including YK-201, YK-202, YK-206, YK-207 and YK-209, have significantly improved encapsulation rate, drug loading concentration and total RNA concentration compared to prior art cationic lipids, such as SM-102, ALC-0315, compound 21, compound 23, HHMA and YK-009. For example, the encapsulation rate of YK-209 can be increased by 41% compared with compound 23, and the drug loading concentration of YK-209 can reach 2 times that of compound 23; the total RNA concentration of YK-201 can reach 1.5 times that of compound 21.

(2) The encapsulation rate and drug loading capacity of LNP formulations prepared from different designed compounds varied greatly. The encapsulation rate of different compounds ranged from 55% to 99%, the drug loading concentration ranged from 25 to 45 µg/mL, and the total RNA concentration ranged from 25 to 50 µg/mL.

**Table 4 Encapsulation rate, drug loading concentration and total RNA concentration detection results-2**

| Number | Name | Encapsulation rate (%) | Drug loading concentration (µg/mL) | Total RNA concentration (µg/mL) |
|---|---|---|---|---|
| 1 | YK-201 | 89.5 | 40.32 | 45.07 |
| 2 | YK-202 | 86.3 | 37.08 | 42.98 |
| 3 | YK-206 | 91.4 | 38.09 | 41.69 |
| 4 | YK-207 | 90.8 | 39.60 | 43.63 |
| 5 | YK-209 | 96.2 | 41.76 | 43.42 |
| 6 | YK-203 | 91.0 | 38.38 | 42.19 |
| 7 | YK-204 | 75.9 | 32.62 | 42.98 |
| 8 | YK-205 | 68.4 | 30.02 | 43.92 |
| 9 | YK-208 | 88.2 | 37.80 | 42.84 |
| 10 | YK-210 | 85.6 | 36.50 | 42.62 |
| 11 | YK-211 | 97.7 | 42.91 | 43.92 |
| 12 | YK-212 | 94.4 | 40.25 | 42.62 |
| 13 | YK-213 | 82.0 | 34.70 | 42.34 |
| 14 | YK-214 | 96.7 | 38.16 | 39.46 |
| 15 | YK-215 | 68.0 | 29.30 | 43.13 |
| 16 | YK-216 | 61.3 | 27.36 | 44.64 |
| 17 | YK-217 | 90.4 | 42.19 | 46.66 |
| 18 | YK-218 | 78.2 | 36.50 | 46.66 |
| 19 | YK-219 | 64.2 | 30.31 | 47.23 |
| 20 | YK-220 | 57.5 | 25.27 | 43.92 |
| 21 | YK-221 | 95.4 | 31.46 | 32.98 |
| 22 | YK-222 | 94.0 | 25.78 | 27.43 |
| 23 | YK-223 | 98.3 | 37.51 | 38.16 |
| 24 | YK-224 | 98.7 | 38.81 | 39.31 |
| 25 | YK-225 | 98.0 | 38.74 | 39.53 |
| 26 | YK-226 | 95.2 | 32.90 | 34.56 |

As can be seen from Table 4, the designed series of compounds have an encapsulation rate of 55% to 99%, a drug loading concentration of 25 to 45 µg/mL, and a total RNA concentration of 25 to 50µg/mL. There were great differences between different compounds. The highest encapsulation rate was YK-224, reaching 98.7%, and the lowest was YK-220, which was only 57.5%; the highest drug loading concentration was YK-217, which was 42.19 µ g/mL, and the lowest was YK-220, which was only 25.27 µ g/mL; the highest total RNA concentration was YK-219, reaching 47.23 µ g/mL, and the lowest was YK-222, which was only 27.43 µ g/mL.

### Summary:

The encapsulation rate and drug loading of LNP formulations prepared by different designed compounds varied greatly. The encapsulation rate of different compounds ranged from 55% to 99%, the drug loading concentration ranged from 25 to 45µg/mL, and the total RNA concentration ranged from 25 to 50µg/mL. It can be seen that LNP formulations prepared by compounds with similar structures do not necessarily have similar encapsulation rate and drug loading capacity.

### Conclusion:

The LNP formulations prepared by some of the designed compounds, including YK-201, YK-202, YK-206, YK-207 and YK-209, have significantly improved encapsulation rate, drug loading concentration and total RNA concentration compared to prior art cationic lipids, such as SM-102, ALC-0315, compound 21, compound 23, HHMA and YK-009. For example, the encapsulation rate of YK-209 can be increased by 41% compared with compound 23, and the drug loading concentration can reach 2 times that of compound 23; the total RNA concentration of YK-201 can reach 1.5 times that of compound 21.

It can be seen that when YK-201, YK-202, YK-206, YK-207 and YK-209 are used to prepare carriers for delivering mRNA, the encapsulation rate and drug loading capacity are significantly improved, so the amount of LNP used can be significantly reduced. In the application of multivalent mRNA vaccines, it can provide LNP-mRNA formulation products with more uniform distribution and more controllable quality.

The encapsulation rate and drug loading capacity of LNP formulations prepared from different compounds in this series of designed compounds varied greatly, with encapsulation rate ranging from 55% to 99%, drug loading concentration between 25 and 45µg/mL, and total RNA concentration between 25 and 50µg/mL. It can be seen that LNP formulations prepared from compounds with similar structures do not necessarily have similar encapsulation rate and drug loading capacity. On the contrary, the encapsulation rate and drug loading capacity are likely to have significant differences, so it is impossible to infer the encapsulation rate and drug loading capacity of the LNP formulations prepared from the compounds based on the structure of the compound.

### Example 7: In vitro validation of the performance of LNP delivery vehicles

Cell recovery and passage: The method was the same as in Example 3.

Plating: The method was the same as Example 3.

### 1. Fluorescence detection of Fluc-mRNA

The LNP formulation containing 0.3 µg Fluc-mRNA (prepared according to Example 4, the LNP formulation carrier components were cationic lipids, neutral lipids, structured lipids and polymer-conjugated lipids at a molar ratio of 49:10:39.5:1.5, and the cationic lipids were cationic lipids listed in Table 1) was added to the cell culture medium of a 96-well plate, and cultured for 24 hours. After that, the corresponding reagents were added according to the instructions of the Gaussia Luciferase Assay Kit, and the fluorescence expression intensity of each well was detected by the IVIS fluorescence detection system. This experiment verified the transfection efficiency of the LNP formulation in the cell, and the specific detection results are shown in Tables 6-9.

Experimental results:
(1) The compounds of the present application, including YK-201, YK-202, YK-206, YK-207 and YK-209, are very different from the cationic lipids of the prior art in chemical structures.

The series of compounds designed in the present application, including YK-201, YK-202, YK-206, YK-207 and YK-209, are very different from the cationic lipids in the prior art in chemical structures. For example, these compounds are completely different from the HHMA structure; compared with SM-102, ALC-0315, compound 21, compound 23 and YK-009, the G₃ group is completely different, and the G₁, G₂, R₁ and R₂ groups are also significantly different. The specific structural comparison is shown in Table 5.

**Table 5 Structural comparison of the designed compounds with representative cationic lipids in the prior art**

| Name | Structure |
|---|---|
| YK-201 | 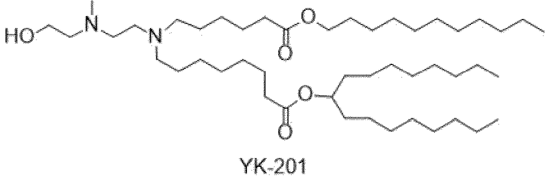 |
| YK-202 | 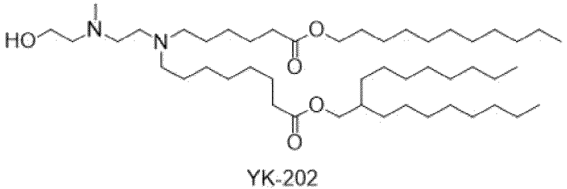 |
| YK-206 | 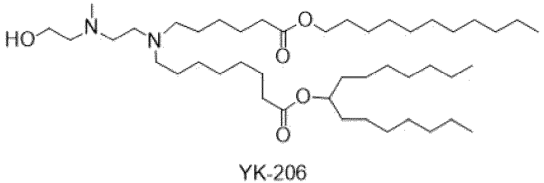 |
| YK-207 | 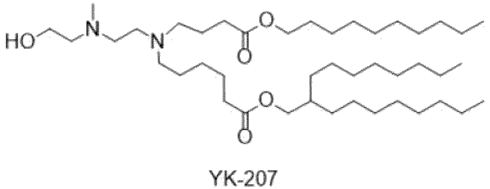 |
| YK-209 | 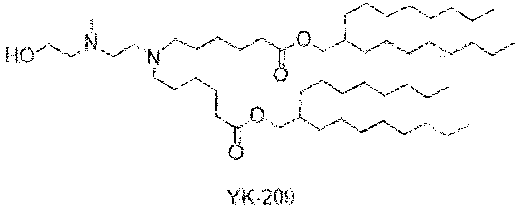 |
| YK-009 | 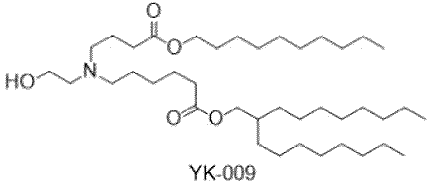 |
| SM-102 | 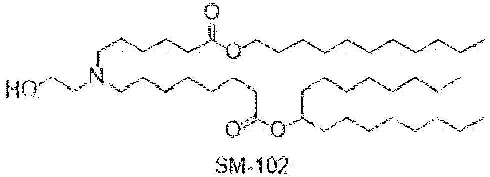 |
| ALC-0315 | 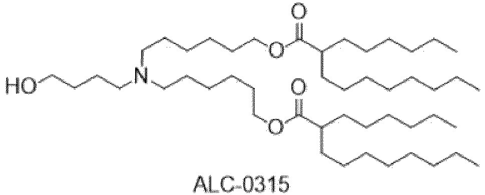 |
| Compound 21 | 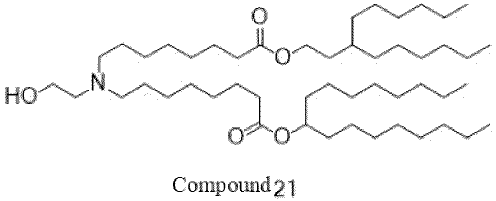 |
| Compound 23 | 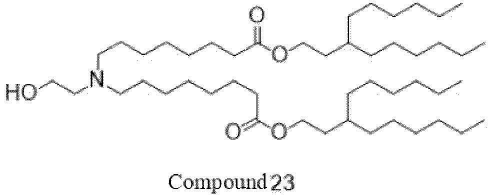 |
| HHMA | 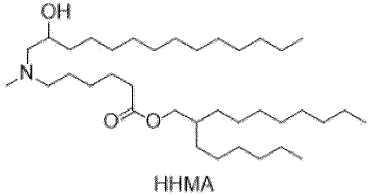 |

As can be seen from Table 3, this series of designed compounds, including YK-201, YK-202, YK-206, YK-207 and YK-209, are significantly different from the representative cationic lipids in the prior art in chemical structures. YK-009 is disclosed in CN114044741B (claim 1), SM-102 is compound 25 disclosed in WO2017049245A2 (page 29 of the specification), ALC-0315 is compound 3 disclosed in CN108368028B (page 24 of the specification), compounds 21 and 23 are disclosed in WO2021055833A1 (page 22 of the specification), and HHMA is compound 1 disclosed in CN112979483B (page 12 of the specification).

### Compared with this series of compounds:

a. The HHMA is the most different in structure. From the chemical structure, it can be seen that the group connected to the central N atom of HHMA has only one side chain similar to one side chain of this series of structures, and the other parts are completely different.
b. Other cationic lipids in the prior art, such as SM-102, ALC-0315, compound 21, compound 23 and YK-009, have completely different G₃ groups. Due to the different structures of the G₃ groups, they also have great differences in polarity, acidity and alkalinity, and hydrophilicity.
c. The G₁, R₁, G₂ and R₂ groups of SM-102, ALC-0315, compound 21, compound 23 and YK-009 are also significantly different.

The details are as follows:
I. YK-201

YK-201 has significant structural differences compared to the cationic lipids in the prior art, such as SM-102, compound 21, compound 23, YK-009, and HHMA.

Compared with SM-102, the G₃ group of YK-201 is completely different, which is HO(CH₂)₂N(CH₃)(CH₂)₂-, while the G₃ group of SM-102 is HO(CH₂)₂-.

Compared with ALC-0315, the G₁ group of YK-201 has one less C than that of ALC-0315; the R₁ group is a straight chain structure, while ALC-0315 is a branched structure; the G₂ group has one more C than that of ALC-0315; one single chain in the double chains of the R₂ group has two more C; the G₃ group is completely different, which is HO(CH₂)₂N(CH₃)(CH₂)₂- In addition, the directions of the ester bonds between the G₁ group and the R₁ group, and between the G₂ group and the R₂ group, are also different in YK-201 and ALC-0315.

Compared with compound 21, the G₁ group of YK-201 has 2 less C than compound 21; the R₁ group is a straight chain structure, while compound 21 is a branched structure; the G₃ group is completely different, which is HO(CH₂)₂N(CH₃)(CH₂)₂-.

Compared with compound 23, the G₁ group of YK-201 has 2 less C than that of compound 23; the R₁ group is a straight chain structure, while compound 23 is a branched structure; the R₂ group has 2 less C in a single chain, and 2 more C in each single chain of the double chain; the G₃ group is completely different, which is HO(CH₂)₂N(CH₃)(CH₂)₂-.

Compared with YK-009, the G₁ group of YK-201 has 2 more C than that of YK-009; the R₁ group has 1 more C; the G₂ group has 2 more C; the R₂ group has 1 less C in a single chain; and the G₃ group is completely different, which is HO(CH₂)₂N(CH₃)(CH₂)₂-.

Compared with HHMA, the structure of YK-201 is completely different. Only one side chain connected to the N atom of HHMA is similar to the side chain structure of YK-201, and the other parts are significantly different.

### II. YK-202

YK-202 has significant structural differences compared to prior art cationic lipids, such as SM-102, compound 21, compound 23, YK-009, and HHMA.

Compared with SM-102, the single chain of the R₂ group of YK-202 has one more C than that of SM-102; the G₃ group is completely different, which is HO(CH₂)₂N(CH₃)(CH₂)₂-.

Compared with ALC-0315, the G₁ group of YK-202 has one less C than that of ALC-0315; the R₁ group is a straight chain structure, while that of ALC-0315 is a branched structure; the G₂ group has one more C; the R₂ group has one more C in a single chain, and two more C in a single chain in the double chains; the G₃ group is completely different, which is HO(CH₂)₂N(CH₃)(CH₂)₂-. In addition, the directions of the ester bonds between the G₁ group and the R₁ group, and between the G₂ group and the R₂ group, are also different for YK-201 and ALC-0315.

Compared with compound 21, the G₁ group of YK-202 has 2 less C; the R₁ group is a straight chain structure, while compound 21 is a branched chain structure; the R₂ group has 1 more C in a single chain than that of compound 21; and the G₃ group is completely different, which is HO(CH₂)₂N(CH₃)(CH₂)₂-.

Compared with compound 23, the G₁ group of YK-202 has 2 less C; the R₁ group is a straight chain structure, while that of compound 23 is a branched structure; the R₂ group has 1 less C in the single chain, and each single chain in the double chain has 2 more C; the G₃ group is completely different, which is HO(CH₂)₂N(CH₃)(CH₂)₂-.

Compared with YK-009, the G₁ group of YK-202 has 2 more C; the R₁ group has 1 more C; the G₂ group has 2 more C; and the G₃ group is completely different, which is HO(CH₂)₂N(CH₃)(CH₂)₂-.

Compared with HHMA, the structure of YK-202 is completely different. Only one side chain connected to the N atom of HHMA is similar to one side chain of YK-202 in structure, and the other parts are significantly different.

### III. YK-206

YK-206 has significant structural differences compared to prior art cationic lipids, such as SM-102, compound 21, compound 23, YK-009, and HHMA.

Compared with SM-102, the R₂ group of YK-206 has one less C in each single chain in the double chain; the G₃ group is completely different, being HO(CH₂)₂N(CH₃)(CH₂)₂-.

Compared with ALC-0315, the G₁ group of YK-206 has one less C than that of ALC-0315; the R₁ group is a straight chain structure, while that of ALC-0315 is a branched structure; the G₂ group has one more C; one single chain of the double chain of the R₂ group has one more C, and one single chain has one less C; the G₃ group is completely different, which is HO(CH₂)₂N(CH₃)(CH₂)₂-. In addition, the directions of the ester bonds between the G₁ group and the R₁ group, and between the G₂ group and the R₂ group, are also different for YK-201 and ALC-0315.

Compared with compound 21, the G₁ group of YK-206 has 2 less carbon atoms; the R₁ group is a straight chain structure, while that of compound 21 is a branched structure; each single chain in the double chain of the R₂ group has 1 less C; and the G₃ group is completely different, being HO(CH₂)₂N(CH₃)(CH₂)₂-.

Compared with compound 23, the G₁ group of YK-206 has 2 less C; the R₁ group is a straight chain structure, while that of compound 23 is a branched structure; the R₂ group has 2 less C in the single chain and 1 less C in each single chain of the double chain; the G₃ group is completely different, being HO(CH₂)₂N(CH₃)(CH₂)₂-.

Compared with YK-009, the G₁ group of YK-206 has 2 more C; the R₁ group has 1 more C; the G₂ group has 2 more C; the R₂ group has 1 less C in the single chain and 1 less C in each single chain of the double chain; the G₃ group is completely different, being HO(CH₂)₂N(CH₃)(CH₂)₂-.

Compared with HHMA, the structure of YK-206 is completely different. Only one side chain connected to the N atom of HHMA is similar to one side chain of YK-206 in structure, and the other parts are significantly different.

### IV. YK-207

YK-207 has significant structural differences compared to the cationic lipids in the prior art, such as SM-102, compound 21, compound 23, YK-009, and HHMA.

Compared with SM-102, the G₁ group of YK-207 has 2 less C; the R₁ group has 1 less C; the G₂ group has 2 less C; the R₂ group has 1 more C in a single chain; and the G₃ group is completely different, being HO(CH₂)₂N(CH₃)(CH₂)₂-.

Compared with ALC-0315, the G₁ group of YK-207 has 3 less C than ALC-0315; the R₁ group is a straight chain structure, while that of ALC-0315 is a branched structure; the G₂ group has 1 less carbon atom; the R₂ group has 1 more carbon atom in a single chain, and 2 more carbon atoms in one single chain of the double chain; the G₃ group is completely different, being HO(CH₂)₂N(CH₃)(CH₂)₂-. In addition, the directions of the ester bonds between the G₁ group and the R₁ group, and between the G₂ group and the R₂ group, are also different for YK-201 and ALC-0315.

Compared with compound 21, the G₁ group of YK-207 has 4 less C; the R₁ group is a straight chain structure, while that of compound 21 is a branched structure; the G₂ group has 2 less C; the R₂ group has 1 more C in a single chain; and the G₃ group is completely different, being HO(CH₂)₂N(CH₃)(CH₂)₂-.

Compared with compound 23, the G₁ group of YK-207 has 4 less C; the R₁ group is a straight chain structure, while that of compound 23 is a branched structure; the G₂ group has 2 less C; the R₂ group single chain has 1 less C, and each single chain in the double chain has 2 more C; the G₃ group is completely different, being HO(CH₂)₂N(CH₃)(CH₂)₂-.

Compared with HHMA, the structure of YK-207 is completely different. Only one side chain connected to the N atom of HHMA is similar to the side chain structure of YK-207, and the other parts are significantly different.

### V. YK-209

YK-209 has significant structural differences compared to the cationic lipids in the prior art, such as SM-102, compound 21, compound 23, YK-009, and HHMA.

Compared with SM-102, the R₁ group of YK-209 is a branched structure, while SM-102 is a straight chain structure; the G₂ group of YK-209 has 2 less C; the R₂ group has 1 more C in a single chain; the G₃ group is completely different, which is HO(CH₂)₂N(CH₃)(CH₂)₂-.

Compared with ALC-0315, the G₁ group of YK-209 has one less C than that of ALC-0315; the R₁ group is a straight chain structure, while that of ALC-0315 is a branched structure; the G₂ group has one less C; the R₂ group has one more C in a single chain, and two more C in one single chain of the double chain; the G₃ group is completely different, being HO(CH₂)₂N(CH₃)(CH₂)₂-. In addition, the directions of the ester bonds between the G₁ group and the R₁ group, and between the G₂ group and the R₂ group, are also different for YK-201 and ALC-0315.

Compared with compound 21, the G₁ group of YK-209 has 2 less C; the R₁ group has 1 less C in the single chain and 2 more C in each single chain of the double chain; the G₂ group has 2 less C; the R₂ group has 1 more C in the single chain; and the G₃ group is completely different, being HO(CH₂)₂N(CH₃)(CH₂)₂-.

Compared with compound 23, the G₁ group of YK-209 has 2 less C; the R₁ group has 1 less C in a single chain, and each single chain in the double chain has 2 more C; the G₂ group has 2 less C; the R₂ group has 1 less C in a single chain, and each single chain in the double chain has 2 more C; the G₃ group is completely different, being HO(CH₂)₂N(CH₃)(CH₂)₂-.

Compared with HHMA, the structure of YK-209 is completely different. Only one side chain connected to the N atom of HHMA is similar to one side chain of YK-209 in structure, and the other parts are significantly different.

From the above comparison, it can be seen that the designed series of compounds, including YK-201, YK-202, YK-206, YK-207 and YK-209, are very different in chemical structure from the cationic lipid compounds in the prior art, such as SM-102, ALC-0315, compound 21, compound 23, HHMA and YK-009. This series of compounds is completely different from HHMA in structure; their G₃ groups are completely different from those of SM-102, ALC-0315, compound 21, compound 23 and YK-009, and their G₁, G₂, R₁ and R₂ groups are also significantly different.

Due to the significant differences in chemical structure, the physicochemical properties of this series of compounds, such as polarity, acidity and alkalinity, and hydrophilicity, are also significantly different from those of SM-102, ALC-0315, compound 21, compound 23, HHMA and YK-009. Therefore, it is impossible to infer the cell transfection efficiency, cytotoxicity, and in vivo expression of the LNP formulations prepared from this series of compounds based on the above cationic lipid compounds disclosed in the prior art.

(2) Among the designed series of compounds, the LNP formulations prepared from YK-201, YK-202 and YK-209 have the highest cell transfection efficiency, which is significantly higher than that of the representative cationic lipids in the prior art. For example, YK-202 can reach 18 times that of SM-102, 21 times that of compound 21 and 22 times that of compound 23.

**Table 6 Fluorescence detection results of Fluc-mRNA-1**

| No. | Name | Relative light unit (RLU) | Ratio relative to SM-102 | Multiples of values that equivalent to YK-201 | Multiples of values that equivalent to YK-202 | Multiples of values that equivalent to YK-209 |
|---|---|---|---|---|---|---|
| 1 | YK-201 | 28482617 | 17.10 | 1.00 | 1.05 | 0.94 |
| 2 | YK-202 | 29957419 | 17.98 | 0.95 | 1.00 | 0.90 |
| 3 | YK-206 | 21058746 | 12.64 | 1.35 | 1.42 | 1.28 |
| 4 | YK-207 | 20878758 | 12.53 | 1.36 | 1.43 | 1.29 |
| 5 | YK-209 | 26861465 | 16.12 | 1.06 | 1.12 | 1.00 |
| 6 | YK-009 | 5125986 | 3.08 | 5.56 | 5.84 | 5.24 |
| 7 | SM-102 | 1665874 | 1.00 | 17.10 | 17.98 | 16.12 |
| 8 | ALC-0315 | 2147045 | 1.29 | 13.27 | 13.95 | 12.51 |
| 9 | Compound 21 | 1421028 | 0.85 | 20.04 | 21.08 | 18.90 |
| 10 | Compound 23 | 1332014 | 0.80 | 21.38 | 22.49 | 20.17 |
| 11 | HHMA | 2099714 | 1.26 | 13.56 | 14.27 | 12.79 |
| 12 | Lipofectamine 3000 | 1201248 | 0.72 | 23.71 | 24.94 | 22.36 |

### Differences in cell transfection efficiency

Table 6 lists the fluorescence detection results of LNP formulations containing Fluc-mRNA prepared by different cationic lipids. Among them, YK-009 is disclosed in CN114044741B (claim 1), compound 21 and compound 23 are disclosed in WO2021055833A1 (page 22 of the specification), SM-102 is compound 25 disclosed in WO2017049245A2 (page 29 of the specification), ALC-0315 is compound 3 disclosed in CN108368028B (page 24 of the specification), and HHMA is compound 1 disclosed in CN112979483B (page 12 of the specification); Lipofectamine 3000 is a widely used cell transfection reagent with good transfection performance.

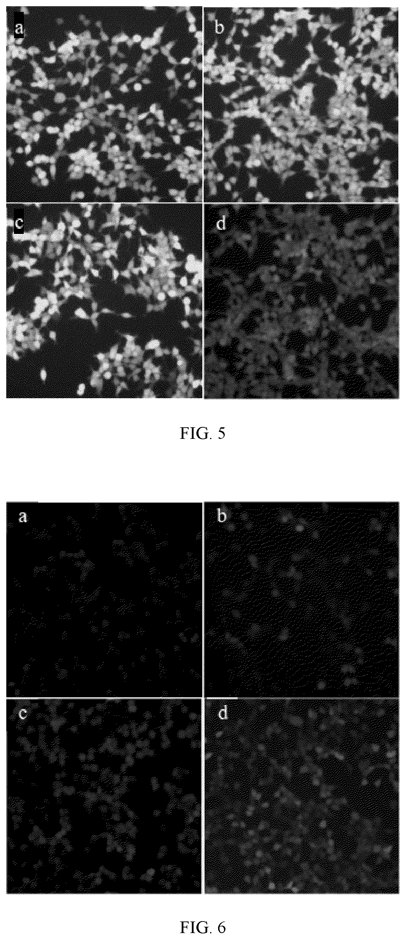

As shown in Table 6 and Figure 5, the LNP formulations containing Fluc-mRNA prepared by YK-201, YK-202 and YK-209 have the strongest fluorescence absorption, and the RLU values are 28482617, 29957419 and 26861465, respectively.

The RLU value of YK-201 can reach 17.10 times that of SM-102, 13.27 times that of ALC-0315, 20.04 times that of compound 21, 21.38 times that of compound 23, 13.56 times that of HHMA, 23.71 times that of Lipofectamine 3000 and 5.56 times that of YK-009.

The RLU value of YK-202 can reach 17.98 times that of SM-102, 13.95 times that of ALC-0315, 21.08 times that of compound 21, 22.49 times that of compound 23, 14.27 times that of HHMA, 24.94 times that of Lipofectamine 3000 and 5.84 times that of YK-009.

The RLU value of YK-209 can reach 16.12 times that of SM-102, 12.51 times that of ALC-0315, 18.90 times that of compound 21, 20.17 times that of compound 23, 12.79 times that of HHMA, 22.36 times that of Lipofectamine 3000 and 5.24 times that of YK-009.

The LNP formulations containing Fluc-mRNA prepared from YK-206 and YK-207 also have strong fluorescence absorption, which can reach 12 times that of SM-102, 9 times that of ALC-0315, 14 times that of compound 21, 15 times that of compound 23, 10 times that of HHMA, 17 times that of Lipofectamine 3000 and 4 times that of YK-009.

The data were analyzed using GraphPad Prism software, and any one of YK-201, YK-202, YK-206, YK-207 and YK-209 showed significant differences from SM-102, ALC-0315, compound 21, compound 23, HHMA, Lipofectamine 3000 and YK-009, and the transfection efficiency was significantly improved.

### Summary:

In terms of chemical structure, the designed series of compounds, including YK-201, YK-202 and YK-209, are very different from the cationic lipids in the prior art. For example, the structure is completely different from that of FH-IMA; compared with SM-102, ALC-0315, compound 21, compound 23 and YK-009, the G₃ group is completely different, and the G₁, G₂, R₁ and R₂ groups are also very different.

The LNP formulations prepared from YK-201, YK-202 and YK-209 have the highest cell transfection efficiency and have significantly increased activity than representative cationic lipids in the prior art. For example, the cell transfection efficiency of YK-202 can reach 18 times that of SM-102, 21 times that of compound 21 and 22 times that of compound 23.

We found that not only compounds with chemical structures similar to those of cationic lipids in the prior art have cell transfection activity. On the contrary, LNP formulations prepared from compounds with very different structures may have significantly improved transfection efficiency and very strong cell transfection activity.

(3) YK-201, YK-202, and YK-209 have the highest cell transfection efficiency among the designed compounds that have the most similar structures and differ only by 1 to 2 carbon atoms in the G₁, G₂, G₃, R₁, or R₂ groups. The cell transfection efficiency of YK-202 can reach 82 times that of other compounds, such as YK-204.

In order to compare whether compounds with similar structures have similar transfection efficiencies, the compounds with the closest structures to YK-201, YK-202 and YK-209 were compared. The structural differences of these compounds were only 1 to 2 C in the G₁, G₂, G₃, R₁ or R₂ groups. The results showed that the activity of this series of compounds varied greatly, among which YK-201, YK-202 and YK-209 had the highest cell transfection efficiency, which was 78 times, 82 times and 57 times respectively that of YK-204, which had the lowest activity, and the transfection efficiency was significantly improved.

**Table 7 Fluorescence detection results of Fluc-mRNA-2**

| No. | Name | Relative light unit (RLU) | Ratio relative to SM-102 | Multiples of values that equivalent to YK-201 | Multiples of values that equivalent to YK-202 | Multiples of values that equivalent to YK-209 |
|---|---|---|---|---|---|---|
| 1 | YK-201 | 28482617 | 17.10 | 1.00 | 1.05 | 0.74 |
| 2 | YK-202 | 29957419 | 17.98 | 0.95 | 1.00 | 0.70 |
| 3 | YK-209 | 21058746 | 12.64 | 1.35 | 1.42 | 1.00 |
| 4 | YK-203 | 3869614 | 2.32 | 7.36 | 7.74 | 5.44 |
| 5 | YK-204 | 363998 | 0.22 | 78.25 | 82.30 | 57.85 |
| 6 | YK-205 | 1029168 | 0.62 | 27.68 | 29.11 | 20.46 |
| 7 | YK-208 | 3858487 | 2.32 | 7.38 | 7.76 | 5.46 |
| 8 | YK-210 | 4628355 | 2.78 | 6.15 | 6.47 | 4.55 |
| 9 | YK-211 | 1370100 | 0.82 | 20.79 | 21.87 | 15.37 |
| 10 | SM-102 | 1665874 | 1.00 | 17.10 | 17.98 | 12.64 |
| 11 | ALC-0315 | 2147045 | 1.29 | 13.27 | 13.95 | 12.51 |
| 12 | Compound 21 | 1421028 | 0.85 | 20.04 | 21.08 | 14.82 |
| 13 | Compound 23 | 1332014 | 0.80 | 21.38 | 22.49 | 15.81 |
| 14 | HHMA | 2099714 | 1.26 | 13.56 | 14.27 | 10.03 |
| 15 | Lipofectamine 3000 | 1201248 | 0.72 | 23.71 | 24.94 | 17.53 |

### a. Differences in cell transfection efficiency

As can be seen from Table 7 and Figure 6, the fluorescence absorption values of the LNP formulations prepared from these compounds are very different from those of YK-201, YK-202 and YK-209.

The fluorescence absorption value of YK-201 can reach 7.36 times that of YK-203, 78.25 times that of YK-204, 27.68 times that of YK-205, 7.38 times that of YK-208, 6.15 times that of YK-210 and 20.79 times that of YK-211.

The fluorescence absorption value of YK-202 can reach 7.74 times that of YK-203, 82.30 times that of YK-204, 29.11 times that of YK-205, 7.76 times that of YK-208, 6.47 times that of YK-210 and 21.87 times that of YK-211.

The fluorescence absorption value of YK-209 can reach 5.44 times that of YK-203, 57.85 times that of YK-204, 20.46 times that of YK-205, 5.46 times that of YK-208, 4.55 times that of YK-210 and 15.37 times that of YK-211.

The activity differences among YK-203, YK-204, YK-205, YK-208, YK-210 and YK-211 were also large. The cell transfection efficiency of LNP formulations prepared from YK-203, YK-208 and YK-210 was stronger than that of SM-102, which were 2.32 times, 2.32 times and 2.78 times that of SM-102 respectively; YK-205 and YK-211 were not much different from SM-102, the cell transfection efficiency of which were 0.62 times and 0.82 times that of SM-102 respectively; YK-204 had the lowest cell transfection efficiency, which was only 0.22 times that of SM-102.

The data were analyzed using GraphPad Prism software. Any one of YK-201, YK-202 and YK-209 was significantly different from YK-203, YK-204, YK-205, YK-208, YK-210 and YK-211, and the transfection efficiency was significantly improved.

### b. Differences in chemical structure

The structures of this series of compounds are very similar, and only differ in the G₁, G₂, G₃, R₁ or R₂ groups by 1 to 2 C. YK-201, YK-202 and YK-209 are very close to the other compounds in structure; the other compounds are also very similar to each other.

### I. Structural differences from YK-201

Compared with YK-201, YK-204 has only one more C in the G₃ group connected to N, and the other structures are exactly the same, but the cell transfection efficiency of YK-201 is 78.25 times that of YK-204.

YK-205 is only different in that the G₃ group has one more C connected to N, and each single chain in the double chain of the R₂ group has one less C. The other structures are exactly the same, but the cell transfection efficiency of YK-201 was 27.68 times that of YK-205.

YK-203 is only different in that in R₂ group the single chain has 2 more C and each single chain in the double chain has 2 less C. The other structures are exactly the same, but the cell transfection efficiency of YK-201 was 7.36 times that of YK-203.

### II. Structural differences from YK-202

Compared with YK-202, YK-204 has only one more C in the G₃ group connected to N and one less C in the single chain of R₂ group. The other structures are exactly the same, but the cell transfection efficiency of YK-202 was 82.30 times that of YK-204.

YK-205 is only different in that the G₃ group connected to N has one more C, the R₂ group has one less C in the single chain, and each single chain in the double chain has one less C. The other structures are exactly the same, but the cell transfection efficiency of YK-202 was 29.11 times that of YK-205.

The only difference of YK-203 is that the single chain of R2 group has one more C and each single chain of the double chain has 2 less C. The other structures are exactly the same, but the cell transfection efficiency of YK-202 was 7.74 times that of YK-203.

### III. Structural differences from YK-209

Compared with YK-209, YK-210 is only different in that the single chains of R₁ and R₂ groups each have one less C. The other structures are exactly the same, but the cell transfection efficiency of YK-209 was 4.55 times that of YK-210.

YK-211 is only different in that the single chains of R₁ and R₂ groups each have one more C, and each single chain of the double chain has two less C. The other structures are exactly the same, but the cell transfection efficiency of YK-209 is 15.37 times that of YK-211.

In addition, compared with YK-206, YK-205 has only one more C in the G₃ group connected to N, and the other structures are exactly the same, but the cell transfection efficiency of YK-206 is 20.29 times that of YK-205. The same is true for other compounds.

### Summary:

Among the series of compounds designed with very similar structures, where only the G₁, G₂, G₃, R₁ or R₂ groups differ by 1 to 2 carbons, YK-201, YK-202 and YK-209 have the highest cell transfection efficiency, and the cell transfection efficiency of YK-202 was 80 times higher than that of YK-204.

Moreover, it is found that there is no corresponding relationship between the structure and the intracellular transfection efficiency of the compound. Even a group of compounds with the most similar structures are likely to have very different cell transfection efficiencies.

Therefore, it is very difficult to screen out cationic lipid compounds with high transfection efficiency from a series of compounds with very similar structures, and it requires a lot of inventive work.

(4) YK-201, YK-202 and YK-209 had the highest transfection efficiency compared to compounds with only minor structural differences in the G₁, G₂, G₃, R₁ or R₂ groups. The transfection efficiency of YK-202 was more than 400 times higher than those of other compounds, such as YK-217.

The differences in cell transfection efficiency between YK-201, YK-202 and YK-209 and other compounds with similar structures were compared. This series of compounds only have some minor differences in individual groups, such as 2 less C in G₁ group, 1 less C in R₁ group, and introduction of an ether bond or an ester bond or a sulfur atom in G₃ group. The results show that this series of compounds are significantly different from YK-201, YK-202 and YK-209. The cell transfection efficiency of YK-201, YK-202 and YK-209 was significantly higher than that of other compounds, even higher than 400 times.

**Table 8 Fluorescence detection results of Fluc-mRNA-3**

| No. | Name | Relative light unit (RLU) | Ratio relative to SM-102 | Multiples of values that equivalent to YK-201 | Multiples of values that equivalent to YK-202 | Multiples of values that equivalent to YK-209 |
|---|---|---|---|---|---|---|
| | | | | | | |
| 1 | YK-201 | 28482617 | 17.10 | 1.00 | 1.05 | 0.74 |
| 2 | YK-202 | 29957419 | 17.98 | 0.95 | 1.00 | 0.70 |
| 3 | YK-209 | 21058746 | 12.64 | 1.35 | 1.42 | 1.00 |
| 4 | YK-212 | 3910590 | 2.35 | 7.28 | 7.66 | 5.39 |
| 5 | YK-213 | 1364505 | 0.82 | 20.87 | 21.95 | 15.43 |
| 6 | YK-214 | 4582178 | 2.75 | 6.22 | 6.54 | 4.60 |
| 7 | YK-215 | 981165 | 0.59 | 29.03 | 30.53 | 21.46 |
| 8 | YK-216 | 2280300 | 1.37 | 12.49 | 13.14 | 9.24 |
| 9 | YK-217 | 71850 | 0.04 | 396.42 | 416.94 | 293.09 |
| 10 | YK-218 | 607080 | 0.36 | 46.92 | 49.35 | 34.69 |
| 11 | YK-219 | 1505963 | 0.90 | 18.91 | 19.89 | 13.98 |
| 12 | YK-220 | 216398 | 0.13 | 131.62 | 138.44 | 97.32 |
| 13 | SM-102 | 1665874 | 1.00 | 17.10 | 17.98 | 12.64 |
| 14 | ALC-0315 | 2147045 | 1.29 | 13.27 | 13.95 | 12.51 |
| 15 | Compound 21 | 1421028 | 0.85 | 20.04 | 21.08 | 14.82 |
| 16 | Compound 23 | 1332014 | 0.80 | 21.38 | 22.49 | 15.81 |
| 17 | HHMA | 2099714 | 1.26 | 13.56 | 14.27 | 10.03 |
| 18 | Lipofectamine 3000 | 1201248 | 0.72 | 23.71 | 24.94 | 17.53 |

### a. Differences in cell transfection efficiency

Although the other compounds have some minor differences in G₃, G₁, R₁, G₂ or R₂ groups compared with YK-201, YK-202 and YK-209, their effects on cell transfection efficiency are very large, with a difference of more than 400 times.

Specifically, it can be seen from Table 8 that the fluorescence absorption values of the LNP formulations prepared from YK-217 and YK-220 are very different from that of YK-201, YK-202 and YK-209.

The fluorescence absorption value of YK-201 can reach 396.42 times that of YK-217 and 131.62 times that of YK-220.

The fluorescence absorption value of YK-202 can reach 416.94 times that of YK-217 and 138.44 times that of YK-220.

The fluorescence absorption value of YK-209 can reach 293.09 times that of YK-217 and 97.32 times that of YK-220.

The activities of YK-213, YK-215, YK-218 and YK-219 are very different from those of YK-201, YK-202 and YK-209.

The fluorescence absorption value of YK-201 can reach 20.87 times that of YK-213, 29.03 times that of YK-215, 46.92 times that of YK-218 and 18.91 times that of YK-219.

The fluorescence absorption value of YK-202 can reach 21.95 times that of YK-213, 30.53 times that of YK-215, 49.35 times that of YK-218 and 19.89 times that of YK-219.

The fluorescence absorption value of YK-209 can reach 15.43 times that of YK-213, 21.46 times that of YK-215, 34.69 times that of YK-218 and 13.98 times that of YK-219.

The activities of YK-212, YK-214 and YK-216 are also very different from those of YK-201, YK-202 and YK-209.

The fluorescence absorption value of YK-201 can reach 7.28 times that of YK-212, 6.22 times that of YK-214 and 12.49 times that of YK-216.

The fluorescence absorption value of YK-202 can reach 7.66 times that of YK-212, 6.54 times that of YK-214 and 13.14 times that of YK-216.

The fluorescence absorption value of YK-209 can reach 5.39 times that of YK-212, 4.60 times that of YK-214 and 9.24 times that of YK-216.

The data were analyzed using GraphPad Prism software. Any one of YK-201, YK-202 and YK-209 is significantly different from the other compounds, and the cell transfection efficiency was significantly improved.

### b. Differences in chemical structure

Compared with YK-201, YK-202 and YK-209, this series of compounds only slightly differ in the G₁, G₂, G₃, R₁ or R₂ groups. YK-201, YK-202 and YK-209 are very close to the other compounds in structure; the other compounds are also very similar to each other.

### I. Structural differences from YK-201

Compared with YK-201, YK-213 has only one more HOCH₂-group connected to N in the G₃ group, a branched structure in the R₁ group, and two less C in the G₂ group. The other structures are exactly the same, but the cell transfection efficiency of YK-201 is 20.87 times that of YK-213.

YK-215 only has an ether bond introduced into the G₃ group and an additional methyl group connected to O. The rest of the structure is exactly the same, but the cell transfection efficiency of YK-201 is 29.03 times that of YK-215.

YK-217 is only different in that the G₃ group is (CH₃)₂N(CH₂)₃SC(O)O(CH₂)₂-, the G₁ group has 2 less C, the R₁ group has 1 less C, the G₂ group has 2 less C, the R₂ group single chain has 2 more C, and each single chain in the double chain has 2 less C. The other structures are exactly the same, but the cell transfection efficiency of YK-201 is 396.42 times that of YK-217.

YK-220 is only different in that the G₃ group is (CH₃)₂N(CH₂)₃SC(O)-, the G₁ group has 2 less C, the R₁ group has 1 less C, the G₂ group has 2 less C, the R₂ group single chain has 2 more C, and each single chain in the double chain has 2 less C. The other structures are exactly the same, but the cell transfection efficiency of YK-201 is 131.62 times that of YK-220.

### II. Structural differences from YK-202

Compared with YK-202, YK-217 is only different in that the G₃ group is (CH₃)₂N(CH₂)₃SC(O)O(CH₂)₂-, the G₁ group has 2 less C, the R₁ group has 1 less C, the G₂ group has 2 less C, the R₂ group single chain has 1 more C, and each single chain in the double chain has 2 less C. The other structures are exactly the same, but the cell transfection efficiency of YK-202 is 416.94 times that of YK-217.

YK-219 is only different in that the G₃ group is (CH₃)₂N(CH₂)₃SC(O)-, the G₁ group has 2 less C, the R₁ group has 1 less C, and the G₂ group has 2 less C. The other structures are exactly the same, but the cell transfection efficiency of YK-202 is 19.89 times that of YK-219.

YK-220 is only different in that the G₃ group is (CH₃)₂N(CH₂)₃SC(O)-, the G₁ group has 2 less C, the R₁ group has 1 less C, the G₂ group has 2 less C, the R₂ group single chain has 1 more C, and each single chain in the double chain has 2 less C. The other structures are exactly the same, but the cell transfection efficiency of YK-202 is 138.44 times that of YK-220.

### III. Structural differences from YK-209

Compared with YK-209, YK-213 has only one more HOCH₂- group connected to N in the G₃ group, and one less C in the single chain of each of the R₁ and R₂ groups. The other structures are exactly the same, but the cell transfection efficiency of YK-209 is 15.43 times that of YK-213 .

YK-217 is only different in that the G₃ group is (CH₃)₂N(CH₂)₃SC(O)O(CH₂)₂-, the G₁ group has 2 less C, the R₁ group has 1 less C, the R₂ group single chain has 1 more C, and each single chain in the double chain has 2 less C. The other structures are exactly the same, but the cell transfection efficiency of YK-209 is 293.09 times that of YK-217.

YK-220 is only different in that the G₃ group is (CH₃)₂N(CH₂)₃SC(O)-, the G₁ group has 2 less C, the R₁ group is a single-chain structure, the R₂ group has 1 more C in the single chain, and each single chain in the double chain has 2 less C. The other structures are exactly the same, but the cell transfection efficiency of YK-209 is 97.32 times that of YK-220.

In addition, compared with YK-207, YK-220 in only different in that the G₃ group is (CH₃)₂N(CH₂)₃SC(O)-, the R₂ group has one more C in the single chain, and two less C in each single chain of the double chain. The other structures are exactly the same, but the cell transfection efficiency of YK-207 is 124.13 times that of YK-220. The other compounds are similar.

### Summary:

Compared with compounds with only minor differences in individual groups, such as G₁ with 2 less carbon atoms, R₁ with 1 less carbon atoms, G₃ with introduction of ether bonds, ester bonds or sulfur atoms, YK-201, YK-202 and YK-209 have the highest cell transfection efficiency. For example, the cell transfection efficiency of YK-201 and YK-202 are 400 times higher than that of YK-217 and 130 times higher than that of YK-220.

Moreover, it is found that there is no corresponding relationship between the structure and the intracellular transfection efficiency of the compound. Even a group of compounds with very small structural differences are likely to have very large differences in cell transfection efficiency.

Therefore, it is very difficult to screen out cationic lipid compounds with high transfection efficiency from a series of compounds with only slight differences in chemical structure, which requires a lot of inventive work.

(5) YK-201, YK-202, and YK-209 have the highest transfection efficiency compared to compounds in which only hydroxyl of the G₃ group is changed to methylamino, or hydroxyl and methyl attached to N are removed. For example, the transfection efficiency of YK-201 and YK-202 is more than 1000 times higher than that of YK-221 and YK-225.

The compounds listed in Table 9 only differ from YK-201, YK-202 and YK-209 only in the G₃ groups, wherein the hydroxyl group is changed to a methylamino group, or the hydroxyl group and the methyl group connected to N are removed. The cell transfection efficiency results show that among this series of compounds with similar structures, the cell transfection efficiency of YK-201, YK-202 and YK-209 is much higher than that of other compounds, and the cell transfection efficiency of YK-201 and YK-202 is more than 1000 times higher than that of YK-221 and YK-225.

**Table 9 Fluorescence detection results of Fluc-mRNA-4**

| No. | Name | Relative light unit (RLU) | Ratio relative to SM-102 | Multiples of values that equivalent to YK-201 | Multiples of values that equivalent to YK-202 | Multiples of values that equivalent to YK-209 |
|---|---|---|---|---|---|---|
| 1 | YK-201 | 28482617 | 17.10 | 1.00 | 1.05 | 0.74 |
| 2 | YK-202 | 29957419 | 17.98 | 0.95 | 1.00 | 0.70 |
| 3 | YK-209 | 21058746 | 12.64 | 1.35 | 1.42 | 1.00 |
| 4 | YK-221 | 27960 | 0.02 | 1018.69 | 1071.44 | 753.17 |
| 5 | YK-222 | 606360 | 0.36 | 46.97 | 49.41 | 34.73 |
| 6 | YK-223 | 75012 | 0.05 | 379.71 | 399.37 | 280.74 |
| 7 | YK-224 | 88020 | 0.05 | 323.59 | 340.35 | 239.25 |
| 8 | YK-225 | 26145 | 0.02 | 1089.41 | 1145.82 | 805.46 |
| 9 | YK-226 | 57660 | 0.03 | 493.98 | 519.55 | 365.22 |
| 10 | SM-102 | 1665874 | 1.00 | 17.10 | 17.98 | 12.64 |
| 11 | ALC-0315 | 2147045 | 1.29 | 13.27 | 13.95 | 12.51 |
| 12 | Compound 21 | 1421028 | 0.85 | 20.04 | 21.08 | 14.82 |
| 13 | Compound 23 | 1332014 | 0.80 | 21.38 | 22.49 | 15.81 |
| 14 | HHMA | 2099714 | 1.26 | 13.56 | 14.27 | 10.03 |
| 15 | Lipofectamine 3000 | 1201248 | 0.72 | 23.71 | 24.94 | 17.53 |

### a. Differences in cell transfection efficiency

By changing the hydroxyl group in the G₃ groups of YK-201, YK-202 and YK-209 to a methylamino group, or removing the hydroxyl group and the methyl group connected to N, the cell transfection efficiency was greatly reduced. The cell transfection efficiency of YK-201 and YK-202 is more than 1000 times higher than that of YK-221 and YK-225.

The details are as follows:
As can be seen from Table 9, the cell transfection efficiency of the two compounds, YK-221 and YK-225, is significantly different from that of YK-201, YK-202 and YK-209.

The cell transfection efficiency of YK-201 is 1018.69 times that of YK-221 and 1089.41 times that of YK-225.

The cell transfection efficiency of YK-202 is 1071.44 times that of YK-221 and 1145.82 times that of YK-225.

The cell transfection efficiency of YK-209 is 753.17 times that of YK-221 and 805.46 times that of YK-225.

The cell transfection efficiency of YK-223, YK-224 and YK-226 is also much lower than that of YK-201, YK-202 and YK-209.

The transfection efficiency of YK-201 cells is 379.71 times that of YK-223, 323.59 times that of YK-224, and 493.98 times that of YK-226.

The transfection efficiency of YK-202 cells is 399.37 times that of YK-223, 340.35 times that of YK-224, and 519.55 times that of YK-226.

The transfection efficiency of YK-209 cells is 280.74 times that of YK-223, 239.25 times that of YK-224, and 365.22 times that of YK-226.

YK-222 is also quite different from YK-201, YK-202 and YK-209. The fluorescence absorption values of YK-201, YK-202 and YK-209 are 46.97 times, 49.41 times and 34.73 times that of YK-222, respectively.

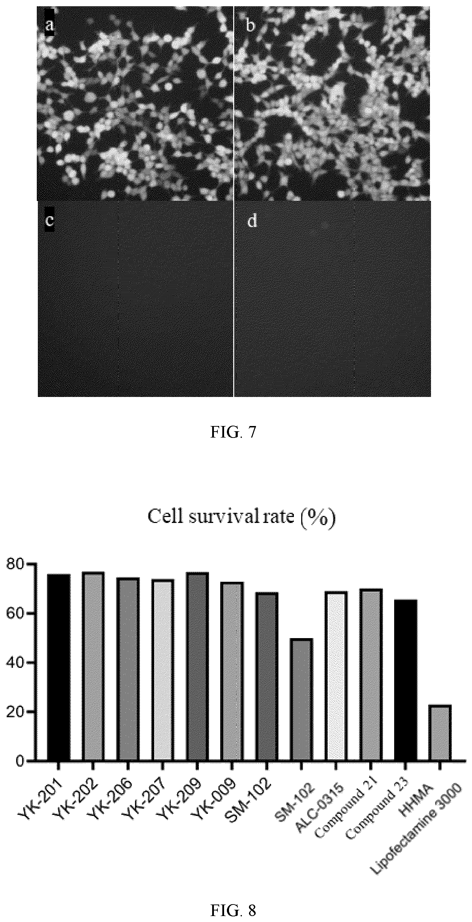

FIG.7 shows the fluorescence absorption images of LNP formulations prepared from YK-201, YK-202, YK-221 and YK-225. It can be seen that the fluorescence absorption of YK-221 and YK-225 is very weak compared with YK-201 and YK-202.

The data were analyzed using GraphPad Prism software. Any one of YK-201, YK-202 and YK-209 was significantly different from the other compounds, and the transfection efficiency was significantly improved.

### b. Differences in chemical structure

This series of compounds has very little structural difference from YK-201, YK-202 and YK-209, and only differs in that the hydroxyl group in the G₃ group is changed to a methylamino group, or the hydroxyl group and the methyl group connected to N are removed, and other parts are slightly changed; the structural differences between these compounds are also very small.

### I. Structural differences from YK-201

Compared with YK-201, YK-221 is only different in that the hydroxyl group in the G₃ group is changed to a methylamino group, the G₁ group has 2 less C, the R₁ group has 1 less C, the G₂ group has 2 less C, and the R₂ group has 1 more C in the single chain. The other structures are exactly the same, but the cell transfection efficiency of YK-201 is 1018.69 times that of YK-221.

YK-225 is only different in that the hydroxyl group in the G₃ group is changed to a methylamino group, the R₁ group is a branched structure, the G₂ group has 2 less C, the R₂ group single chain has 2 more C, and each single chain in the double chain has 2 less C. The other structures are exactly the same, but the cell transfection efficiency of YK-201 is 1089.41 times that of YK-225.

YK-226 is only different in that the hydroxyl group and the methyl group connected to N are removed from the G₃ group, the G₂ group has 3 less C, and the R₂ group has 1 more C in the single chain. The other structures are exactly the same, but the cell transfection efficiency of YK-201 is 493.98 times that of YK-226.

### II. Structural differences from YK-202

Compared with YK-201, YK-221 is only different in that the hydroxyl group in the G₃ group is changed to a methylamino group, the G₁ group has 2 less C, the R₁ group has 1 less C, and the G₂ group has 2 less C. The other structures are exactly the same, but the cell transfection efficiency of YK-202 is 1071.44 times that of YK-221.

YK-225 is only different in that the hydroxyl group in the G₃ group is changed to a methylamino group, the R₁ group is a branched structure, the G₂ group has 2 less C, the R₂ group single chain has 1 more C, and each single chain in the double chain has 2 less C. The other structures are exactly the same, but the cell transfection efficiency of YK-202 is 1145.82 times that of YK-225.

YK-226 is only different in that the hydroxyl group and the methyl group connected to N are removed from the G₃ group, and the G₂ group has 3 less C. The other structures are exactly the same, but the cell transfection efficiency of YK-202 is 519.55 times that of YK-226.

### III. Structural differences from YK-209

Compared with YK-209, YK-221 is only different in that the hydroxyl group in the G₃ group is changed to a methylamino group, the G₁ group has 2 less carbon atoms, and the R₁ group is a branched structure. The other structures are exactly the same, but the cell transfection efficiency of YK-209 is 753.17 times that of YK-221.

YK-223 is only different in that the hydroxyl group in the G₃ group is changed to a methylamino group, and the other structures are exactly the same, but the cell transfection efficiency of YK-209 is 280.74 times that of YK-223.

YK-225 is only different in that the hydroxyl group in the G₃ group is changed to a methylamino group, the single chains of the R₁ and R₂ groups each have one more C, and each single chain in the double chain has two less C. The other structures are exactly the same, but the cell transfection efficiency of YK-209 is 805.46 times that of YK-225.

In addition, compared with YK-207, YK-221 is only different in that the hydroxyl group in the G₃ group is changed to a methylamino group, and the other structures are exactly the same, but the cell transfection efficiency of YK-207 is 960.71 times that of YK-221. The other compounds are similar.

### Summary:

From the above, it can be seen that compared with the series of compounds in which the hydroxyl group in the G₃ group is changed to a methylamino group, or the hydroxyl group and the methyl group connected to N are removed, YK-201, YK-202 and YK-209 have the highest cell transfection efficiency. For example, the cell transfection efficiency of YK-201 and YK-202 can reach more than 1000 times that of YK-221 and YK-225.

Moreover, it is found that for compounds with similar structures, it is impossible to infer the difference in cell transfection efficiency based on the structural differences. Even a group of compounds with very small structural differences are very likely to have very large differences in cell transfection efficiency.

Therefore, it is very difficult to screen out cationic lipid compounds with high transfection efficiency from a series of compounds with similar chemical structures, and it requires a lot of inventive work.

### Conclusion:

1) Through multiple designs of compound structures and a lot of inventive work, we designed and screened cationic lipid compounds with high cell transfection efficiency, such as YK-201, YK-202 and YK-209.

This series of designed compounds have significant differences in chemical structure from representative cationic lipids in the prior art, such as SM-102, compound 21, compound 23, HHMA and YK-009. The G₃ group is completely different, and other parts are also different. Therefore, there are also great differences in polarity, acidity and alkalinity, and hydrophilicity.

2) The LNP formulations prepared from YK-201, YK-202 and YK-209 have the highest cell transfection efficiency and are significantly more active than representative cationic lipids in the prior art. For example, the cell transfection efficiency of YK-202 is 18 times that of SM-102, 21 times that of compound 21, and 22 times that of compound 23.

YK-201, YK-202 and YK-209 have the highest cell transfection efficiency among the compounds designed, which are most similar in structure and differ only by 1 to 2 C in G₁, G₂, G₃, R₁ or R₂ groups. The cell transfection efficiency of YK-202 can reach 80 times that of other compounds, such as YK-204.

YK-201, YK-202 and YK-209 show the highest transfection efficiency compared with compounds that have only some minor differences in individual groups, such as 2 less carbons in G₁, 1 less carbon in R₁, and ether bonds, ester bonds or sulfur atoms introduced in G₃. The transfection efficiency of YK-202 is more than 400 times higher than that of other compounds, such as YK-217.

YK-201, YK-202 and YK-209 have the highest cell transfection efficiency compared to compounds in which only the hydroxyl group in the G₃ group is changed to a methylamino group, or the hydroxyl group and the methyl group connected to N are removed. For example, compared with YK-221 and YK-225, the cell transfection efficiency of YK-201 and YK-202 can be increased by more than 1000 times.

3) There is no corresponding relationship between the structure of the compound and the intracellular transfection efficiency. Compounds with small structural differences are likely to have very large differences in transfection efficiency. Therefore, screening cationic lipid compounds with high transfection efficiency requires multiple designs and a lot of inventive work.

### 2. Cell survival rate Assay

The LNP formulations containing 1.5 µg Fluc-mRNA (prepared according to Example 4, the LNP formulation carrier components are cationic lipids, neutral lipids, structured lipids and polymer-conjugated lipids at a molar ratio of 49:10:39.5:1.5, wherein the cationic lipids are the cationic lipids listed in Table 1) and the Lipofectamine 3000 formulation were added to the cell culture medium of the 96-well plate, and the culture was continued for 24 hours. Then, 10 µL of CCK-8 solution was added to each well, and the culture plate was incubated in the incubator for 1 hour, and the absorbance at 450 nm was measured by a microplate reader. The results are shown in Tables 10 to 13.

### Experimental results:

(1) Among the designed series of compounds, the LNP formulations prepared from YK-201, YK-202 and YK-209 have significantly lower cytotoxicity than the representative cationic lipids in the prior art. For example, the cell survival rate of YK-202 is 26.85% higher than that of ALC-0315, 8.26% higher than that of SM-102, and 11.25% higher than that of HHMA.

**Table 10 Cell survival rate-1**

| Number | Cationic lipids | Cell survival rate (%) |
|---|---|---|
| 1 | YK-201 | 75.93 |
| 2 | YK-202 | 76.81 |
| 3 | YK-206 | 74.57 |
| 4 | YK-207 | 73.88 |
| 5 | YK-209 | 76.69 |
| 6 | YK-009 | 72.88 |
| 7 | SM-102 | 68.55 |
| 8 | ALC-0315 | 49.96 |
| 9 | Compound 21 | 69.02 |
| 10 | Compound 23 | 70.10 |
| 11 | HHMA | 65.56 |
| 12 | Lipofectamine 3000 | 23.05 |

### a. Difference in cell survival rate

Table 10 lists the results of cytotoxicity detection of LNP formulations prepared from different cationic lipid compounds. Among them, YK-009 is disclosed in CN114044741B (claim 1), SM-102 is compound 25 disclosed in WO2017049245A2 (page 29 of the specification), ALC-0315 is compound 3 disclosed in CN108368028B (page 24 of the specification), compounds 21 and 23 are disclosed in WO2021055833A1 (page 22 of the specification), and HHMA is compound 1 disclosed in CN112979483B (page 12 of the specification); Lipofectamine 3000 is a widely used cell transfection reagent with good transfection performance.

As shown in Table 10, the LNP formulations of Fluc-mRNA prepared by YK-201, YK-202 and YK-209 have the lowest cytotoxicity, and the cell survival rate can reach 75.93%, 76.81% and 76.69%, respectively.

The cell survival rate of YK-201 is 7.38% higher than that of SM-102, 25.97% higher than that of ALC-0315, 6.91% higher than that of compound 21, 5.83% higher than that of compound 23, 10.37% higher than that of HHMA, and 52.88% higher than that of Lipofectamine 3000.

The cell survival rate of YK-202 is 8.26% higher than that of SM-102, 26.85% higher than that of ALC-0315, 7.79% higher than that of compound 21, 6.71% higher than that of compound 23, 11.25% higher than that of HHMA, and 53.76% higher than that of Lipofectamine 3000.

The cell survival rate of YK-209 is 8.14% higher than that of SM-102, 26.73% higher than that of ALC-0315, 7.67% higher than that of compound 21, 6.59% higher than that of compound 23, 11.13% higher than that of HHMA, and 53.64% higher than that of Lipofectamine 3000.

The cell survival rate of YK-206 and YK-207 are 74.57% and 73.88%, respectively, which are also higher than that of the existing cationic lipids, 6.02% and 5.33% higher than that of SM-102, 24.61% and 23.92% higher than that of ALC-0315, 5.55% and 4.86% higher than that of compound 21, 4.47% and 3.78% higher than that of compound 23, 9.01% and 8.32% higher than that of HHMA, and 51.52% and 50.83% higher than that of Lipofectamine 3000. (Figure 8)

The data were analyzed using GraphPad Prism software, and any one of YK-201, YK-202 and YK-209 had significant differences from SM-102, ALC-0315, compound 21, compound 23 and Lipofectamine 3000, and the cytotoxicity was significantly reduced.

### b. Differences in chemical structure

The chemical structures of YK-201, YK-202 and YK-209 are very different from those of cationic lipids in the prior art, and most different from the HHMA structure. From the chemical structure diagram, it can be seen that only one side chain of the group connected to the central N atom of HHMA is similar to one side chain of this series of structures, and the other parts are completely different. Compared with SM-102, ALC-0315, compound 21, compound 23 and YK-009, the G₃ group is completely different; the G₁, R₁, G₂ and R₂ groups are also significantly different.

### Summary:

Among the designed series of compounds, the LNP formulations prepared by YK-201, YK-202 and YK-209 have the lowest cytotoxicity and significantly improve the cell survival rate compared with the representative cationic lipids in the prior art. For example, the cell survival rate of YK-202 is 26.85% higher than that of ALC-0315, 8.26% higher than that of SM-102, and 11.25% higher than that of HHMA. The cytotoxicity of YK-206 and YK-207 is also lower than that of the representative cationic lipids in the prior art.

Compared with the representative cationic lipids in the prior art, the chemical structures of YK-201, YK-202 and YK-209 are significantly different, wherein the G₃ groups are completely different, and the G₁, R₁, G₂ and R₂ groups are also significantly different.

Therefore, it is not only LNP formulations prepared from compounds with similar structures to the cationic lipids in the prior art that have lower cytotoxicity. On the contrary, LNP formulations prepared from compounds with significantly different structures are likely to have significantly reduced cytotoxicity.

(2) YK-201, YK-202 and YK-209 show the lowest cytotoxicity among the compounds designed that are most similar in structure and differ only by 1-2 carbons in the G₁, G₂, G₃, R₁ or R₂ groups. The cell survival rate of the three compounds is increased by 40% compared with other compounds, such as YK-203.

In order to compare the differences in cytotoxicity of compounds with similar structures, YK-201, YK-202 and YK-209 were compared with the compounds closest in structure which only differ by 1 to2 C in the G₁, G₂, G₃, R₁ or R₂ groups. The results show that the differences in cytotoxicity of this series of compounds are very significant. Among them, YK-201, YK-202 and YK-209 have the highest cell survival rate, reaching 75.93%, 76.81% and 76.69% respectively. The cell survival rate of the three could be 40% higher than that of other compounds, such as YK-203.

**Table 11 Cell vability-2**

| Number | Cationic lipids | Cell survival rate (%) |
|---|---|---|
| 1 | YK-201 | 75.93 |
| 2 | YK-202 | 76.81 |
| 3 | YK-209 | 76.69 |
| 4 | YK-203 | 35.56 |
| 5 | YK-204 | 42.57 |
| 6 | YK-205 | 58.92 |
| 7 | YK-208 | 46.68 |
| 8 | YK-210 | 38.38 |
| 9 | YK-211 | 52.30 |
| 10 | SM-102 | 68.55 |
| 11 | ALC-0315 | 49.96 |
| 12 | Compound 21 | 69.02 |
| 13 | Compound 23 | 70.10 |
| 14 | HHMA | 65.56 |
| 15 | Lipofectamine 3000 | 23.05 |

### a. Difference in cell survival rate

As shown in Table 11, the cytotoxicity of the LNP formulations prepared by these compounds varies greatly, among which YK-201, YK-202 and YK-209 have the lowest toxicity and the highest cell survival rate. YK-203 and YK-210 have the lowest cell survival rate, which are only 35.56% and 38.38%, respectively.

The cell survival rate of YK-201 is 40.37% higher than that of YK-203 and 37.55% higher than that of YK-210.

The cell survival rate of YK-202 is 41.25% higher than that of YK-203 and 38.43% higher than that of YK-210.

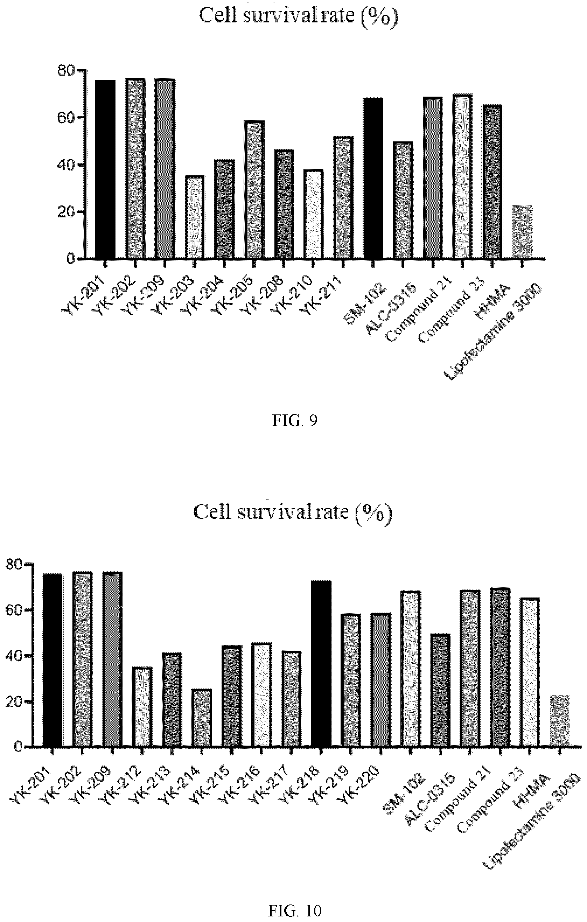

The cell survival rate of YK-209 is 41.13% higher than that of YK-203 and 38.31% higher than that of YK-210. (Figure 9)

The cell survival rate of YK-204, YK-205, YK-208 and YK-211 are 42.57%, 58.92%, 46.68% and 52.30%, respectively.

The cell survival rate of YK-201 is 33.36% higher than that of YK-204, 17.01% higher than that of YK-205, 29.25% higher than that of YK-208, and 23.63% higher than that of YK-211.

The cell survival rate of YK-202 is 34.24% higher than that of YK-204, 17.89% higher than that of YK-205, 30.13% higher than that of YK-208, and 24.51% higher than that of YK-211.

The cell survival rate of YK-209 is 34.12% higher than that of YK-204, 17.77% higher than that of YK-205, 30.01% higher than that of YK-208, and 24.39% higher than that of YK-211.

The data were analyzed using GraphPad Prism software, and any one of YK-201, YK-202 and YK-209 had significant differences in cytotoxicity compared with the other compounds, and the cytotoxicity was significantly reduced.

### b. Differences in chemical structure

The structures of this series of compounds are very similar, with only slight differences in individual groups. YK-201, YK-202 and YK-209 are very close to the structures of other compounds; the other compounds are also very similar to each other.

### Summary:

Among a group of compounds with very similar structures, where only the G₁, G₂, G₃, R₁ or R₂ groups differ by 1 to 2 carbons, YK-201, YK-202 and YK-209 have the lowest cytotoxicity, and their cell survival rate is increased by 40.37%, 41.25% and 41.13% respectively compared with the lowest YK-003.

Moreover, it is found that there is no corresponding relationship between the structure of the compound and its cytotoxicity. Even for a group of compounds with the most similar structures, the cytotoxicity is likely to be very different.

Therefore, it is very difficult to screen out cationic lipid compounds with low cytotoxicity from a series of compounds with only slight differences in chemical structure, which requires a lot of inventive work.

(3) YK-201, YK-202 and YK-209 show the lowest cytotoxicity compared with compounds with only minor structural differences in the G₁, G₂, G₃, R₁ or R₂ groups. The cell survival rate of the three compounds is increased by 50% compared with other compounds, such as YK-214.

The differences in cytotoxicity were further compared between YK-201, YK-202 and YK-209 and other compounds with similar structures. This series of compounds has only some minor differences in individual groups, such as 2 less C in G₁, 1 less C in R₁, and introduction of ether bonds, ester bonds or sulfur atoms in G₃. The results show that the cytotoxicity of this series of compounds is very different. YK-201, YK-202 and YK-209 have the highest cell survival rate, which is 50% higher than that of other compounds, such as YK-214.

**Table 12 Cell survival rate-3**

| Number | Cationic lipids | Cell survival rate (%) |
|---|---|---|
| 1 | YK-201 | 75.93 |
| 2 | YK-202 | 76.81 |
| 3 | YK-209 | 76.69 |
| 4 | YK-212 | 35.26 |
| 5 | YK-213 | 41.44 |
| 6 | YK-214 | 25.58 |
| 7 | YK-215 | 44.59 |
| 8 | YK-216 | 45.80 |
| 9 | YK-217 | 42.27 |
| 10 | YK-218 | 72.84 |
| 11 | YK-219 | 58.61 |
| 12 | YK-220 | 58.99 |
| 13 | SM-102 | 68.55 |
| 14 | ALC-0315 | 49.96 |
| 15 | Compound 21 | 69.02 |
| 16 | Compound 23 | 70.10 |
| 17 | HHMA | 65.56 |
| 18 | Lipofectamine 3000 | 23.05 |

### a. Difference in cell survival rate

Although the other compounds have only some minor differences in G₁, G₂, G₃, R₁ or R₂ groups compared to YK-201, YK-202 and YK-209, their effect on cytotoxicity is very large. The cell survival rate of YK-201, YK-202 and YK-209 can be up to 50% higher than that of other compounds.

As can be seen from Table 12, the cell survival rate of YK-214 is the lowest, only 25.58%. The cell survival rate of YK-201 is 50.35% higher than that of YK-214, the cell survival rate of YK-202 is 51.23% higher than that of YK-214, and the cell survival rate of YK-209 is 51.11% higher than that of YK-214.

The cell survival rates of YK-212, YK-213, YK-215, YK-216 and YK-217 are 35.26%, 41.44%, 44.59%, 45.80% and 42.27%, respectively.

The cell survival rate of YK-201 is 40.67% higher than that of YK-212, 34.49% higher than that of YK-213, 31.34% higher than that of YK-215, 30.13% higher than that of YK-216, and 33.66% higher than that of YK-217.

The cell survival rate of YK-202 is 41.55% higher than that of YK-212, 35.37% higher than that of YK-213, 32.22% higher than that of YK-215, 31.01% higher than that of YK-216, and 34.54% higher than that of YK-217.

The cell survival rate of YK-209 is 41.43% higher than that of YK-212, 35.25% higher than that of YK-213, 32.10% higher than that of YK-215, 30.89% higher than that of YK-216, and 34.42% higher than that of YK-217.

The cell survival rate of YK-219 and YK-220 are high, at 58.61% and 58.99% respectively.

The cell survival rate of YK-201 is 17.32% higher than that of YK-219, and16.94% higher than that of YK-220.

The cell survival rate of YK-202 is 18.20% higher than that of YK-219, and 17.82% higher than that of YK-220.

The cell survival rate of YK-209 is 18.08% higher than that of YK-219, and 17.70% higher than that of YK-220.

The cell survival rate of YK-218 is 72.84%, slightly lower than that of YK-201, YK-202 and YK-209. (Figure 10)

The data were analyzed using GraphPad Prism software, and any one of YK-201, YK-202 and YK-209 had significant differences in cytotoxicity compared with YK-212, YK-213, YK-214, YK-215, YK-216, YK-217, YK-219 and YK-220, and the cytotoxicity was significantly reduced.

### b. Differences in chemical structure

This series of compounds has only some minor differences in individual groups, such as 2 less C in G₁, 1 less C in R₁, and introduction of an ether bond, ester bond or sulfur atom in G₃.

### Summary:

Compared with only minor differences in individual groups, such as 2 less carbon atoms in G₁, 1 less carbon atoms in R₁, and ether bonds, ester bonds, or sulfur atoms introduced in G₃, YK-201, YK-202, and YK-209 have the lowest cytotoxicity. For example, the cell survival rates of YK-201, YK-202, and YK-209 are all 50% higher than that of YK-014.

Moreover, it is found that there is no corresponding relationship between the structure of the compound and its cytotoxicity. Even if the structure is slightly different, the cytotoxicity is likely to be very different.

Therefore, it is very difficult to screen out cationic lipid compounds with low cytotoxicity from a series of compounds with only some minor differences in individual groups, which requires a lot of inventive work.

(4) YK-201, YK-202 and YK-209 show significantly lower cytotoxicity compared with compounds in which only the hydroxyl group in the G₃ group is changed to a methylamino group, or the hydroxyl group and the methyl group connected to N are removed. For example, compared with YK-221, the cell survival rate of all three compounds is increased by 55%.

Cytotoxicity results show that the cell survival rate is greatly reduced when the hydroxyl group in the G₃ group in YK-201, YK-202 and YK-209 is changed to a methylamino group, or the hydroxyl group and the methyl group connected to N are removed. For example, the cell survival rate of YK-201, YK-202 and YK-209 is increased by 55% compared with YK-221.

**Table 13 Cell survival rate-4**

| Number | Cationic lipids | Cellsurvival rate (%) |
|---|---|---|
| 1 | YK-201 | 75.93 |
| 2 | YK-202 | 76.81 |
| 3 | YK-209 | 76.69 |
| 4 | YK-221 | 20.95 |
| 5 | YK-222 | 33.75 |
| 6 | YK-223 | 36.64 |
| 7 | YK-224 | 23.47 |
| 8 | YK-225 | 43.41 |
| 9 | YK-226 | 27.90 |
| 10 | SM-102 | 68.55 |
| 11 | ALC-0315 | 49.96 |
| 12 | Compound21 | 69.02 |
| 13 | Compound23 | 70.10 |
| 14 | HHMA | 65.56 |
| 15 | Lipofectamine 3000 | 23.05 |

### a. Difference in cell survival rate

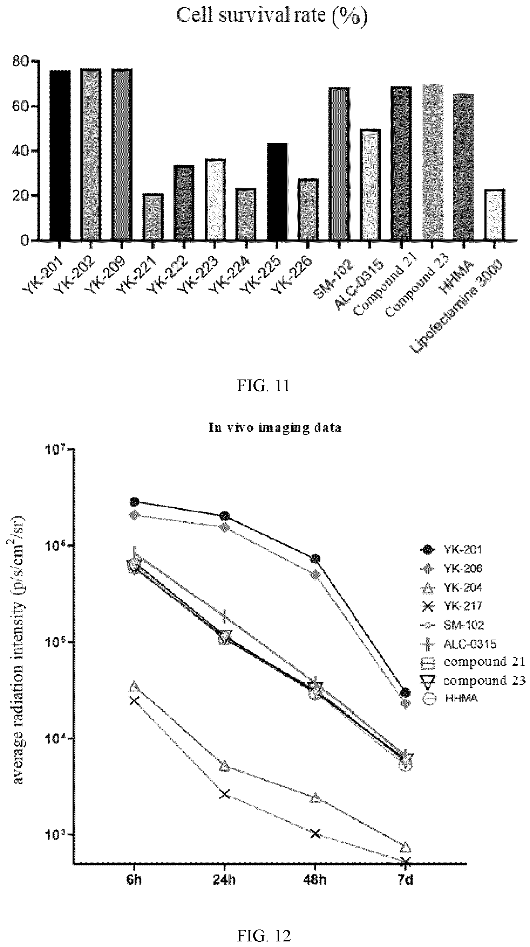

As can be seen from Table 13, by changing the hydroxyl group in the G₃ group of YK-201, YK-202 and YK-209 to a methylamino group, or removing the hydroxyl group and the methyl group connected to N, the cytotoxicity is greatly increased. For example, the cell survival rate of YK-201 and YK-202 is 55% higher than that of YK-221. (Figure 11)

Specifically, the cell survival rates of YK-221, YK-224 and YK-226 are the lowest, all between 20% and 30%, namely 20.95%, 23.47% and 27.90% respectively.

The cell survival rate of YK-201 is 54.98% higher than that of YK-221, 52.46% higher than that of YK-224, and 48.03% higher than that of YK-226.

The cell survival rate of YK-202 is 55.86% higher than that of YK-221, 53.34% higher than that of YK-224, and 48.91% higher than that of YK-226.

The cell survival rate of YK-209 is 55.74% higher than that of YK-221, 53.22% higher than that of YK-224, and 48.79% higher than that of YK-226.

The cell survival rates of YK-222, YK-223, and YK-225 are 33.75%, 36.64%, and 43.41%, respectively.

The cell survival rate of YK-201 is 42.18% higher than that of YK-222, 39.29% higher than that of YK-223, and 32.52% higher than that of YK-225.

The cell survival rate of YK-202 is 43.06% higher than that of YK-222, 40.17% higher than that of YK-223, and 33.40% higher than that of YK-225.

The cell survival rate of YK-209 is 42.94% higher than that of YK-222, 40.05% higher than that of YK-223, and 33.28% higher than that of YK-225.

The data were analyzed using GraphPad Prism software, and any one of YK-201, YK-202 and YK-209 have significant differences from YK-221, YK-222 and YK-223, YK-224, YK-225 and YK-226, and the cytotoxicity is significantly reduced.

### b. Differences in chemical structure

The structural differences of this series of compounds are very small. Compared with YK-201, YK-202 and YK-209, in the other compounds, the hydroxyl group in the G₃ group is changed to a methylamino group, or the hydroxyl group and the methyl group connected to N are removed, and the other structures are exactly the same.

### Summary:

Compared with compounds in which the hydroxyl group in the G₃ group is changed to a methylamino group, or the hydroxyl group and the methyl group connected to N are removed, YK-201, YK-202 and YK-209 have the lowest cytotoxicity. For example, the cell survival rates of the three compounds are increased by more than 50% compared with those of YK-221 and YK-224.

Moreover, it is found that there is no corresponding relationship between the structure of the compound and its cytotoxicity. Even if there are only some differences in the structure of the G₃ group, the cytotoxicity is likely to be very different.

Therefore, it is very difficult to screen cationic lipid compounds with low cytotoxicity from a series of compounds that have only some minor differences in individual groups, which requires a lot of inventive work.

### Conclusion:

1) Cell survival rate assay was performed on LNP formulations prepared from a series of designed compounds and cationic lipid compounds with low cytotoxicity were screened out, such as YK-201, YK-202, and YK-209.

This series of designed compounds have significant differences in chemical structure from representative cationic lipids in the prior art, such as SM-102, compound 21, compound 23, HHMA and YK-009, wherein the G₃ group is completely different, and other parts are also different. Therefore, there are also great differences in polarity, acidity and alkalinity, and hydrophilicity.

2) The LNP formulations prepared by YK-201, YK-202 and YK-209 have the lowest cytotoxicity and significantly improved cell survival rate compared with the representative cationic lipids in the prior art. For example, the cell survival rate of YK-202 is 26.85% higher than that of ALC-0315, 8.26% higher than that of SM-102, and 11.25% higher than that of HHMA.

YK-201, YK-202 and YK-209 have the lowest cytotoxicity among the compounds designed, which have the most similar structures, that is, only the G₁, G₂, R₁ or R₂ groups differ by 1 to 2 C. The cell survival rate of the three compounds can be increased by 40% compared with other compounds, such as YK-203.

Compared with compounds with only minor differences in individual groups in structure, YK-201, YK-202 and YK-209 show the lowest cytotoxicity. The cell survival rate of all three compounds is increased by 50% compared with other compounds, such as YK-214.

YK-201, YK-202 and YK-209 show the lowest cytotoxicity compared with compounds in which only the hydroxyl group of the G₃ group is changed to a methylamino group, or the hydroxyl group and the methyl group connected to N are removed. For example, the cell survival rates of YK-201, YK-202 and YK-209 are all 55% higher than that of YK-221.

3) There is no correspondence between the structure and cytotoxicity of a compound. Even compounds with small structural differences are likely to have very different cytotoxicity. Therefore, it is impossible to predict cytotoxicity based on chemical structure. It is very difficult to screen out cationic lipid compounds with low cytotoxicity and requires a lot of inventive work.

### Example 8: In vivo validation of the performance of cationic lipid delivery carriers

In addition, the protein expression and duration of mRNA delivered by the designed cationic lipids in mice was also verified. In vivo experiments further proved that the LNP delivery carrier can effectively deliver mRNA into the body and express it efficiently and continuously.

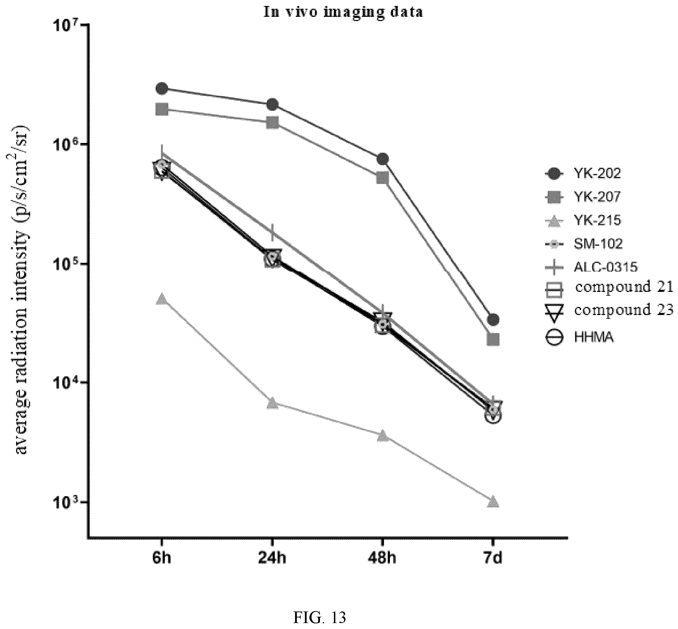

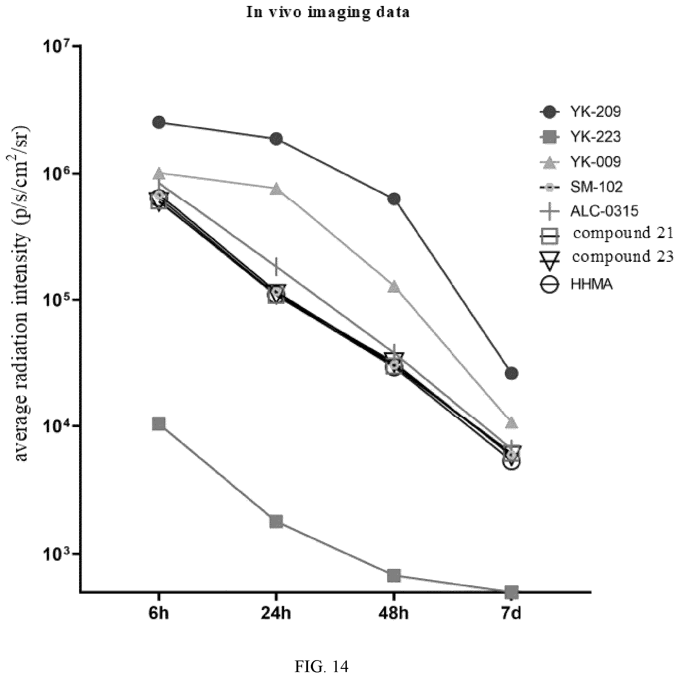

The LNP formulation containing 10 µg Fluc-mRNA (prepared according to Example 4) was injected intramuscularly into female BALB/C mice aged 4 to 6 weeks and weighing 17 to 19 g, and the mice were intraperitoneally injected with fluorescent imaging substrates at specific time points (6h, 24h, 48h and 7d) after administration. The mice moved freely for 5 minutes, and then the average radiation intensity (corresponding to the fluorescence expression intensity) of the protein expressed by the mRNA carried by the LNP in the mice was detected by IVIS Spectrum small animal in vivo imager. The test results are shown in Tables 14 to 17 and FIGs. 12 to 14.

### Experimental results:

(1) Among the designed series of compounds, the LNP formulations prepared by YK-201, YK-202 and YK-209 showed high and sustained expression of mRNA in mice, which was significantly higher than that of representative cationic lipids in the prior art. For example, the expression level of mRNA of YK-202 was 24 times that of SM-102, 25 times that of compound 21 and 23 times that of compound 23. The expression of mRNA in mice was consistent with the cell transfection activity.

**Table 14 Mouse-in vivo imaging experimental data-1**

| NO. | Cationic lipids | Time | | | |
|---|---|---|---|---|---|
| | | **6h** | **24h** | **48h** | **7d** |
| 1 | YK-201 | 2858500 | 2025680 | 730760 | 30151 |
| 2 | YK-202 | 2935500 | 2158840 | 755000 | 33524 |
| 3 | YK-209 | 2524250 | 1869220 | 629080 | 26361 |
| 4 | YK-206 | 2068900 | 1553050 | 500150 | 23015 |
| 5 | YK-207 | 1974100 | 1523040 | 525800 | 22896 |
| 6 | YK-009 | 1012400 | 767850 | 128570 | 10741 |
| 7 | SM-102 | 678870 | 115800 | 30560 | 5903 |
| 8 | ALC-0315 | 842355 | 182350 | 38130 | 6588 |
| 9 | Compound21 | 600390 | 108000 | 30025 | 6102 |
| 10 | Compound23 | 594050 | 110870 | 32074 | 5827 |
| 11 | HHMA | 638870 | 109650 | 29445 | 5312 |

Table 14 lists the expression intensity of mRNA of the LNP formulations containing Fluc-mRNA prepared by different cationic lipids at different times in mice. Among them, YK-009 is disclosed in CN114044741B (claim 1), SM-102 is compound 25 disclosed in WO2017049245A2 (page 29 of the specification), ALC-0315 is compound 3 disclosed in CN108368028B (page 24 of the specification), compound 21 and compound 23 are disclosed in WO2021055833A1 (page 22 of the specification), and HHMA is compound 1 disclosed in CN112979483B (page 12 of the specification). These cationic lipids can be used to prepare carriers for delivering mRNA.

As shown in Table 14, the Fluc-mRNA-containing LNP formulations prepared by YK-201, YK-202 and YK-209 expressed mRNA highly and continuously in mice.

The average radiation intensity of YK-201 at 6 h was 2858500, which was 4.21 times that of SM-102, 3.39 times that of ALC-0315, 4.76 times that of compound 21, 4.81 times that of compound 23, and 4.47 times that of HHMA. The average radiation intensity of YK-201 at 24 h was 2025680, which was 17.49 times that of SM-102, 11.11 times that of ALC-0315, 18.76 times that of compound 21, 18.27 times that of compound 23, and 18.47 times that of HHMA. The average radiation intensity of YK-201 at 48 h was 730760, which was 23.91 times that of SM-102, 19.16 times that of ALC-0315, 24.34 times that of compound 21, 22.78 times that of compound 23, and 24.82 times that of HHMA. The average radiation intensity of YK-201 at 7d was 30151, which was 5.11 times that of SM-102, 4.58 times that of ALC-0315, 4.94 times that of compound 21, 5.17 times that of compound 23, and 5.68 times that of HHMA.

The average radiation intensity of YK-202 at 6 h was 2935500, which was 4.32 times that of SM-102, 3.48 times that of ALC-0315, 4.89 times that of compound 21, 4.94 times that of compound 23, and 4.59 times that of HHMA. The average radiation intensity of YK-202 at 24 h was 2158840, which was 18.64 times that of SM-102, 11.84 times that of ALC-0315, 19.99 times that of compound 21, 19.47 times that of compound 23, and 19.69 times that of HHMA. The average radiation intensity of YK-202 at 48h was 755000, which was 24.71 times that of SM-102, 19.80 times that of ALC-0315, 25.15 times that of compound 21, 23.54 times that of compound 23, and 25.64 times that of HHMA. The average radiation intensity of YK-202 at 7d was 33524, which was 5.68 times that of SM-102, 5.09 times that of ALC-0315, 5.49 times that of compound 21, 5.75 times that of compound 23, and 6.31 times that of HHMA.

The average radiation intensity of YK-209 at 6 h was 2524250, which was 3.72 times that of SM-102, 3.00 times that of ALC-0315, 4.20 times that of compound 21, 4.25 times that of compound 23, and 3.95 times that of HHMA. The average radiation intensity of YK-209 at 24 h was 1869220, which was 16.14 times that of SM-102, 10.25 times that of ALC-0315, 17.31 times that of compound 21, 16.86 times that of compound 23, and 17.05 times that of HHMA. The average radiation intensity of YK-209 at 48h was 629080, which was 20.59 times that of SM-102, 16.50 times that of ALC-0315, 20.95 times that of compound 21, 19.61 times that of compound 23, and 21.36 times that of HHMA. The average radiation intensity of YK-209 at 7d was 26361, which was 4.47 times that of SM-102, 4.00 times that of ALC-0315, 4.32 times that of compound 21, 4.52 times that of compound 23, and 4.96 times that of HHMA.

The mRNA of LNP formulations prepared by YK-206 and YK-207 was also highly and continuously expressed in mice. For example, the mRNA expression level of YK-206 and YK-207 could reach 16 times that of SM-102, compound 21, compound 23 and HHMA and 13 times that of ALC-0315.

The data were analyzed using GraphPad Prism software. Any one of YK-201, YK-202 and YK-209 had significant differences from SM-102, ALC-0315, compound 21, compound 23, HHMA and YK-009 at each time, and their expression level and duration were significantly increased.

### b. Differences in chemical structure

Compared with cationic lipids in the prior art, such as SM-102, compound 21, compound 23, HHMA and YK-009, YK-201, YK-202 and YK-209 have very different chemical structures. Among them, the structural difference with HHMA is the greatest. Except only one side chain connected to the central N atom of HHMA is similar to one side chain of YK-201, YK-202 and YK-209, the other structures are completely different. Compared with SM-102, ALC-0315, compound 21, compound 23 and YK-009, the G₃ groups of YK-201, YK-202 and YK-209 are completely different, and the G₁, R₁, G₂ and R₂ groups are also very different.

### Summary:

Among the designed series of compounds, the LNP formulations prepared by YK-201, YK-202 and YK-209 have the highest mRNA expression in mice, and the expression is sustained. The expression levels at 6h, 24h, 48h and 7d are significantly higher than those of representative cationic lipids in the prior art. For example, YK-202 can reach 24 times that of SM-102, 25 times that of compound 21 and 23 times that of compound 23. The expression of mRNA in mice is consistent with the results of the cell transfection experiment in Example 7.

Furthermore, the structures of YK-201, YK-202 and YK-209 are very different from those of representative cationic lipids in the prior art. The G₃ groups are completely different, and the G₁, R₁, G₂ and R₂ groups are also significantly different.

Therefore, it is not only the LNP formulations prepared from compounds with similar structures to the cationic lipids in the prior art that have high expression in mice. On the contrary, LNP formulations prepared from compounds with significantly different structures are also very likely to have high and sustained expression of mRNA.

(2) YK-201, YK-202, and YK-209, among the most structurally similar compounds designed, that is, compounds with only 1 to 2 carbons different in G₁, G₂, G₃, R₁, or R₂, have the highest mRNA expression in mice and the longest duration. The expression level of YK-202 is more than 400 times that of other compounds, such as YK-204. The mRNA expression in mice is consistent with the cell transfection activity.

In order to compare the differences in the expression intensity and duration of the delivered mRNA in mice by the delivery carriers prepared by the compounds with the closest structures, that is, only 1 to 2 C different in G₁, G₂, G₃, R₁ or R₂ groups, YK-201, YK-202 and YK-209 were compared with YK-204. The results show that the LNP formulations prepared by this series of compounds had very different mRNA expression in mice, among which YK-201, YK-202 and YK-209 had the highest expression and the longest duration, and the expression of YK-202 could reach more than 400 times that of YK-204.

**Table 15 Mouse in vivo imaging experimental data-2**

| NO. | Cationic lipids | Time | | | |
|---|---|---|---|---|---|
| | | **6h** | **24h** | **48h** | **7d** |
| 1 | YK-201 | 2858500 | 2025680 | 730760 | 30151 |
| 2 | YK-202 | 2935500 | 2158840 | 755000 | 33524 |
| 3 | YK-209 | 2524250 | 1869220 | 629080 | 26361 |
| 4 | YK-204 | 35245 | 5230 | 2440 | 754 |
| 5 | SM-102 | 678870 | 115800 | 30560 | 5903 |
| 6 | ALC-0315 | 842355 | 182350 | 38130 | 6588 |
| 7 | Compound21 | 600390 | 108000 | 30025 | 6102 |
| 8 | Compound23 | 594050 | 110870 | 32074 | 5827 |
| 9 | HHMA | 638870 | 109650 | 29445 | 5312 |

It can be seen from Table 15 that among this series of compounds that are most similar in structure but only slightly different in the structure of individual groups, the LNP formulations prepared by YK-201, YK-202 and YK-209 have the highest mRNA expression level and the longest duration in mice.

The mRNA expression level of YK-201 was 81.10 times that of YK-204 at 6 h, 387.32 times at 24 h, 299.49 times at 48 h, and 39.99 times at 7 d.

The mRNA expression level of YK-202 was 83.29 times that of YK-204 at 6 h, 412.78 times at 24 h, 309.43 times at 48 h, and 44.46 times at 7 d.

The mRNA expression level of YK-209 was 71.62 times that of YK-204 at 6 h, 357.40 times at 24 h, 257.82 times at 48 h, and 34.96 times at 7d.

The data were analyzed using GraphPad Prism software. Any one of YK-201, YK-202 and YK-209 had significant differences from the other compounds at each time, with both the expression level and duration significantly increased.

### b. Differences in chemical structure

The structural differences of this series of compounds are very small, with only 1 to 2 carbons different in the G₁, G₂, G₃, R₁ or R₂ groups. For example, compared with YK-201, the G₃ group of YK-204 has one more carbon connected to N, and the other structures are exactly the same, but the mRNA expression of YK-201 can reach 380 times that of YK-204. Other compounds are similar.

### Summary:

Compared with the compounds that have the most similar structures and only differ by 1 to 2 C in the G₁, G₂, G₃, R₁ or R₂ groups, the LNP formulations prepared by YK-201, YK-202 and YK-209 have the highest mRNA expression intensity and the longest duration in mice. For example, the mRNA expression level of YK-202 can reach more than 400 times that of YK-204 in 24 hours, and can still reach 40 times in 7 days. The mRNA expression in mice is consistent with the results of the cell transfection experiment in Example 7.

It is found that there is no correspondence between mRNA expression in mice and cationic lipid structure, and that even LNP formulations prepared from a group of compounds that have the most similar structures and only differ by 1 to 2 C in the G₁, G₂, G₃, R₁ or R₂ groups, are likely to have very different mRNA expression levels and durations in mice.

Therefore, it is very difficult to screen out cationic lipid compounds with high and sustained expression in animals from a series of most structurally similar compounds, which requires a lot of inventive work.

(3) YK-201, YK-202, and YK-209 showed the highest mRNA expression level and longest duration in mice compared to compounds with only minor structural differences in the G₁, G₂, G₃, R₁, or R₂ groups. The expression level of YK-202 was more than 800 times that of other compounds, such as YK-217. The mRNA expression in mice was consistent with the cell transfection activity.

The differences in expression in mice of LNP formulations containing mRNA prepared from YK-201, YK-202 and YK-209 and other structurally similar compounds were compared. This series of compounds have only some minor differences in individual groups, such as 2 less C in G₁, 1 less C in R₁, and ether bonds, ester bonds or sulfur atoms introduced in G₃. The results show that YK-201, YK-202 and YK-209 had the highest expression levels and the longest duration, which was significantly higher than those of other compounds. The expression level of YK-202 can be 800 times higher than that of YK-217.

**Table 16 Mouse in vivo imaging experimental data-3**

| NO. | Cationic lipids | Time | | | |
|---|---|---|---|---|---|
| | | **6h** | **24h** | **48h** | **7d** |
| 1 | YK-201 | 2858500 | 2025680 | 730760 | 30151 |
| 2 | YK-202 | 2935500 | 2158840 | 755000 | 33524 |
| 3 | YK-209 | 2524250 | 1869220 | 629080 | 26361 |
| 4 | YK-215 | 50564 | 6815 | 3640 | 1020 |
| 5 | YK-217 | 24585 | 2650 | 1024 | 523 |
| 6 | SM-102 | 678870 | 115800 | 30560 | 5903 |
| 7 | ALC-0315 | 842355 | 182350 | 38130 | 6588 |
| 8 | Compound21 | 600390 | 108000 | 30025 | 6102 |
| 9 | Compound23 | 594050 | 110870 | 32074 | 5827 |
| 10 | HHMA | 638870 | 109650 | 29445 | 5312 |

### a. Differences in expression in mice

As can be seen from Table 16, among this series of compounds, the LNP formulations prepared from YK-201, YK-202 and YK-209 have the highest mRNA expression level and the longest duration in mice.

The expression level of YK-201 was 56.53 times that of YK-215 and 116.27 times that of YK-217 at 6 hours, 297.24 times that of YK-215 and 764.41 times that of YK-217 at 24 hours, 200.76 times that of YK-215 and 713.63 times that of YK-217 at 48 hours, and 29.56 times that of YK-215 and 57.65 times that of YK-217 at 7 d.

The expression level of YK-202 was 58.06 times that of YK-215 and 119.40 times that of YK-217 at 6 hours, 316.78 times that of YK-215 and 814.66 times that of YK-217 at 24 hours, 207.42 times that of YK-215 and 737.30 times that of YK-217 at 48 hours, and 32.87 times that of YK-215 and 64.10 times that of YK-217 at 7 d.

The expression level of YK-209 was 49.92 times that of YK-215 and 102.67 times that of YK-217 at 6 hours, 274.28 times that of YK-215 and 705.37 times that of YK-217 at 24 hours, 172.82 times that of YK-215 and 614.34 times that of YK-217 at 48 hours, and 25.84 times that of YK-215 and 50.40 times that of YK-217 at 7 d.

The data were analyzed using GraphPad Prism software. Any one of YK-201, YK-202 and YK-209 had significant differences from the other compounds at each time, with both the expression level and duration significantly increased.

### b. Differences in chemical structure

This series of compounds has some minor differences in individual groups, such as 2 less C in G₁, 1 less C in R₁, and ether bonds, ester bonds or sulfur atoms introduced in G₃. Compared with YK-201, in the G₃ group of YK-215, only an ether bond is introduced and one more methyl group is connected to O. The other structures are exactly the same. But the mRNA expression level of YK-201 can reach 300 times that of YK-215. The other compounds are similar.

### Summary:

Compared with compounds with very similar structures but with some minor differences in G₁, G₂, G₃, R₁ or R₂ groups, the LNP formulations prepared by YK-201, YK-202 and YK-209 have the highest mRNA expression intensity and the longest duration in mice. For example, the mRNA expression level of YK-202 can reach 800 times that of YK-217 in 24 hours and can still reach 60 times in 7 days. The expression of mRNA in mice is consistent with the results of the cell transfection experiment in Example 7.

It is found that there is no correspondence between the mRNA expression in mice and the cationic lipid structure. Even if there are only some minor differences in individual groups, such as the 2 less C in G₁, 1 less C in R₁, and the introduction of ether bonds, ester bonds or sulfur atoms in G₃, the LNP formulations prepared from these compounds would most likely have very different mRNA expression levels and duration in mice.

Therefore, it is very difficult to screen out cationic lipid compounds with high and sustained expression in animals from a series of compounds with only minor differences in individual groups, and this requires a lot of inventive work.

(4) Compared with compounds that differ only slightly in the G₃ group, YK-201, YK-202, and YK-209 show the highest mRNA expression in mice and the longest duration of expression. The expression levels of the three compounds are more than 1,000 times that of other compounds, such as YK-223. The mRNA expression in mice is consistent with the cell transfection activity.

The compounds listed in Table 16 differ from YK-201, YK-202 and YK-209 only in that the G₃ group is slightly different, such as the hydroxyl group is changed to a methylamino group. The results of in vivo transfection in mice showed that the expression levels of YK-201, YK-202 and YK-209 were the highest and lasted the longest, significantly higher than other compounds, and all three were more than 1,000 times higher than YK-223.

**Table 17 Mouse in vivo imaging experimental data-4**

| No. | Cationic lipids | Time | | | |
|---|---|---|---|---|---|
| | | **6h** | **24h** | **48h** | **7d** |
| 1 | YK-201 | 2858500 | 2025680 | 730760 | 30151 |
| 2 | YK-202 | 2935500 | 2158840 | 755000 | 33524 |
| 3 | YK-209 | 2524250 | 1869220 | 629080 | 26361 |
| 4 | YK-223 | 10355 | 1786 | 678 | 440 |
| 5 | SM-102 | 678870 | 115800 | 30560 | 5903 |
| 6 | ALC-0315 | 842355 | 182350 | 38130 | 6588 |
| 7 | Compound21 | 600390 | 108000 | 30025 | 6102 |
| 8 | Compound23 | 594050 | 110870 | 32074 | 5827 |
| 9 | HHMA | 638870 | 109650 | 29445 | 5312 |

### a. Expression differences in mice

As can be seen from Table 17, among this series of compounds, the LNP formulations prepared from YK-201, YK-202 and YK-209 have the highest mRNA expression level and the longest duration in mice.

The mRNA expression level of YK-201 can reach 276.05 times that of YK-223 at 6 h, 1134.20 times at 24 h, 1077.82 times at 48 h, and 68.53 times at 7 d.

The mRNA expression level of YK-202 can reach 283.49 times that of YK-223 at 6 h, 1208.76 times at 24 h, 1113.57 times at 48 h, and 76.19 times at 7 d.

The mRNA expression level of YK-209 can reach 243.77 times that of YK-223 at 6 h, 1046.60 times at 24 h, 927.85 times at 48 h, and 59.91 times at 7 d.

The data were analyzed using GraphPad Prism software. Any one of YK-201, YK-202 and YK-209 had significant differences from the other compounds at each time, with both the expression level and duration significantly increased.

### b. Differences in chemical structure

The chemical structure of this series of compounds is slightly different in the G₃ group. For example, compared with YK-209, YK-223 is different only in the G₃ group, that is, the hydroxyl group in YK-209 is changed to methylamino, and the other structures are exactly the same. But the expression level of YK-209 can reach more than 1,000 times that of YK-223. Other compounds are similar.

### Summary:

Compared with compounds with very similar structures and only slightly different G₃ groups, the LNP formulations prepared by YK-201, YK-202 and YK-209 have the highest mRNA expression intensity and the longest duration in mice, and the expression levels of the three can reach more than 1000 times that of YK-223. The expression of mRNA in mice is consistent with the results of the cell transfection experiment in Example 7.

It is found that there is no correspondence between the expression of mRNA in mice and the cationic lipid structure. Even if the structure is very similar, where only the hydroxyl group in the G₃ group is changed to a methylamino group, or the hydroxyl group and the methyl group connected to N are removed, the LNP formulations prepared from these compounds are likely to have very different mRNA expression levels and duration in mice.

Therefore, it is very difficult to screen out cationic lipid compounds with high and sustained expression in animals from a series of compounds with very similar structures, which requires a lot of inventive work.

### Conclusion:

1) In vivo animal delivery experiments was performed on the LNP formulations prepared from a series of designed compounds and cationic lipid compounds with high and sustained expression of mRNA in mice, such as YK-201, YK-202 and YK-209, were screened out.

This series of designed compounds have significant differences in chemical structure from representative cationic lipids in the prior art, such as SM-102, ALC-0315, compound 21, compound 23, HHMA and YK-009. The G₃ group is completely different, and other parts are also different. Therefore, there are also great differences in polarity, acidity and alkalinity, and hydrophilicity.

2) The LNP formulations prepared by YK-201, YK-202 and YK-209 show high and sustained expression of mRNA in mice, which is significantly higher than that of the representative cationic lipids in the prior art. For example, the mRNA expression level of YK-202 is 24 times that of SM-102, 25 times that of compound 21 and 23 times that of compound 23.

YK-201, YK-202 and YK-209 have the highest mRNA expression level and the longest duration in mice among a series of compounds designed with the most similar structures, only with 1 to 2 C differences in G₁, G₂, G₃, R₁ or R₂ groups. For example, the mRNA expression level of YK-202 can reach more than 400 times that of YK-204 at 24 h, and can still reach 40 times at 7d.

YK-201, YK-202 and YK-209 have the highest mRNA expression level and the longest duration in mice among a series of compounds with only slight differences in G₁, G₂, G₃, R₁ or R₂ groups. For example, the mRNA expression level of YK-202 can be 800 times higher than that of YK-217.

Among a series of compounds in which only the hydroxyl group in the G₃ group is changed to a methylamino group, or the hydroxyl group and the methyl group connected to N are removed, YK-201, YK-202 and YK-209 have the highest mRNA expression level and the longest duration in mice. For example, the mRNA expression of the three compounds is more than 1,000 times higher than that of YK-223.

3) There is no correspondence between the structure of cationic lipids and the high and sustained expression of delivered mRNA in mice. Even the mRNA in the LNP formulations prepared from the cationic lipid compounds with small structural differences is likely to have very different expressions in animals. It is impossible to predict whether mRNA is highly and continuously expressed in animals based on the chemical structure of cationic lipids. It is very difficult to screen out cationic lipid compounds with high and sustained mRNA expression, which requires a lot of creative work.

### Conclusion:

1. The designed series of compounds, including YK-201, YK-202 and YK-209, as well as YK-206 and YK-207, have significant differences in chemical structure from the cationic lipids in the prior art, such as SM-102, ALC-0315, compound 21, compound 23, HHMA and YK-009. The G₃ group is completely different, and the other parts are also different, so there are also great differences in polarity, acidity and alkalinity, and hydrophilicity.

In this series of designed compounds, the LNP formulations prepared from YK-201, YK-202 and YK-209 have significantly improved encapsulation rate, drug loading concentration and total RNA concentration, significantly improved cell transfection efficiency, significantly reduced cytotoxicity, and significantly increased mRNA expression level and duration in mice compared with representative cationic lipids in the prior art. For example, the encapsulation rate of YK-209 can be increased by 41% compared with compound 23, the drug loading concentration of YK-209 can reach 2 times that of compound 23, and the total RNA concentration of YK-201 can reach 1.5 times that of compound 21; the cell transfection efficiency of YK-202 can reach 18 times that of SM-102, 21 times that of compound 21, and 22 times that of compound 23; the cell survival rate of YK-202 can be 26.85% higher than that of ALC-0315, 8.26% higher than that of SM-102, and 11.25% higher than that of HHMA; the mRNA expression level in mice of YK-202 can reach 24 times that of SM-102, 2.5 times that of compound 21, and 2.3 times that of compound 23.

Among the series of compounds designed with very small differences in chemical structure, the LNP formulations prepared from YK-201, YK-202 and YK-209 have significantly improved cell transfection efficiency, significantly reduced cytotoxicity, and significantly increased mRNA expression level and duration in mice compared with other compounds.

Structurally, this series of compounds only differ from YK-201, YK-202 and YK-209 in 1 to 2 C atoms in individual groups, such as 2 less C atoms in G₁, 1 less C in R₁, an ether bond, an ester bond or a sulfur atom introduced into G₃, or the hydroxyl group in the G₃ group being changed to a methylamino group, or the hydroxyl group and the methyl group connected to N being removed from the G₃ group. However, the cell transfection efficiency of YK-201 and YK-202 can reach more than 1000 times that of YK-221 and YK-225, the cytotoxicity can be reduced by 55% compared with YK-221, and the mRNA expression level in mice can reach more than 1000 times that of YK-223.

2. There is no obvious correspondence between the structure of cationic lipid compounds and the intracellular transfection efficiency, toxicity to cells, and high and sustained expression in animals of mRNA in LNP formulation prepared from the cationic lipid compounds. The compounds with small structural differences are very likely to have very large differences in transfection efficiency and/or toxicity to cells, and intracellular expression.

For example, compared with YK-201, YK-204 has only one more C in the G₃ group connected to N, and the other structures are exactly the same. However, the cell transfection efficiency of YK-201 is 78 times that of YK-204, and the toxicity of YK-201 to transfected cells is 30% lower than that of YK-204. The mRNA expression level of YK-201 in mice can reach 380 times that of YK-204. Compared with YK-209, YK-225 is different in that only the hydroxyl group in the G₃ group is changed to methylamino, and the single chains of R₁ and R₂ groups each have one more C, and each single chain in the double chain has 2 less C. The other structures are exactly the same. But the cell transfection efficiency of YK-209 reaches 800 times that of YK-225, and the toxicity of YK-209 to transfected cells is 30% lower than that of YK-225.

Therefore, it is very difficult to screen out suitable cationic lipid compounds that can simultaneously have high transfection efficiency and low toxicity to cells, as well as high and sustained expression of mRNA in mice, which requires a lot of inventive work.

3. Through unique design and extensive screening, the present disclosure has found some compounds, such as YK-201, YK-202, YK-209, YK-206 and YK-207, which can deliver nucleic acids with significantly improved encapsulation rate, drug loading concentration and total RNA concentration, significantly improved cell transfection efficiency, significantly reduced cytotoxicity, and significantly increased expression level and duration in animals compared to other compounds in the prior art, which achieves unexpected technical effects.

## Claims

1. A compound of Formula (I) 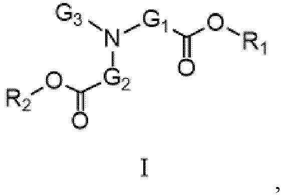 or an N-oxide, a solvate, a pharmaceutically acceptable salt or a stereoisomer thereof, wherein
G₁ is C₁₋₆ alkylene;
G₂ is C₂₋₈ alkylene;
R₁ is C₆₋₂₀ straight chain or branched alkyl;
R₂ is C₁₂₋₂₅ branched alkyl;
G₃ is HO(CH₂)₂N(CH₃)(CH₂)₂-, HO(CH₂)₂N(CH₂CH₃)(CH₂)₂-, (HO(CH₂)₂)₂N(CH₂)₂-, CH₃O(CH₂)₂N(CH₃)(CH₂)₂-, (CH₃)₂N(CH₂)₃SC(O)O(CH₂)₂-, (CH₃)₂N(CH₂)₃SC(O)-, CH₃NH(CH₂)₂N(CH₃)(CH₂)₂- or CH₃CH₂NH(CH₂)₂-.

2. The compound of Formula (I), or the N-oxide, solvate, pharmaceutically acceptable salt or stereoisomer thereof according to claim 1, wherein G₁ is unsubstituted C₂₋₅ alkylene.

3. The compound of Formula (I), or the N-oxide, solvate, pharmaceutically acceptable salt or stereoisomer thereof according to claim 2, wherein G₁ is unsubstituted C₅ alkylene or unsubstituted C₃ alkylene.

4. The compound of Formula (I), or the N-oxide, solvate, pharmaceutically acceptable salt or stereoisomer thereof according to any one of the preceding claims, wherein G₂ is unsubstituted C₄₋₇ alkylene.

5. The compound of Formula (I), or the N-oxide, solvate, pharmaceutically acceptable salt or stereoisomer thereof according to claim 4, wherein G₂ is unsubstituted C₇ alkylene or unsubstituted C₅ alkylene or unsubstituted C₄ alkylene.

6. The compound of Formula (I), or the N-oxide, solvate, pharmaceutically acceptable salt or stereoisomer thereof according to any one of the preceding claims, wherein R₁ is unsubstituted C₈₋₁₂ straight chain alkyl.

7. The compound of Formula (I), or the N-oxide, solvate, pharmaceutically acceptable salt or stereoisomer thereof according to claim 6, wherein R₁ is unsubstituted C₁₁ straight chain alkyl or unsubstituted C₁₀ straight chain alkyl.

8. The compound of Formula (I), or the N-oxide, solvate, pharmaceutically acceptable salt or stereoisomer thereof according to any one of claims 1 to 5, wherein R₁ is unsubstituted C₁₈ branched alkyl, unsubstituted C₁₇ branched alkyl or unsubstituted C₁₅ branched alkyl.

9. The compound of Formula (I), or the N-oxide, solvate, pharmaceutically acceptable salt or stereoisomer thereof according to claim 8, wherein R₁ is

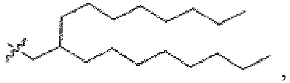

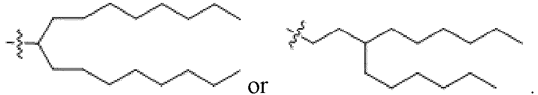

10. The compound of Formula (I), or the N-oxide, solvate, pharmaceutically acceptable salt or stereoisomer thereof according to any one of the preceding claims, wherein R₂ is unsubstituted C₁₄₋₂₂ branched alkyl.

11. The compound of Formula (I), or the N-oxide, solvate, pharmaceutically acceptable salt or stereoisomer thereof according to claim 10, wherein R₂ is unsubstituted C₁₇ branched alkyl or unsubstituted C₁₈ branched alkyl or unsubstituted C₁₅ branched alkyl.

12. The compound of Formula (I), or the N-oxide, solvate, pharmaceutically acceptable salt or stereoisomer thereof according to claim 11, wherein R₂ is 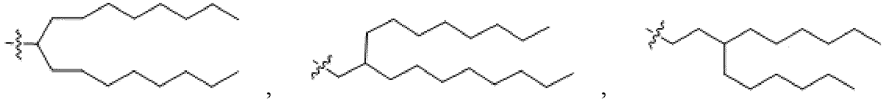 or

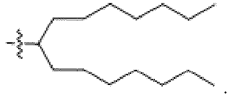

13. The compound of Formula (I), or the N-oxide, solvate, pharmaceutically acceptable salt or stereoisomer thereof according to claim 1, wherein the compound of Formula (I) has a structure selected from the group consisting of: 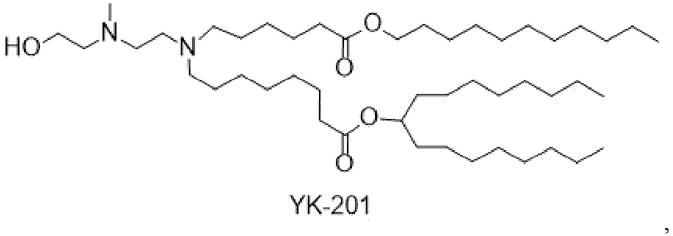 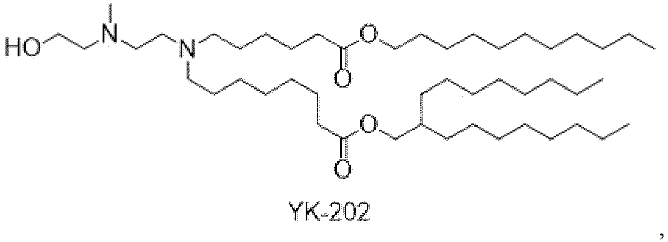 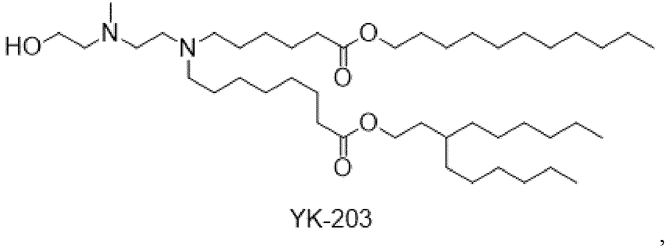 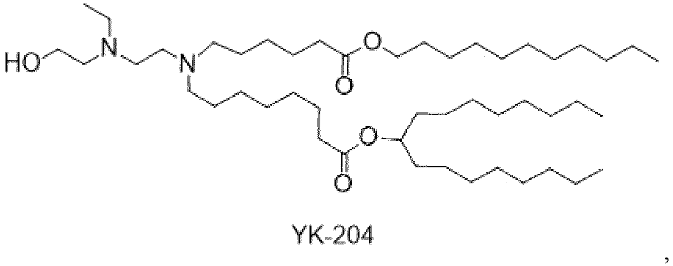 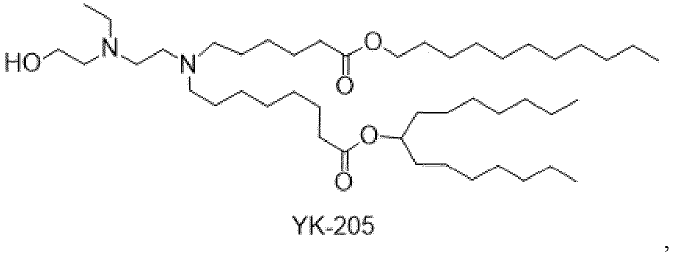 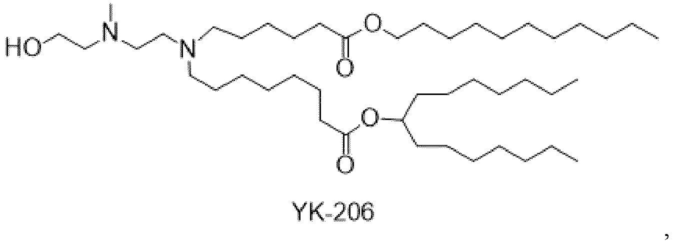 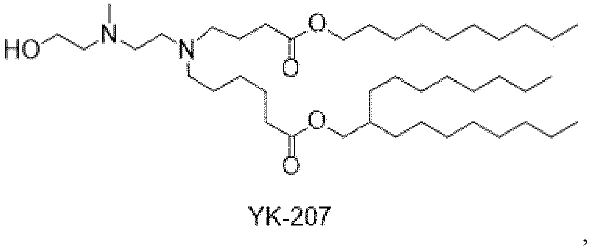 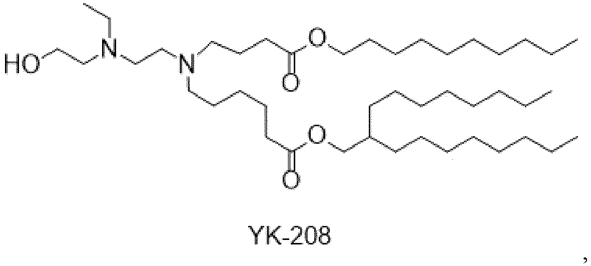 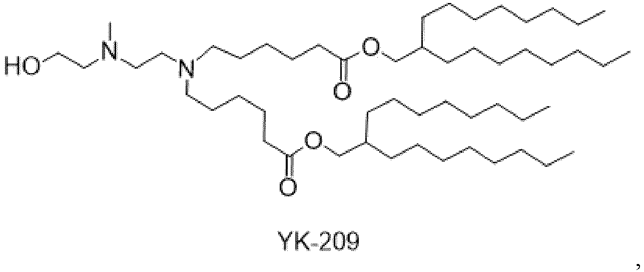 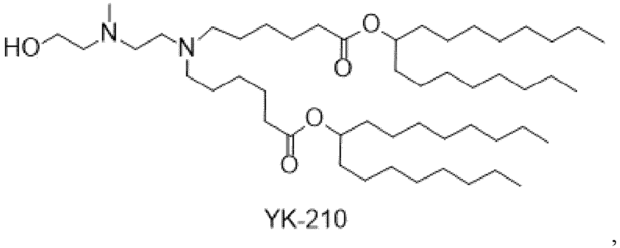 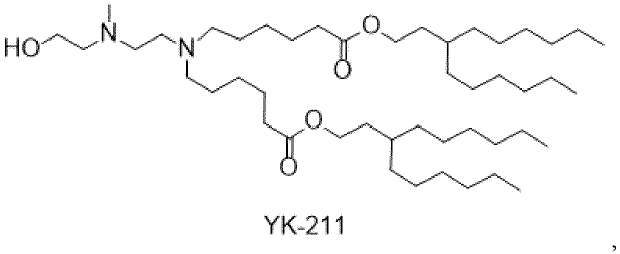 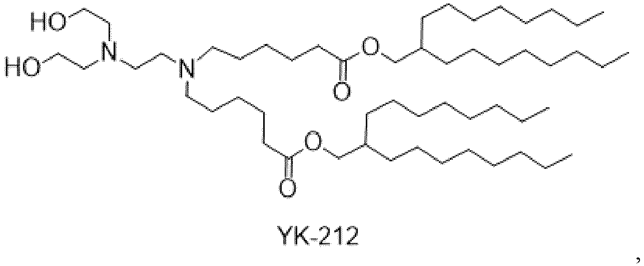 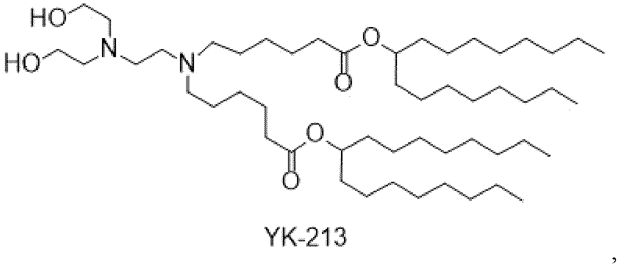 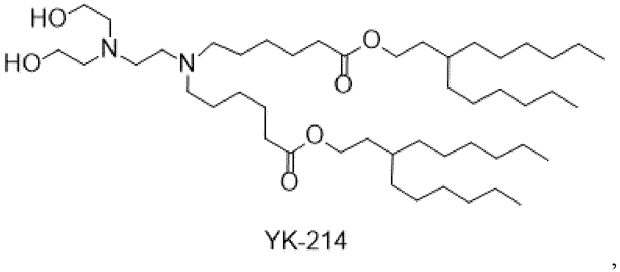 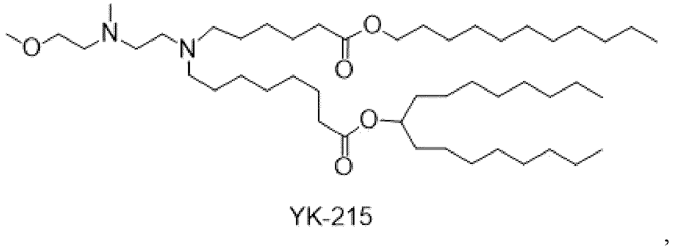 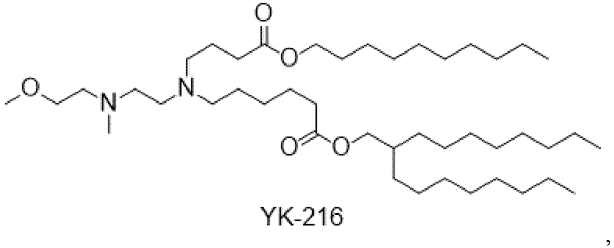 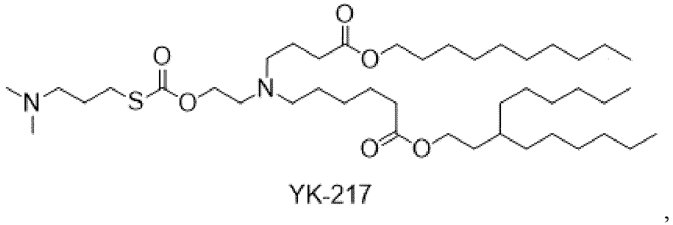 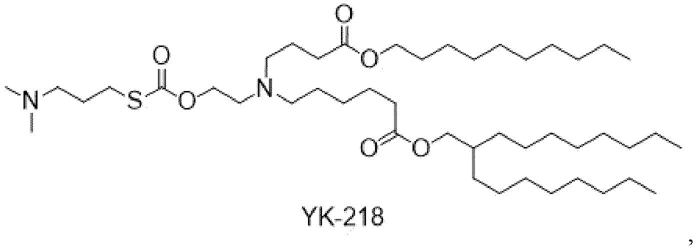 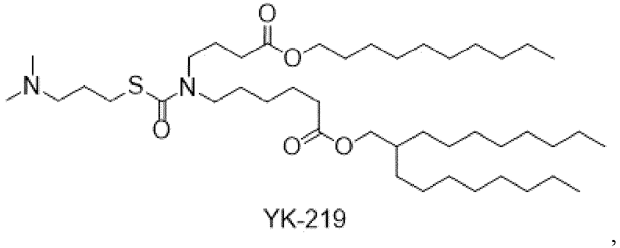 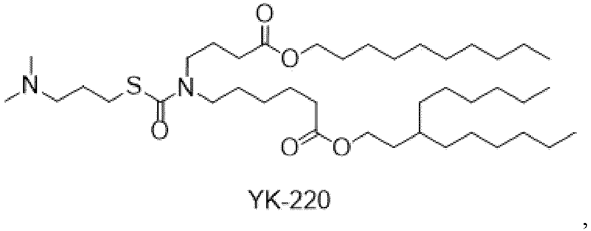 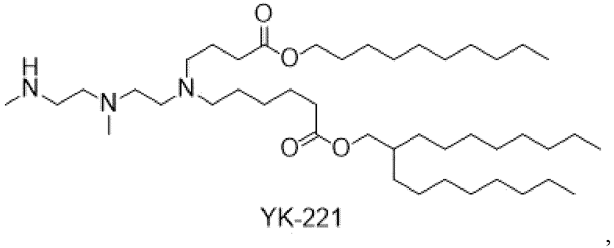 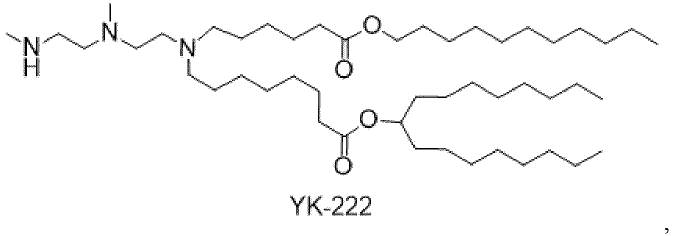 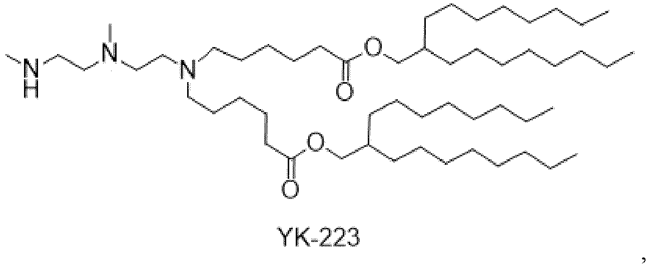 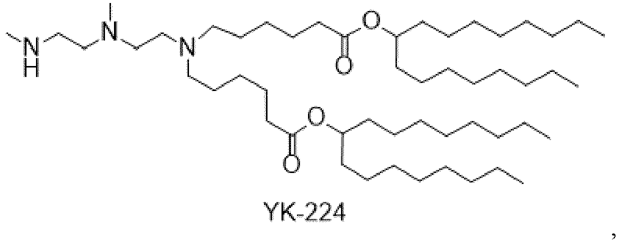 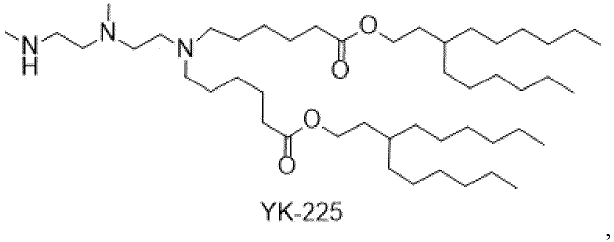 and

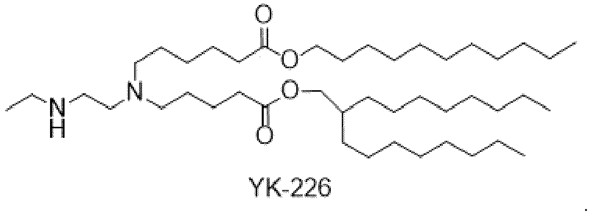

14. The compound of Formula (I), or the N-oxide, solvate, pharmaceutically acceptable salt or stereoisomer thereof according to claim 1, wherein the compound of Formula (I) is compound YK-201 having the following structure:

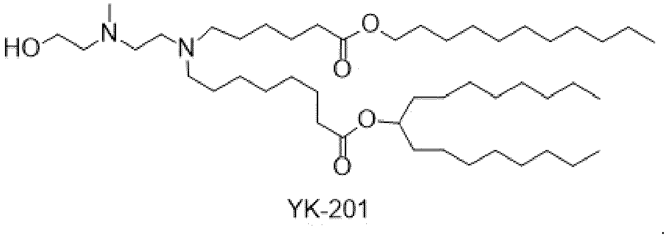

15. The compound of Formula (I), or the N-oxide, solvate, pharmaceutically acceptable salt or stereoisomer thereof according to claim 1, wherein the compound of Formula (I) is compound YK-202 having the following structure:

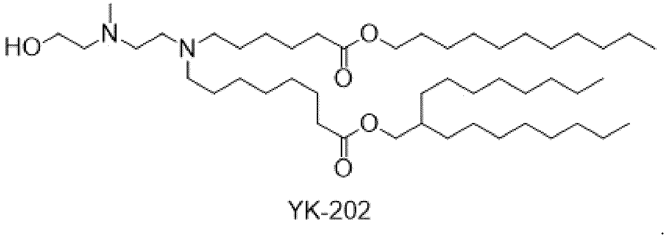

16. The compound of Formula (I), or the N-oxide, solvate, pharmaceutically acceptable salt or stereoisomer thereof according to claim 1, wherein the compound of Formula (I) is compound YK-209 having the following structure:

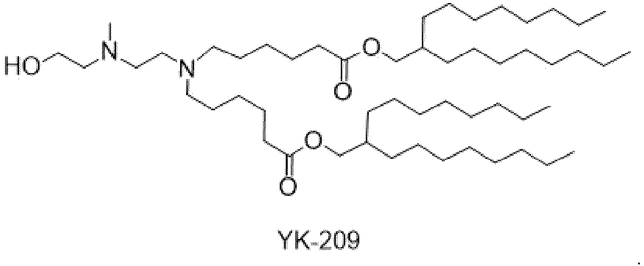

17. The compound of Formula (I), or the N-oxide, solvate, pharmaceutically acceptable salt or stereoisomer thereof according to claim 1, wherein the compound of Formula (I) is compound YK-206 having the following structure:

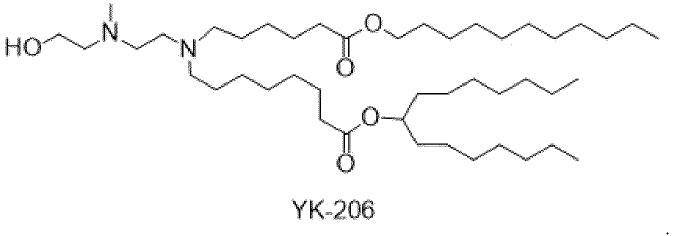

18. The compound of Formula (I), or the N-oxide, solvate, pharmaceutically acceptable salt or stereoisomer thereof according to claim 1, wherein the compound of Formula (I) is compound YK-207 having the following structure:

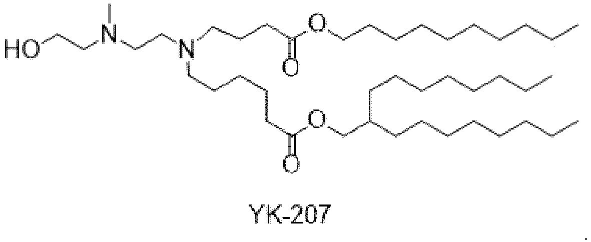

19. A composition comprising a carrier, wherein the carrier comprises a cationic lipid, and the cationic lipid comprises the compound of Formula (I) or the N-oxide, solvate, pharmaceutically acceptable salt or stereoisomer thereof according to any one of the preceding claims.

20. The composition according to claim 19, wherein a molar ratio of the cationic lipid to the carrier is 25% to 75%.

21. The composition according to any one of claims 19 to 20, wherein the carrier further comprises a neutral lipid.

22. The composition according to claim 21, wherein a molar ratio of the cationic lipid to the neutral lipid is 1:1 to 15:1, preferably 4.5:1.

23. The composition according to claims 21 to 22, wherein the neutral lipid is selected from the group consisting of phosphatidylcholine, phosphatidylethanolamine, sphingomyelin, ceramide, sterol, a derivative thereof and a combination thereof.

24. The composition according to claim 23, wherein the neutral lipid is one or more selected from the group consisting of: 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesteryl hemisuccinyl-sn-glycero-3-phosphocholine (OChemsPC), 1-hexadecyl-sn-glycero-3-phosphocholine (C16 Lyso PC), 1,2-dialinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dialinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), dipalmitoylphosphatidylglycerol (DPPG), palmitoyloleoylphosphatidylethanolamine (POPE), distearoyl-phosphatidyl-ethanolamine (DSPE), dipalmitoylphosphatidylethanolamine (DPPE), dimyristoylphosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethanolamine, phosphatidylserine, phosphatidylinositol, phosphatidic acid, palmitoyloleoylphosphatidylcholine, lysophosphatidylcholine, lysophosphatidylethanolamine (LPE) and a mixture thereof.

25. The composition according to claim 24, wherein the neutral lipid is DOPE and/or DSPC.

26. The composition according to any one of claims 19 to 25, wherein the carrier further comprises a structured lipid.

27. The composition according to claim 26, wherein a molar ratio of the cationic lipid to the structured lipid is 0.6:1 to 3:1.

28. The composition according to any one of claims 26 to 27, wherein the structured lipid is selected from the group consisting of: cholesterol, non-sterols, sitosterol, ergosterol, campesterol, stigmasterol, brassicasterol, tomatine, tomatine, ursolic acid, α-tocopherol, corticosteroids, and a mixture thereof.

29. The composition according to claim 28, wherein the structured lipid is cholesterol.

30. The composition according to any one of claims 19 to 29, wherein the carrier further comprises a polymer-conjugated lipid.

31. The composition according to claim 30, wherein a molar ratio of the polymer-conjugated lipid to the carrier is 0.5% to 10%, preferably 1.5%.

32. The composition according to any one of claims 30 to 31, wherein the polymer-conjugated lipid is one or more selected from the group consisting of: PEG-modified phosphatidylethanolamine, PEG-modified phosphatidic acid, PEG-modified ceramide, PEG-modified dialkylamine, PEG-modified diacylglycerol, PEG-modified dialkylglycerol, and a combination thereof.

33. The composition according to claim 32, wherein the polymer-conjugated lipid is one or more selected from the group consisting of: distearoylphosphatidylethanolamine polyethylene glycol 2000 (DSPE-PEG2000), dimyristoylglycerol-3-methoxypolyethylene glycol 2000 (DMG-PEG2000) and methoxypolyethylene glycol ditetradecanoyl acetamide (ALC-0159).

34. The composition according to any one of claims 19 to 33, wherein the carrier comprises a neutral lipid, a structured lipid and a polymer-conjugated lipid, and a molar ratio of the cationic lipid, the neutral lipid, the structured lipid and the polymer-conjugated lipid is (25 to 75):(5 to 25):(15 to 65):(0.5 to 10).

35. The composition according to claim 34, wherein a molar ratio of the cationic lipid, the neutral lipid, the structured lipid, and the polymer-conjugated lipid is (35 to 49):(7.5 to 15):(35 to 55):(1 to 5).

36. The composition according to claim 35, wherein a molar ratio of the cationic lipid, the neutral lipid, the structured lipid, and the polymer-conjugated lipid is 45:10:43.5:1.5.

37. The composition according to any one of claims 19 to 36, wherein the composition is a nanoparticle formulation having an average particle size of 10 nm to 300 nm and a polydispersity coefficient of ≤50%.

38. The composition according to claim 37, wherein the nanoparticle formulation has an average particle size of 90 nm to 260 nm and a polydispersity coefficient of ≤40%.

39. The composition according to any one of claims 19 to 38, wherein the cationic lipid further comprises one or more other ionizable lipid compounds.

40. The composition according to any one of claims 19 to 39, further comprising a therapeutic agent or a prophylactic agent.

41. The composition according to claim 40, wherein a mass ratio of the carrier to the therapeutic agent or prophylactic agent is 10:1 to 30:1.

42. The composition according to claim 41, wherein a mass ratio of the carrier to the therapeutic agent or prophylactic agent is 12.5:1 to 20:1.

43. The composition according to claim 42, wherein a mass ratio of the carrier to the therapeutic agent or prophylactic agent is 15:1.

44. The composition according to any one of claims 40 to 43, wherein the therapeutic agent or prophylactic agent comprises one or more of a nucleic acid molecule, a small molecule compound, a polypeptide or a protein.

45. The composition according to any one of claims 40 to 44, wherein the therapeutic agent or prophylactic agent is a vaccine or a compound capable of eliciting an immune response.

46. The composition according to any one of claims 40 to 43, wherein the therapeutic agent or prophylactic agent is a nucleic acid.

47. The composition according to any one of claims 40 to 43, wherein the therapeutic agent or prophylactic agent is ribonucleic acid (RNA).

48. The composition according to any one of claims 40 to 43, wherein the therapeutic or prophylactic agent is deoxyribonucleic acid (DNA).

49. The composition according to claim 47, wherein the RNA is selected from the group consisting of small interfering RNA (siRNA), asymmetric interfering RNA (aiRNA), micro RNA (miRNA), Dicer-substrate RNA (dsRNA), small hairpin RNA (shRNA), messenger RNA (mRNA) and a mixture thereof.

50. The composition according to claim 49, wherein the RNA is mRNA.

51. The composition according to any one of claims 19 to 50, wherein the composition further comprises one or more pharmaceutically acceptable excipients or diluents.

52. Use of the compound of Formula (I) or the N-oxide, solvate, pharmaceutically acceptable salt or stereoisomer thereof according to any one of claims 1 to 18 or the composition according to any one of claims 19 to 51 in the manufacture of a nucleic acid drug, a gene vaccine, a small molecule drug, a polypeptide or a protein drug.

53. Use of the compound of Formula (I) or the N-oxide, solvate, pharmaceutically acceptable salt or stereoisomer thereof according to any one of claims 1 to 18 or the composition according to any one of claims 19 to 51 in the manufacture of a medicament for treating a disease or condition in a mammal in need thereof.

54. The use according to any one of claims 52 to 53, wherein the disease or condition is **characterized by** dysfunctional or abnormal protein or polypeptide activity.

55. The use according to claim 54, wherein the disease or condition is selected from the group consisting of infectious diseases, cancer and proliferative diseases, genetic diseases, autoimmune diseases, diabetes, neurodegenerative diseases, cardiovascular and renal vascular diseases, and metabolic diseases.

56. The use according to claim 55, wherein the infectious disease is selected from the group consisting of diseases caused by coronavirus, influenza virus, or HIV virus, pediatric pneumonia, Rift Valley fever, yellow fever, rabies, and various herpes.

57. The use according to any one of claims 53 to 56, wherein the mammal is a human.

58. The use according to any one of claims 52 to 57, wherein the composition is to be administered intravenously, intramuscularly, intradermally, subcutaneously, intranasally or by inhalation.

59. The use according to claim 58, wherein the composition is to be administered subcutaneously.

60. The use according to any one of claims 53 to 59, wherein the therapeutic or prophylactic agent is to be administered to a mammal at an amount of about 0.001 mg/kg to about 10 mg/kg.
